# EUROPEAN PATENT SPECIFICATION

(11) **EP 1 708 751 B1**
(45) Date of publication and mention of the grant of the patent: **28.09.2011**
(21) Application number: 04812982.9
(22) Date of filing: 06.12.2004
(51) Int. Cl.: A61K 39/395, C07K 16/30, C07H 21/04

(54) **METHODS OF KILLING TUMOR CELLS BY TARGETING INTERNAL ANTIGENS EXPOSED ON APOPTOTIC TUMOR CELLS**
VERFAHREN ZUM ABTÖTEN VON TUMORZELLEN MITTELS TARGETING VON AUF APOPTOTISCHEN TUMORZELLEN EXPONIERTEN INTERNEN ANTIGENEN
PROCEDES D'ELIMINATION DE CELLULES TUMORALES PAR CIBLAGE D'ANTIGENE INTERNE EXPOSE SUR DES CELLULES TUMORALES APOPTOTIQUES

(30) Priority: 04.12.2003 US 526572 P; 23.12.2003 US 531688 P
(43) Date of publication of application: 11.10.2006
(73) Proprietor: Vaccinex, Inc., Rochester, NY 14620 (US)
(72) Inventor: EVANS, Elizabeth E., Bloomfield, New York 14469 (US); PARIS, Mark J., W. Henrietta, New York 14586 (US); SAHASRABUDHE, Deepak M., Rochester, New York 14618 (US); SMITH, Ernest S., Ontario, New York 14519 (US); ZAUDERER, Maurice, Pittsford, New York 14534 (US)
(74) Representative: Harding, Charles Thomas
(86) International application number: PCT/US2004/040573
(87) International publication number: WO 2005/055936

(56) References cited:
- WO-A-03/104428
- US-A1- 2002 155 447
- SAHASRABUDHE D M ET AL: "Shared T cell-defined antigens on independently derived tumors" JOURNAL OF IMMUNOLOGY, THE WILLIAMS AND WILKINS CO. BALTIMORE, US, vol. 151, no. 11, 1 December 1993 (1993-12-01), pages 6302-6310, XP002107628 ISSN: 0022-1767

### SEQUENCE LISTING

<110> Vaccinex, Inc.
<120> Methods of Killing Tumor Cells by Targeting Internal Antigens Exposed on Apoptotic Tumor Cells
<130> 1843.019PC03
<150> US 60/526,572
   <151> 2003-12-04
<150> US 60/531,688
   <151> 2003-12-23
<160> 61
<170> PatentIn version 3.3
<210> 1
   <211> 354
   <212> DNA
   <213> Homo sapiens
<220>
   <221> CDS
   <222> (7) .. (354)
<400> 1
   gccgcg atg agc ggg gag ccg ggg cag acg tcc gta gcg ccc cct ccc        48
          Met Ser Gly Glu Pro Gly Gln Thr Ser Val Ala Pro Pro Pro
          1               5                   10
   gag gag gtc gag ccg ggc agt ggg gtc cgc atc gtg gtg gag tac tgt       96
   Glu Glu Val Glu Pro Gly Ser Gly Val Arg Ile Val Val Glu Tyr Cys
   15                  20                  25                  30
   gaa ccc tgc ggc ttc gag gcg acc tac ctg gag ctg gcc agt gct gtg      144
   Glu Pro Cys Gly Phe Glu Ala Thr Tyr Leu Glu Leu Ala Ser Ala Val
                   35                  40                  45
   aag gag cag tat ccg ggc atc gag atc gag tcg cgc ctc ggg ggc aca      192
   Lys Glu Gln Tyr Pro Gly Ile Glu Ile Glu Ser Arg Leu Gly Gly Thr
               50                  55                  60
   ggt gcc ttt gag ata gag ata aat gga cag ctg gtg ttc tcc aag ctg      240
   Gly Ala Phe Glu Ile Glu Ile Asn Gly Gln Leu Val Phe Ser Lys Leu
           65                  70                  75
   gag aat ggg ggc ttt ccc tat gag aaa gat ctc att gag gcc atc cga      288
   Glu Asn Gly Gly Phe Pro Tyr Glu Lys Asp Leu Ile Glu Ala Ile Arg
       80                  85                  90
   aga gcc agt aat gga gaa acc cta gaa aag atc acc aac agc cgt cct      336
   Arg Ala Ser Asn Gly Glu Thr Leu Glu Lys Ile Thr Asn Ser Arg Pro
   95                  100                 105                 110
   ccc tgc gtc atc ctg tga                                              354
   Pro Cys Val Ile Leu
                   115
<210> 2
   <211> 115
   <212> PRT
   <213> Homo sapiens
<400> 2
   Met Ser Gly Glu Pro Gly Gln Thr Ser Val Ala Pro Pro Pro Glu Glu
   1               5                   10                  15
   Val Glu Pro Gly Ser Gly Val Arg Ile Val Val Glu Tyr Cys Glu Pro
               20                  25                  30
   Cys Gly Phe Glu Ala Thr Tyr Leu Glu Leu Ala Ser Ala Val Lys Glu
           35                  40                  45
   Gln Tyr Pro Gly Ile Glu Ile Glu Ser Arg Leu Gly Gly Thr Gly Ala
       50                  55                  60
   Phe Glu Ile Glu Ile Asn Gly Gln Leu Val Phe Ser Lys Leu Glu Asn
   65                  70                  75                  80
   Gly Gly Phe Pro Tyr Glu Lys Asp Leu Ile Glu Ala Ile Arg Arg Ala
                   85                  90                  95
   Ser Asn Gly Glu Thr Leu Glu Lys Ile Thr Asn Ser Arg Pro Pro Cys
               100                 105                 110
   Val Ile Leu
           115
<210> 3
   <211> 627
   <212> DNA
   <213> Mus sp.
<400> 3
   gaatttagcg gccgcgaatt cgcccttcga ctggagcacg ggacactgac atggactgaa      60
   ggagtagaaa acatctctct cattagaggt tgatctttga ggaaaacagg gtgttgccta     120
   aaggatgaaa gtgttgagtc tgttgtacct gttgacagcc attcctggta tcctgtctga     180
   tgtacagctt caggagtcag gacctggcct cgtgaaacct tctcagtctc tgtctctcac     240
   ctgctctgtc actggctact ccatcaccag tggttatttc tggaactgga tccggcagtt     300
   tccagggaac aaactggaat ggatgggcta cataagctac gacggtagca ataactccaa     360
   cccatctctc aaaaatcgaa tctccttcac tcgtgacaca tctaagaacc agtttttcct     420
   gaagtttaat tctgtgacta ctgacgactc agctgcatat tactgtacaa gaggaactac 480
   ggggtttgct tactggggcc aagggactct ggtcactgtc tctgcagcca aaacgacacc 540
   cccatctgtc tatccactgg cccctggatc tgctgcccaa actaactcca agggcgaatt 600
   cgtttaaacc tgcaggacta gtccctt 627
<210> 4
   <211> 116
   <212> PRT
   <213> Mus sp.
<400> 4
   Asp Val Gln Leu Gln Glu Ser Gly Pro Gly Leu Val Lys Pro Ser Gln
   1               5                   10                  15
   Ser Leu Ser Leu Thr Cys Ser Val Thr Gly Tyr Ser Ile Thr Ser Gly
               20                  25                  30
   Tyr Phe Trp Asn Trp Ile Arg Gln Phe Pro Gly Asn Lys Leu Glu Trp
           35                  40                  45
   Met Gly Tyr Ile Ser Tyr Asp Gly Ser Asn Asn Ser Asn Pro Ser Leu
       50                  55                  60
   Lys Asn Arg Ile Ser Phe Thr Arg Asp Thr Ser Lys Asn Gln Phe Phe
   65                  70                  75                  80
   Leu Lys Phe Asn Ser Val Thr Thr Asp Asp Ser Ala Ala Tyr Tyr Cys
                   85                  90                  95
   Thr Arg Gly Thr Thr Gly Phe Ala Tyr Trp Gly Gln Gly Thr Leu Val
               100                 105                 110
   Thr Val Ser Ala
           115
<210> 5
   <211> 540
   <212> DNA
   <213> Mus sp.
<400> 5
   cgcgaattcg cccttcgact ggagcacgag gacactgaca tggactgaag gagtagaaaa      60
   attagctagg gaccaaaatt caaagacaga atggattttc aggtgcagat tttcagcttc     120
   ctgctaatca gtgcctcagt cagaatgtcc agaggacaaa ttgttctcac ccagtctcca     180
   gcaatcatgt ctgcatctcc aggggagaag gtcaccatat cctgcagtgc cagctcaagt     240
   gtaagttaca tgaactggta ccagcagaag ccaggatcct cccccaaacc ctggatttat     300
   cacacatcca acctggcttc tggagtccct gctcgcttca gtggcagtgg gtctgggacc     360
   tcttactctc tcacaatcag cagcatggag gctgaagatg ctgccactta ttactgccaa     420
   cagtatcata gttacccacc cacgttcgga ggggggacca agctggaaat aaaacgggct     480
   gatgctgcac caactgtatc catcttccca ccatccagtg agcaaagggc gaattcgttt     540
<210> 6
   <211> 106
   <212> PRT
   <213> Mus sp.
<400> 6
   Gln Ile Val Leu Thr Gln Ser Pro Ala Ile Met Ser Ala Ser Pro Gly
   1               5                   10                  15
   Glu Lys Val Thr Ile Ser Cys Ser Ala Ser Ser Ser Val Ser Tyr Met
               20                  25                  30
   Asn Trp Tyr Gln Gln Lys Pro Gly Ser Ser Pro Lys Pro Trp Ile Tyr
           35                  40                  45
   His Thr Ser Asn Leu Ala Ser Gly Val Pro Ala Arg Phe Ser Gly Ser
       50                  55                  60
   Gly Ser Gly Thr Ser Tyr Ser Leu Thr Ile Ser Ser Met Glu Ala Glu
   65                  70                  75                  80
   Asp Ala Ala Thr Tyr Tyr Cys Gln Gln Tyr His Ser Tyr Pro Pro Thr
                   85                  90                  95
   Phe Gly Gly Gly Thr Lys Leu Glu Ile Lys
               100                 105
<210> 7
   <211> 618
   <212> DNA
   <213> Mus sp.
<400> 7
   cgcgaattcg cccttcgact ggagcacgag gacactggac atggactgaa ggagtagaaa     60
   atctctctca ctggaggctg atttttgaag aaaggggttg tagcctaaaa gatgatggtg    120
   ttaagtcttc tgtacctgtt gacagccctt ccgggtatcc tgtcagaggt gcagcttcag    180
   gagtcaggac ctagcctcgt gaaaccttct cagactctgt ccctcacctg ttctgtcact    240
   ggcgactcca tcaccagtgg ttactggaac tggatccgga aattcccagg aaataaactt    300
   gaatacgtgg ggtacataag ctacagtggt ggcacttact acaatccatc tctcaaaagt    360
   cgaatctcca tcactcgaga cacatccaag aaccactact acctgcagtt gaattctgtg    420
   actactgagg acacagccac atattactgt gcaagaggtg cttactacgg gggggccttt    480
   tttccttact tcgatgtctg gggcgctggg accacggtca ccgtctcctc agccaaaacg    540
   acacccccat ctgtctatcc actggcccct ggatctgctg cccaaactaa ctccaagggc    600
   gaattcgttt aaacctgc                                                  618
<210> 8
   <211> 122
   <212> PRT
   <213> Mus sp.
<400> 8
   Glu Val Gln Leu Gln Glu Ser Gly Pro Ser Leu Val Lys Pro Ser Gln
   1               5                   10                  15
   Thr Leu Ser Leu Thr Cys Ser Val Thr Gly Asp Ser Ile Thr Ser Gly
               20                  25                  30
   Tyr Trp Asn Trp Ile Arg Lys Phe Pro Gly Asn Lys Leu Glu Tyr Val
           35                  40                  45
   Gly Tyr Ile Ser Tyr Ser Gly Gly Thr Tyr Tyr Asn Pro Ser Leu Lys
       50                  55                  60
   Ser Arg Ile Ser Ile Thr Arg Asp Thr Ser Lys Asn His Tyr Tyr Leu
   65                  70                  75                  80
   Gln Leu Asn Ser Val Thr Thr Glu Asp Thr Ala Thr Tyr Tyr Cys Ala
                   85                  90                  95
   Arg Gly Ala Tyr Tyr Gly Gly Ala Phe Phe Pro Tyr Phe Asp Val Trp
               100                 105                 110
   Gly Ala Gly Thr Thr Val Thr Val Ser Ser
           115                 120
<210> 9
   <211> 528
   <212> DNA
   <213> Mus sp.
<400> 9
   gaattcgccc ttcccctgga gcacgaggac actgacatgg actgaaggag tagaaaatca      60
   gttcctgcca ggacacagtt tagatatgag gttccaggtt caggttctgg ggctccttct    120
   gctctggata tcaggtgccc actgtgatgt ccagataacc cagtctccat cttttcttgc    180
   tgcatctcct ggagaaacca ttactattaa ttgcagggca agtaagtaca ttagcaaaca    240
   tttagtctgg tatcaggaga aacctggaga aactaaaaag cttcttatct actctggatc    300
   cactttgcaa tctggacttc catcaaggtt cagtggcagt ggatctggta cagatttcac    360
   tctcaccatc agtagcctgg agcctgaaga ttttgcaatg tattactgtc aacagcataa    420
   tgaatacccg ctcacgttcg gtgctgggac caagctggag ctgaaacggg ctgatgctgc    480
   accaactgta tccatcttcc caccatccag tgagcaaagg gcgaattc                 528
<210> 10
   <211> 107
   <212> PRT
   <213> Mus sp.
<400> 10
   Asp Val Gln Ile Thr Gln Ser Pro Ser Phe Leu Ala Ala Ser Pro Gly
   1               5                   10                  15
   Glu Thr Ile Thr Ile Asn Cys Arg Ala Ser Lys Tyr Ile Ser Lys His
               20                  25                  30
   Leu Val Trp Tyr Gln Glu Lys Pro Gly Glu Thr Lys Lys Leu Leu Ile
           35                  40                  45
   Tyr Ser Gly Ser Thr Leu Gln Ser Gly Leu Pro Ser Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Met Tyr Tyr Cys Gln Gln His Asn Glu Tyr Pro Leu
                   85                  90                  95
   Thr Phe Gly Ala Gly Thr Lys Leu Glu Leu Lys
               100                 105
<210> 11
   <211> 553
   <212> DNA
   <213> Homo sapiens
<400> 11
   gaattcgccc ttaattgcgg ccgcaaacca tgggatggag ctgtatcatc ctcttcttgg     60
   tagcaacagc tacaggcgcg cactccgagg tgcagctggt ggagtctggg ggaggcgtgg    120
   tccagcctgg gaggtccctg agactctcct gtgcagcgtc tggattcaac ttcggtacct    180
   atgccatgca ctgggtccgc caggctcaag gcaaggggct ggagtgggtg gcactcatat    240
   ggtatgatgg aactaagaaa tactatgcag actccgtgaa gggccgatac accatctcca    300
   gagacaattc ccagaacacg ctgtatctgc aaatgaacac cctgagagcc gacgacacgg    360
   ctgtgtatta ctgtgcgaaa tcaaaactcc aggggcgcgt tatagactac tggggccagg    420
   gaaccctggt caccgtctcc tcagcctcca ccaagggccc atcggtcttc cccctggcac    480
   cctcctccaa gagcacctct gggggcacag cggccctggg ctgcctggtc aaggactact    540
   taagggcgaa ttc                                                       553
<210> 12
   <211> 119
   <212> PRT
   <213> Homo sapiens
<400> 12
   Glu Val Gln Leu Val Glu Ser Gly Gly Gly Val Val Gln Pro Gly Arg
   1               5                   10                  15
   Ser Leu Arg Leu Ser Cys Ala Ala Ser Gly Phe Asn Phe Gly Thr Tyr
               20                  25                  30
   Ala Met His Trp Val Arg Gln Ala Gln Gly Lys Gly Leu Glu Trp Val
           35                  40                  45
   Ala Leu Ile Trp Tyr Asp Gly Thr Lys Lys Tyr Tyr Ala Asp Ser Val
       50                  55                  60
   Lys Gly Arg Tyr Thr Ile Ser Arg Asp Asn Ser Gln Asn Thr Leu Tyr
   65                  70                  75                  80
   Leu Gln Met Asn Thr Leu Arg Ala Asp Asp Thr Ala Val Tyr Tyr Cys
                   85                  90                  95
   Ala Lys Ser Lys Leu Gln Gly Arg Val Ile Asp Tyr Trp Gly Gln Gly
               100                 105                 110
   Thr Leu Val Thr Val Ser Ser
           115
<210> 13
   <211> 528
   <212> DNA
<213> Homo sapiens
<400> 13
   gaattcgccc ttaattgcgg ccgcaaacat gggatggagc tgtatcatcc tcttcttggt      60
   agcaacagct acaggcgtgc actccgacat ccagatgacc cagtctccag actccctggc     120
   tgtgtctctg ggcgagaggg ccaccatcaa ctgcaagtcc agccagagtg ttttatacag     180
   ctccaacaat aagaactact tagcttggta ccagcagaaa ccaggacagc ctcctaagct     240
   gctcatttac tgggcatcta cccgggaatc cggggtccct gaccgattca gtggcagcgg     300
   gtctgggaca gatttcactc tcaccatcag cagcctgcag gctgaagatg tggcagttta     360
   ttactgtcag caatattata gtactcctct gtggacgttc ggccaaggga ccaagctcga     420
   gatcaaacga actgtggctg caccatctgt cttcatcttc ccgccatctg atgagcagtt     480
   gaaatctgga actgcctctg ttgtgtgcct gctgaaaagg gcgaattc                 528
<210> 14
   <211> 113
   <212> PRT
   <213> Homo sapiens
<400> 14
   Ile Gln Met Thr Gln Ser Pro Asp Ser Leu Ala Val Ser Leu Gly Glu
   1               5                   10                  15
   Arg Ala Thr Ile Asn Cys Lys Ser Ser Gln Ser Val Leu Tyr Ser Ser
               20                  25                  30
   Asn Asn Lys Asn Tyr Leu Ala Trp Tyr Gln Gln Lys Pro Gly Gln Pro
           35                  40                  45
   Pro Lys Leu Leu Ile Tyr Trp Ala Ser Thr Arg Glu Ser Gly Val Pro
       50                  55                  60
   Asp Arg Phe Ser Gly Ser Gly Ser Gly Thr Asp Phe Thr Leu Thr Ile
   65                  70                  75                  80
   Ser Ser Leu Gln Ala Glu Asp Val Ala Val Tyr Tyr Cys Gln Gln Tyr
                   85                  90                  95
   Tyr Ser Thr Pro Leu Trp Thr Phe Gly Gln Gly Thr Lys Leu Glu Ile
               100                 105                 110
   Lys
<210> 15
   <211> 29
   <212> DNA
   <213> Artificial
<220>
   <223> Primer used in expression of selected antibodies as secreted human IgG1
<400> 15
   attaggatcc ggtcaccgtc tcctcagcc 29
<210> 16
   <211> 31
   <212> DNA
   <213> Artificial
<220>
   <223> Primer used in expression of selected antibodies as secreted human IgG1
<400> 16
   attagtcgac tcatttaccc ggagacaggg a 31
<210> 17
   <211> 1084
   <212> DNA
   <213> Homo sapiens
<400> 17
   gcggccgcaa accatgggat ggagctgtat catcctcttc ttggtagcaa cagctacagg      60
   cgcgcatatg gtcaccgtct cctcagcctc caccaagggc ccatcggtct tccccctggc     120
   accctcctcc aagagcacct ctgggggcac agcggccctg ggctgcctgg tcaaggacta     180
   cttccccgaa ccggtgacgg tgtcgtggaa ctcaggcgcc ctgaccagcg gcgtgcacac     240
   cttcccggct gtcctacagt cctcaggact ctactccctc agcagcgtcg tgaccgtgcc     300
   ctccagcagc ttgggcaccc agacctacat ctgcaacgtg aatcacaagc ccagcaacac     360
   caaggtggac aagaaagttg agcccaaatc ttgtgacaaa actcacacat gcccaccgtg     420
   cccagcacct gaactcctgg ggggaccgtc agtcttcctc ttccccccaa aacccaagga     480
   caccctcatg atctcccgga cccctgaggt cacatgcgtg gtggtggacg tgagccacga     540
   agaccctgag gtcaagttca actggtacgt ggacggcgtg gaggtgcata atgccaagac     600
   aaagccgcgg gaggagcagt acaacagcac gtaccgtgtg gtcagcgtcc tcaccgtcct     660
   gcaccaggac tggctgaatg gcaaggagta caagtgcaag gtctccaaca aagccctccc     720
   agcccccatc gagaaaacca tctccaaagc caaagggcag ccccgagaac cacaggtgta     780
   caccctgccc ccatcccggg atgagctgac caagaaccag gtcagcctga cctgcctggt     840
   caaaggcttc tatcccagcg acatcgccgt ggagtgggag agcaatgggc agccggagaa     900
   caactacaag accacgcctc ccgtgctgga ctccgacggc tccttcttcc tctacagcaa     960
   gctcaccgtg gacaagagca ggtggcagca ggggaacgtc ttctcatgct ccgtgatgca    1020
   tgaggctctg cacaaccact acacgcagaa gagcctctcc ctgtctccgg gtaaatgagt    1080
   cgac                                                                 1084
<210> 18
   <211> 413
   <212> DNA
   <213> Homo sapiens
<400> 18
   gcggccgcaa accatgggat ggagctgtat catcctcttc ttggtagcaa cagctacagg      60
   cgtgcacttg actcgagatc aaacgaactg tggctgcacc atctgtcttc atcttcccgc     120
   catctgatga gcagttgaaa tctggaactg cctctgttgt gtgcctgctg aataacttct     180
   atcccagaga ggccaaagta cagtggaagg tggataacgc cctccaatcg ggtaactccc     240
   aggagagtgt cacagagcag gacagcaagg acagcaccta cagcctcagc agcaccctga     300
   cgctgagcaa agcagactac gagaaacaca aagtctacgc ctgcgaagtc acccatcagg     360
   gcctgagctc gcccgtcaca aagagcttca acaggggaga gtgttaggtc gac            413
<210> 19
   <211> 422
   <212> DNA
   <213> Homo sapiens
<400> 19
   gcggccgcaa accatgggat ggagctgtat catcctcttc ttggtagcaa cagctacagg      60
   cgtgcacttg actcgagaag cttaccgtcc tacgaactgt ggctgcacca tctgtcttca     120
   tcttcccgcc atctgatgag cagttgaaat ctggaactgc ctctgttgtg tgcctgctga     180
   ataacttcta tcccagagag gccaaagtac agtggaaggt ggataacgcc ctccaatcgg     240
   gtaactccca ggagagtgtc acagagcagg acagcaagga cagcacctac agcctcagca     300
   gcaccctgac gctgagcaaa gcagactacg agaaacacaa agtctacgcc tgcgaagtca     360
   cccatcaggg cctgagctcg cccgtcacaa agagcttcaa caggggagag tgttaggtcg     420
   ac                                                                    422
<210> 20
   <211> 29
   <212> DNA
   <213> Artificial
<220>
   <223> IgG2-F primer
<400> 20
   attaggatcc ggtcaccgtc tcctcagcc 29
<210> 21
   <211> 31
   <212> DNA
   <213> Artificial
<220>
   <223> IgI2-R primer
<400> 21
   attagtcgac tcatttaccc ggagacaggg a 31
<210> 22
   <211> 29
   <212> DNA
   <213> Artificial
<220>
   <223> IgG3-F primer
<400> 22
   attaggatcc ggtcaccgtc tcctcagct 29
<210> 23
   <211> 31
   <212> DNA
   <213> Artificial
<220>
   <223> IgG3-R primer
<400> 23
   attagtcgac tcatttaccc ggagacaggg a 31
<210> 24
   <211> 29
   <212> DNA
   <213> Artificial
<220>
   <223> IgG4-F primer
<400> 24
   attaggatcc ggtcaccgtc tcctcagct 29
<210> 25
   <211> 31
   <212> DNA
   <213> Artificial
<220>
   <223> IgG4-R primer
<400> 25
   attagtcgac tcatttaccc agagacaggg a 31
<210> 26
   <211> 30
   <212> DNA
   <213> Artificial
<220>
   <223> IgA1-F primer
<400> 26
   attaggatcc ggtcaccgtc tcctcagcat 30
<210> 27
   <211> 31
   <212> DNA
   <213> Artificial
<220>
   <223> IgA1-R primer
<400> 27
   attagtcgac tcagtagcag gtgccgtcca c 31
<210> 28
   <211> 30
   <212> DNA
   <213> Artificial
<220>
   <223> IgA2-F primer ,
<400> 28
   attaggatcc ggtcaccgtc tcctcagcat 30
<210> 29 <211> 31
   <212> DNA
   <213> Artificial
<220>
   <223> IgA2-R primer
<400> 29
   attagtcgac tcagtagcag gtgccgtcca c 31
<210> 30
   <211> 30
   <212> DNA
   <213> Artificial
<220>
   <223> IgD-F primer
<400> 30
   attaggatcc ggtcaccgtc tcctcagcac 30
<210> 31
   <211> 31
   <212> DNA
   <213> Artificial
<220>
   <223> IgD-R primer
<400> 31
   attagtcgac tcatttcatg gggccatggt c 31
<210> 32 <211> 29
   <212> DNA
   <213> Artificial
<220>
   <223> IgE-F primer
<400> 32
   attaggatcc ggtcaccgtc tcctcagcc 29
<210> 33
   <211> 31
   <212> DNA
   <213> Artificial
<220>
   <223> IgE-R
<400> 33
   attagtcgac tcatttaccg ggatttacag a 31
<210> 34
   <211> 29
   <212> DNA
   <213> Artificial
<220>
   <223> IgM-F
<400> 34
   attaggatcc ggtcaccgtc tcctcaggg 29
<210> 35
   <211> 31
   <212> DNA
   <213> Artificial
<220>
   <223> IgM-R
<400> 35
   attagtcgac tcagtagcag gtgccagctg t 31
<210> 36
   <211> 6
   <212> DNA
   <213> Artificial
<220>
   <223> BssHII site
<400> 36
   gcgcgc 6
<210> 37
   <211> 7
   <212> DNA
   <213> Artificial
<220>
   <223> BsteII site
<400> 37
   ggtcacc 7
<210> 38
   <211> 6
   <212> DNA
   <213> Artificial
<220>
   <223> ApaL1 site
<400> 38
   gtgcac 6
<210> 39
   <211> 6
   <212> DNA
   <213> Artificial
<220>
   <223> Xho1 site
<400> 39
   ctcgag 6
<210> 40
   <211> 39
   <212> DNA
   <213> Artificial
<220>
   <223> 1F2VK forward primer
<400> 40
   tatccgtgca ctcccaaatt gttctcaccc agtctccag 39
<210> 41
   <211> 29
   <212> DNA
   <213> Artificial
<220>
   <223> 1F2VK reverse primer
<400> 41
   atattctcga gcttggtccc ccctccgaa 29
<210> 42
   <211> 6
   <212> DNA
   <213> Artificial
<220>
   <223> ApaL1 site
<400> 42
   gtgcac 6
<210> 43
   <211> 6
   <212> DNA
   <213> Artificial
<220>
   <223> Xho1 site
<400> 43
   ctcgag 6
<210> 44
   <211> 41
   <212> DNA
   <213> Artificial
<220>
   <223> 1F2VH forward primer
<400> 44
   tataagcgcg cactccgatg tacagcttca ggagtcagga c 41
<210> 45
   <211> 28
   <212> DNA
   <213> Artificial
<220>
   <223> 1F2VH reverse primer
<400> 45
   atattggtga ccagagtccc ttggcccc 28
<210> 46
   <211> 40
   <212> DNA
   <213> Artificial
<220>
   <223> 1B3VK forward primer
<400> 46
   tatccgtgca ctccgatgtc cagataaccc agtctccatc 40
<210> 47
   <211> 29
   <212> DNA
   <213> Artificial
<220>
   <223> 1B3VK reverse primer
<400> 47
   atattctcga gcttggtccc agcaccgaa 29
<210> 48
   <211> 41
   <212> DNA
   <213> Artificial
<220>
   <223> 1B3VH forward primer
<400> 48
   tataagcgcg cactccgagg tgcagcttca ggagtcagga c 41
<210> 49
   <211> 24
   <212> DNA
   <213> Artificial
<220>
   <223> 1B3VH reverse primer
<400> 49
   atattggtga ccgtggtccc agcg 24
<210> 50
   <211> 935
   <212> DNA
   <213> Homo sapiens
<400> 50
   tcttctctct gcagagcgca aatgttgtgt cgagtgccca ccgtgcccag gtaagccagc 60
   ccaggcctcg ccctccagct caaggcggga caggtgccct agagtagcct gcatccaggg 120
   acaggcccca gctgggtgct gacacgtcca cctccatctc ttcctcagca ccacctgtgg 180
   caggaccgtc agtcttcctc ttccccccaa aacccaagga caccctcatg atctcccgga 240
   cccctgaggt cacgtgcgtg gtggtggacg tgagccacga agaccccgag gtccagttca 300
   actggtacgt ggacggcgtg gaggtgcata atgccaagac aaagccacgg gaggagcagt 360
   tcaacagcac gttccgtgtg gtcagcgtcc tcaccgttgt gcaccaggac tggctgaacg 420
   gcaaggagta caagtgcaag gtctccaaca aaggcctccc agcccccatc gagaaaacca 480
   tctccaaaac caaaggtggg acccgcgggg tatgagggcc acatggacag acggcggctt 540
   cggcccaccc tctgccctgg gagtgaccgc tgtgccaacc tctgtcccta cagggcagcc 600
   ccgagaacca caggtgtaca ccctgccccc atcccgggag gagatgacca agaaccaggt 660
   cagcctgacc tgcctggtca aaggcttcta ccccagcgac atcgccgtgg agtgggagag 720
   caatgggcag ccggagaaca actacaagac cacacctccc atgctggact ccgacggctc 780
   cttcttcctc tacagcaagc tcaccgtgga caagagcagg tggcagcagg ggaacgtctt 840
   ctcatgctcc gtgatgcatg aggctctgca caaccactac acgcagaaga gcctctccct 900
   gtctccgggt aaatgagtgc cacggccggc aagcc 935
<210> 51
   <211> 935
   <212> DNA
   <213> Homo sapiens
<400> 51
   tcttctctct gcagagcgca aatgttgtgt cgagtgccca ccgtgcccag gtaagccagc 60
   ccaggcctcg ccctccagct caaggcggga caggtgccct agagtagcct gcatccaggg 120
   acaggcccca gctgggtgct gacacgtcca cctccatctc ttcctcagca ccacctgtgg 180
   caggaccgtc agtcttcctc ttccccccaa aacccaagga caccctcatg atctcccgga 240
   cccctgaggt cacgtgcgtg gtggtggacg tgagccacga agaccccgag gtccagttca 300
   actggtacgt ggacggcatg gaggtgcata atgccaagac aaagccacgg gaggagcagt 360
   tcaacagcac gttccgtgtg gtcagcgtcc tcaccgtcgt gcaccaggac tggctgaacg 420
   gcaaggagta caagtgcaag gtctccaaca aaggcctccc agcccccatc gagaaaacca 480
   tctccaaaac caaaggtggg acccgcgggg tatgagggcc acatggacag acggcggctt 540
   cggcccaccc tctgccctgg gagtgaccgc tgtgccaacc tctgtcccta cagggcagcc 600
   ccgagaacca caggtgtaca ccctgccccc atcccgggag gagatgacca agaaccaggt 660
   cagcctgacc tgcctggtca aaggcttcta ccccagcgac atcgccgtgg agtgggagag 720
   caatgggcag ccggagaaca actacaagac cacacctccc atgctggact ccgacggctc 780
   cttcttcctc tacagcaagc tcaccgtgga caagagcagg tggcagcagg ggaacgtctt 840
   ctcatgctcc gtgatgcatg aggctctgca caaccactac acacagaaga gcctctccct 900
   gtctccgggt aaatgagtgc cacggccggc aagcc 935
<210> 52
   <211> 1780
   <212> DNA
   <213> Homo sapiens
<400> 52
   ctcgaggacc tgctcttagg ttcagaagcg aacctcacgt gcacactgac cggcctgaga     60
   gatgcctctg gtgccacctt cacctggacg ccctcaagtg ggaagagcgc tgttcaagga    120
   ccacctgagc gtgacctctg tggctgctac agcgtgtcca gtgtcctgcc tggctgtgcc    180
   cagccatgga accatgggga gaccttcacc tgcactgctg cccaccccga gttgaagacc    240
   ccactaaccg ccaacatcac aaaatccggt gggtccagac cctgctcggg gccctgctca    300
   gtgctctggt ttgcaaagca tattcctggc ctgcctcctc cctcccaatc ctgggctcca    360
   gtgctcatgc caagtacaca gggaaactga ggcaggctga ggggccagga cacagcccag    420
   ggtgcccacc agagcagagg ggctctctca tcccctgccc agccccctga cctggctctc    480
   taccctccag gaaacacatt ccggcccgag gtccacctgc tgccgccgcc gtcggaggag    540
   ctggccctga acgagctggt gacgctgacg tgcctggcac gtggcttcag ccccaaggat    600
   gtgctggttc gctggctgca ggggtcacag gagctgcccc gcgagaagta cctgacttgg    660
   gcatcccggc aggagcccag ccagggcacc accacctacg ctgtaaccag catactgcgc    720
   gtggcagctg aggactggaa gaagggggag accttctcct gcatggtggg ccacgaggcc    780
   ctgccgctgg ccttcacaca gaagaccatc gaccgcatgg cgggtaaacc cacccacatc    840
   aatgtgtctg ttgtcatggc ggaggcggat ggcacctgct actgagccgc ccgcctgtcc    900
   ccacccctga ataaactcca tgctccccca agcagcccca cgcttccatc cggcgcctgt    960
   ctgtccatcc tcagggtctc agcacttggg aaagggccag ggcatggaca gggaagaata   1020
   ccccctgccc tgagcctcgg ggggcccctg gcacccccat gagactttcc accctggtgt  1080
   gagtgtgagt tgtgagtgtg agagtgtgtg gtgcaggagg cctcgctggt gtgagatctt   1140
   aggtctgcca aggcaggcac agcccaggat gggttctgag agacgcacat gccccggaca   1200
   gttctgagtg agcagtggca tggccgtttg tccctgagag agccgcctct ggctgtagct   1260
   gggagggaat agggagggta aaaggagcag gctagccaag aaaggcgcag gtagtggcag   1320
   gagtggcgag ggagtgaggg gctggactcc agggccccac tgggaggaca agctccagga   1380
   gggccccacc accctagtgg gtgggcctca ggacgtccca ctgacgcatg caggaagggg   1440
   cacctcccct taaccacact gctctgtacg gggcacgtgg gcacacatgc acactcacac   1500
   tcacatatac gcctgagccc tgcaggagtg gaacgttcac agcccagacc cagttccaga   1560
   aaagccaggg gagtcccctc ccaagccccc aagctcagcc tgctccccca ggcccctctg    1620
   gcttccctgt gtttccactg tgcacagctc agggaccaac tccacagacc cctcccaggc    1680
   agcccctgct ccctgcctgg ccaagtctcc catcccttcc taagcccaac taggacccaa    1740
   agcatagaca gggaggggcc gcgtggggtg gcatcagaag                          1780
<210> 53
   <211> 220
   <212> PRT
   <213> Homo sapiens
<400> 53
   Leu Glu Asp Leu Leu Leu Gly Ser Glu Ala Asn Leu Thr Cys Thr Leu
   1               5                   10                  15
   Thr Gly Leu Arg Asp Ala Ser Gly Ala Thr Phe Thr Trp Thr Pro Ser
               20                  25                  30
   Ser Gly Lys Ser Ala Val Gln Gly Pro Pro Glu Arg Asp Leu Cys Gly
           35                  40                  45
   Cys Tyr Ser Val Ser Ser Val Leu Pro Gly Cys Ala Gln Pro Trp Asn
       50                  55                  60
   His Gly Glu Thr Phe Thr Cys Thr Ala Ala His Pro Glu Leu Lys Thr
   65                  70                  75                  80
   Pro Leu Thr Ala Asn Ile Thr Lys Ser Gly Asn Thr Phe Arg Pro Glu
                   85                  90                  95
   Val His Leu Leu Pro Pro Pro Ser Glu Glu Leu Ala Leu Asn Glu Leu
               100                 105                 110
   Val Thr Leu Thr Cys Leu Ala Arg Gly Phe Ser Pro Lys Asp Val Leu
           115                 120                 125
   Val Arg Trp Leu Gln Gly Ser Gln Glu Leu Pro Arg Glu Lys Tyr Leu
       130                 135                 140
   Thr Trp Ala Ser Arg Gln Glu Pro Ser Gln Gly Thr Thr Thr Tyr Ala
   145                 150                 155                 160
   Val Thr Ser Ile Leu Arg Val Ala Ala Glu Asp Trp Lys Lys Gly Glu
                   165                 170                 175
   Thr Phe Ser Cys Met Val Gly His Glu Ala Leu Pro Leu Ala Phe Thr
               180                 185                 190
   Gln Lys Thr Ile Asp Arg Met Ala Gly Lys Pro Thr His Ile Asn Val
           195                 200                 205
   Ser Val Val Met Ala Glu Ala Asp Gly Thr Cys Tyr
       210                 215                 220
<210> 54
   <211> 1059
   <212> DNA
   <213> Homo sapiens
<400> 54
   gcaagcttga ccagccccaa ggtcttcccg ctgagcctct gcagcaccca gccagatggg      60
   aacgtggtca tcgcctgcct ggtccagggc ttcttccccc aggagccact cagtgtgacc     120
   tggagcgaaa gcggacaggg cgtgaccgcc agaaacttcc cacccagcca ggatgcctcc     180
   ggggacctgt acaccacgag cagccagctg accctgccgg ccacacagtg cctagccggc     240
   aagtccgtga catgccacgt gaagcactac acgaatccca gccaggatgt gactgtgccc     300
   tgcccagttc cctcaactcc acctacccca tctccctcaa ctccacctac cccatctccc     360
   tcatgctgcc acccccgact gtcactgcac cgaccggccc tcgaggacct gctcttaggt     420
   tcagaagcga acctcacgtg cacactgacc ggcctgagag atgcctcagg tgtcaccttc     480
   acctggacgc cctcaagtgg gaagagcgct gttcaaggac cacctgaccg tgacctctgt     540
   ggctgctaca gcgtgtccag tgtcctgtcg ggctgtgccg agccatggaa ccatgggaag     600
   accttcactt gcactgctgc ctaccccgag tccaagaccc cgctaaccgc caccctctca     660
   aaatccggaa acacattccg gcccgaggtc cacctgctgc cgccgccgtc ggaggagctg     720
   gccctgaacg agctggtgac gctgacgtgc ctggcacgtg gcttcagccc caaggatgtg     780
   ctggttcgct ggctgcaggg gtcacaggag ctgccccgcg agaagtacct gacttgggca     840
   tcccggcagg agcccagcca gggcaccacc accttcgctg tgaccagcat actgcgcgtg     900
   gcagccgagg actggaagaa gggggacacc ttctcctgca tggtgggcca cgaggccctg     960
   ccgctggcct tcacacagaa gaccatcgac cgcttggcgg gtaaacccac ccatgtcaat    1020
   gtgtctgttg tcatggcgga ggtggacggc acctgctac                           1059
<210> 55
   <211> 353
   <212> PRT
   <213> Homo sapiens
<400> 55
   Ala Ser Leu Thr Ser Pro Lys Val Phe Pro Leu Ser Leu Cys Ser Thr
   1 5 10 15
   Gln Pro Asp Gly Asn Val Val Ile Ala Cys Leu Val Gln Gly Phe Phe
   20 25 30
   Pro Gln Glu Pro Leu Ser Val Thr Trp Ser Glu Ser Gly Gln Gly Val
   35 40 45
   Thr Ala Arg Asn Phe Pro Pro Ser Gln Asp Ala Ser Gly Asp Leu Tyr
   50 55 60
   Thr Thr Ser Ser Gln Leu Thr Leu Pro Ala Thr Gln Cys Leu Ala Gly
   65 70 75 80
   Lys Ser Val Thr Cys His Val Lys His Tyr Thr Asn Pro Ser Gln Asp
   85 90 95
   Val Thr Val Pro Cys Pro Val Pro Ser Thr Pro Pro Thr Pro Ser Pro
   100 105 110
   Ser Thr Pro Pro Thr Pro Ser Pro Ser Cys Cys His Pro Arg Leu Ser
   115 120 125
   Leu His Arg Pro Ala Leu Glu Asp Leu Leu Leu Gly Ser Glu Ala Asn
   130 135 140
   Leu Thr Cys Thr Leu Thr Gly Leu Arg Asp Ala Ser Gly Val Thr Phe
   145 150 155 160
   Thr Trp Thr Pro Ser Ser Gly Lys Ser Ala Val Gln Gly Pro Pro Asp
   165 170 175
   Arg Asp Leu Cys Gly Cys Tyr Ser Val Ser Ser Val Leu Ser Gly Cys
   180 185 190
   Ala Glu Pro Trp Asn His Gly Lys Thr Phe Thr Cys Thr Ala Ala Tyr
   195 200 205
   Pro Glu Ser Lys Thr Pro Leu Thr Ala Thr Leu Ser Lys Ser Gly Asn
   210 215 220
   Thr Phe Arg Pro Glu Val His Leu Leu Pro Pro Pro Ser Glu Glu Leu
   225 230 235 240
   Ala Leu Asn Glu Leu Val Thr Leu Thr Cys Leu Ala Arg Gly Phe Ser
   245 250 255
   Pro Lys Asp Val Leu Val Arg Trp Leu Gln Gly Ser Gln Glu Leu Pro
   260 265 270
   Arg Glu Lys Tyr Leu Thr Trp Ala Ser Arg Gln Glu Pro Ser Gln Gly
   275 280 285
   Thr Thr Thr Phe Ala Val Thr Ser Ile Leu Arg Val Ala Ala Glu Asp
   290 295 300
   Trp Lys Lys Gly Asp Thr Phe Ser Cys Met Val Gly His Glu Ala Leu
   305 310 315 320
   Pro Leu Ala Phe Thr Gln Lys Thr Ile Asp Arg Leu Ala Gly Lys Pro
   325 330 335
   Thr His Val Asn Val Ser Val Val Met Ala Glu Val Asp Gly Thr Cys
   340 345 350
   Tyr
<210> 56
   <211> 366
   <212> DNA
   <213> Artificial
<220>
   <223> Chimeric mouse/human 141D10 VH H732
<400> 56
   caggtgcagc tgcaggagtc gggcccagga ctggtgaagc ctccggagac cctgtccctc      60
   acctgcaatg tctctggtgg ctctatcggt agatactatt ggaactggat ccgacagtcc     120
   ccagggaagg ggctggagtg gattggccat atccattaca gtgggagcac catctaccat     180
   ccctccctca agagtcgagt cagcatatcg ctggacacgt ccaagaacca ggtctccctg     240
   aagttgagtt ctgtgaccgc tgcggacacg gccgtgtatt actgtgcacg aggtgcttac     300
   tacggggggg ccttttttcc ttacttcgat gtctggggcc aagggaccac ggtcaccgtc     360
   tcctca                                                                366
<210> 57
   <211> 122
   <212> PRT
   <213> Artificial
<220>
   <223> Chimeric mouse/human 141D10 VH H732
<400> 57
   Gln Val Gln Leu Gln Glu Ser Gly Pro Gly Leu Val Lys Pro Pro Glu
   1               5                   10                  15
   Thr Leu Ser Leu Thr Cys Asn Val Ser Gly Gly Ser Ile Gly Arg Tyr
               20                  25                  30
   Tyr Trp Asn Trp Ile Arg Gln Ser Pro Gly Lys Gly Leu Glu Trp Ile
           35                  40                  45
   Gly His Ile His Tyr Ser Gly Ser Thr Ile Tyr His Pro Ser Leu Lys
       50                  55                  60
   Ser Arg Val Ser Ile Ser Leu Asp Thr Ser Lys Asn Gln Val Ser Leu
   65                  70                  75                  80
   Lys Leu Ser Ser Val Thr Ala Ala Asp Thr Ala Val Tyr Tyr Cys Ala
                   85                  90                  95
   Arg Gly Ala Tyr Tyr Gly Gly Ala Phe Phe Pro Tyr Phe Asp Val Trp
               100                 105                 110
   Gly Gln Gly Thr Thr Val Thr Val Ser Ser
           115                 120
<210> 58
   <211> 321
   <212> DNA
   <213> Artificial
<220>
   <223> Chimeric mouse/human UH8 VK L120
<400> 58
   gacatccaga tgacccagtc tccatcctcc ctgtctgcat ctatgggaga cagagtcacc      60
   atcacttgcc gggcgagtca gggcattagg aatcatttag cctggtatca gcagaaacca     120
   gggaaagctc ctaatctcct gatctctgct gcatccactt tgcaatcagg ggtcccaact     180
   cgattcagtg gcagtggatc tggaacagat ttcactctca ccatcagcag cctgcagcct     240
   gaagactctg caacttatta ctgccaacag tataatcggt accccctcac tttcggccaa      300
   gggaccaagc tcgagatcaa a                                                321
<210> 59
   <211> 107
   <212> PRT
   <213> Artificial
<220>
   <223> Chimeric mouse/human UH8 VK L120
<400> 59
   Asp Ile Gln Met Thr Gln Ser Pro Ser Ser Leu Ser Ala Ser Met Gly
   1               5                   10                  15
   Asp Arg Val Thr Ile Thr Cys Arg Ala Ser Gln Gly Ile Arg Asn His
               20                  25                  30
   Leu Ala Trp Tyr Gln Gln Lys Pro Gly Lys Ala Pro Asn Leu Leu Ile
           35                  40                  45
   Ser Ala Ala Ser Thr Leu Gln Ser Gly Val Pro Thr Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Ser Ala Thr Tyr Tyr Cys Gln Gln Tyr Asn Arg Tyr Pro Leu
                   85                  90                  95
   Thr Phe Gly Gln Gly Thr Lys Leu Glu Ile Lys
               100                 105
<210> 60
   <211> 369
   <212> DNA
   <213> Artificial
<220>
   <223> Chimeric mouse/human MSH3 VH (H835)
<400> 60
   caggtgcagc tgcaggagtc gggaggaggc ttagttcagc ctggggggtc cctgagactc      60
   tcttgtgcag gctctggatt caccttcagt agttactgga tgcactgggt ccgccaagct    120
   ccagggaagg ggctggtgtg ggtctcacgt attgacactg atgggagtac cacaacctac    180
   gcggactccg tgaagggccg attcaccatc tccagagaca acgccaagaa cacactgtat    240
   ctgcaaatga acagcctgag agtcgaggac acggccgtgt attactgtgc acgaggtgct    300
   tactacgggg gggccttttt tccttacttc gatgtctggg gccaagggac cacggtcacc    360
   gtctcctca                                                            369
<210> 61
   <211> 123
   <212> PRT
   <213> Artificial
<220>
   <223> Chimeric mouse/human MSH3 VH (H835)
<400> 61
   Gln Val Gln Leu Gln Glu Ser Gly Gly Gly Leu Val Gln Pro Gly Gly
   1               5                   10                  15
   Ser Leu Arg Leu Ser Cys Ala Gly Ser Gly Phe Thr Phe Ser Ser Tyr
               20                  25                  30
   Trp Met His Trp Val Arg Gln Ala Pro Gly Lys Gly Leu Val Trp Val
           35                  40                  45
   Ser Arg Ile Asp Thr Asp Gly Ser Thr Thr Thr Tyr Ala Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Asn Ser Leu Arg Val Glu Asp Thr Ala Val Tyr Tyr Cys
                   85                  90                  95
   Ala Arg Gly Ala Tyr Tyr Gly Gly Ala Phe Phe Pro Tyr Phe Asp Val
               100                 105                 110
   Trp Gly Gln Gly Thr Thr Val Thr Val Ser Ser
           115                 120

## Description

### BACKGROUND OF THE INVENTION

Cell growth is a carefully regulated process which responds to specific needs of the body. Occassionally, the intricate, and highly regulated controls dictating the rules for cellular division break down. When this occurs, the cell begins to grow and divide independently of its homeostatic regulation resulting in a condition commonly referred to as cancer. In fact, cancer is the second leading cause of death among Americans aged 25-44.

Current therapies for cancer include chemotherapy and radiation therapy. Chemotherapeutic drugs kill cancer cells mainly by inducing apoptosis (Fisher, D.E., Cell 78:539-542 (1994); Fung, C.Y., and D.E. Fisher, J. Clin. Oncol. 13:801-807 (1995); Lowe, S.W., et al., Cell 74:957-967 (1993)). Radiation therapy kills cancer cells by inducing apoptosis and by other mechanisms. However, chemotherapy and radiation therapy do not kill all cells in a given tumor, and cells that survive such treatment continue to grow. Thus, these treatments are often insufficient for eradicating an entire tumor. There is therefore a need for improved therapeutic methods of treating cancer.

Immunotherapeutic strategies for cancer have also been developed that target surface membrane markers differentially expressed in tumor cells using antibodies (e.g., U.S. Patent number: 5,770,195, "Monoclonal
Antibodies to the HER2 Receptor", Filed: May 23, 1995; Issued, Jun. 23, 1998). Many antigens differentially expressed in tumors are, however, not exposed on the surface of tumor cells. As a result, such

intracellular antigens are not suitable as targets for antibody-based therapeutics. Therefore, there is a need for additional targets for immunotherapeutic methods of treating cancer.
US 2002/0155447 discloses the human C35 gene, and the use of anti-C35 antibodies in tumor therapy. WO 03/104428 discloses anti-C35 antibodies and their therapeutic use to kill cancer cells.

### SUMMARY OF THE INVENTION

The invention provides an antibody and use as defined in the appended claims.

### Methods Of Killing Cancer Cells

Disclosed herein is a method of killing cancer cells by administering an effective amount of an apoptosis-inducing therapy, and administering an effective amount of an antibody conjugate or antibody complex that binds a cancer-associated antigen which is expressed intracellularly in cancer cells, but which becomes exposed on the cell surface in cancer cells that are undergoing apoptosis. The timing of administration of the apoptosis-inducing therapy and the antibody conjugate or antibody complex is planned such that the antibody reaches the cancer cell at the time that apoptosis is being or has been induced. In a preferred embodiment, the cancer associated antigen is a prenylated protein which, although normally expressed intracellulary, become exposed on the cell surface in tumor cells that are undergoing apoptosis. In another preferred embodiment, the prenylated protein is the C35 antigen. The antibody is conjugated to or complexed with a toxin, which insures that the cell to which the antibody binds will be killed, and/or surrounding cancer cells that are exposed to the toxin are killed. In one embodiment, the toxin is a radioisotope.

In one embodiment, the method involves administering a chemotherapeutic agent followed by or simultaneous with an antibody or fragment or variant thereof that is conjugated to a radioactive agent.

In another embodiment, the method involves administering an antibody that is not conjugated to or complexed with a toxin, and cells which bind the antibody die.

The method may be performed in vitro or in vivo, and may be used as a therapeutic in a patient, including a mammal such as a human.

### Antibodies Against C35 And Methods Of Using C35 Antibodies

Disclosed herein are antibodies that bind C35 5 polypeptides. This encompasses antibodies (including molecules comprising, or alternatively consisting of, antibody fragments or variants thereof) that immunospecifically bind to a C35 polypeptide or polypeptide fragment or variant of a C35 polypeptide such as that of SEQ ID NO:2.

The present inventors have generated mouse and human antibodies that immunospecifically bind one or more C35 polypeptides (e.g., SEQ ID NO:2) and polynucleotides encoding VH and VL regions from these antibodies. Disclosed herein are polynucleotides,
inlcuding those set forth in SEQ ID NO:s 56, 58, and 60, and those listed in Tables 2 and 3 below, which were deposited with the American Type Culture Collection ("ATCC") on the dates listed in Tables 2 and 3 and given the ATCC Deposit Numbers identified in Tables 2 and 3. The ATCC is located at 10801 University Boulevard, Manassas, VA 20110-2209, USA. The ATCC deposit was made pursuant to the terms of the Budapest Treaty on the international recognition of the deposit of microorganisms for purposes of patent procedure.

Disclosed herein are the deposited nucleotide clones that encode VH and VL regions that immunospecifically bind one or more C35 polypeptides (e.g., SEQ ID NO:2), cells comprising the deposited polynucleotides, antibodies comprising VH and/or VL regions encoded by the deposited polynucleotides, polynucleotides encoding such antibodies, and cells comprising such polynucleotides. Disclosed herein are cells comprising the polynucleotides of SEQ ID NO:s 56, 58, and 60, antibodies comprising VH and/or VL regions encoded by SEQ ID NO:s 56, 58, and 60, polynucleotides encoding such antibodies, and cells comprising such polynucleotides. Such antibodies may or may not have the same epitope specificity as the original antibodies comprising the VH and VL regions encoded by the deposited polynucleotides, and may or may not have an affinity for C35 the same as or higher than the affinity of the original antibodies. In one embodiment , the antibodies bind the epitope representing residues 105 to 115 of the native C35 sequence.

Disclosed herein are antibodies comprising, or alternatively consisting of, fragments or variants of these antibodies (e.g., scFvs, diabodies, triabodies, tetrabodies, minibodies, heavy chains, VH regions, VH CDRs (Complementarity Determining Regions), light chains, VL regions, or VL CDRs) having an amino acid sequence of any one of the VH, VH CDRs, VLs, VL CDRs encoded by a polynucleotide of the invention. Such antibodies may or may not have the same epitope specificity as the original antibodies comprising the VH and VL regions encoded by the deposited polynucleotides, and may or may not have an affinity for C35 the same as or higher than the affinity of the original antibodies.

Disclosed herein are antibodies or fragments or variants thereof that bind one or more C35 polypeptides, and which are coupled to a detectable label, such as an enzyme, a fluorescent label, a luminescent label, or a bioluminescent label. Disclosed herein are antibodies or fragments or variants thereof that bind one or more C35 polypeptides, and which are coupled to a therapeutic or a toxin, e.g., a radioactive material. In one embodiment, the antibodies are coupled to a radioisotope.

Disclosed herein are nucleic acid molecule(s) generally isolated, encoding an antibody (including molecules, such as scFvs, diabodies, triabodies, tetrabodies, minibodies, VH regions, or VL regions, that comprise, or alternatively consist of, an antibody fragment or variant thereof. Disclosed herein is a host cell transformed with a nucleic acid molecule encoding an antibody (including molecules, such as scFvs, diabodies, triabodies, tetrabodies, minibodies, VH regions, or VL regions, that comprise, or alternatively consist of, an antibody fragment or variant thereof) and progeny thereof. Disclosed herein is a method for the production of an antibody (including a molecule comprising, or alternatively consisting of, an antibody fragment or variant thereof) of the invention. Disclosed herein is a method of expressing an antibody (including a molecule comprising, or alternatively consisting of, an antibody fragment or variant thereof) from a nucleic acid molecule.

Disclosed herein are methods and compositions for treating cancer comprising administering to a mammal, preferably a human, an effective amount of one or more antibodies or fragments or variants thereof, or related molecules, that immunospecifically bind a C35 polypeptide or a fragment or variant thereof. Disclosed herein are antibody-based methods and compositions for treating breast cancer, ovarian cancer, bladder cancer, lung cancer, prostate cancer, pancreatic cancer, colon cancer, and melanoma.

Disclosed herein is a combination therapy for treating cancer comprising administering to a mammal, preferably a human, an effective amount of a chemotherapeutic and an effective amount of one or more antibodies, or fragments or variants thereof, that are conjugated with a toxin, e.g., a radioactive material.

Disclosed herein are methods and compositions for detecting, diagnosing, or prognosing cancer comprising administering to a mammal, preferably a human, an effective amount of one or more antibodies or fragments or variants thereof, or related molecules, that immunospecifically bind to C35 or a fragment or variant thereof. Disclosed herein are antibody-based methods and compositions for detecting, diagnosing, or prognosing breast cancer, ovarian cancer, bladder cancer, lung cancer, prostate cancer, pancreatic cancer, colon cancer, and melanoma.

Disclosed herein is the use of the antibodies of the invention as a diagnostic tool to monitor the expression of C35 or in cancer. In certain embodiments, the method may also be employed as a diagnostic to confirm the efficacy of an apoptosis inducing regimen.

### BRIEF DESCRIPTION OF THE FIGURES

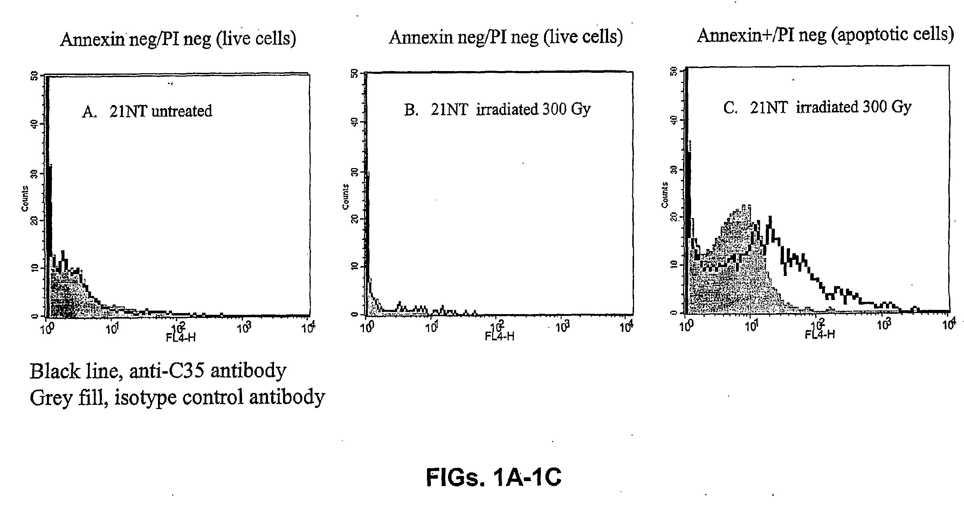

Figure 1 shows C35 surface staining of breast tumor cells following radiation induced apoptosis in 21MT1 breast tumor cells that express the C35 tumor antigen. Fig. 1A shows that untreated live cells (PI negative), that are not undergoing apoptosis (Annexin V negative) do not express C35 on the surface membrane as evidenced by absence of differential staining with anti-C35 antibody and the isotype control antibody. Fig. 1B shows, similarly, that irradiated tumor cells that remain viable (PI negative) and have not been induced to undergo apoptosis (Annexin V negative) also do not express C35 on the tumor cell surface membrane. Fig. 1C shows, in contrast, that irradiated tumor cells that are viable (PI negative), but undergoing apoptosis (Annexin V positive), are clearly differentially stained with anti-C35 antibodies as compared to isotype control antibody.

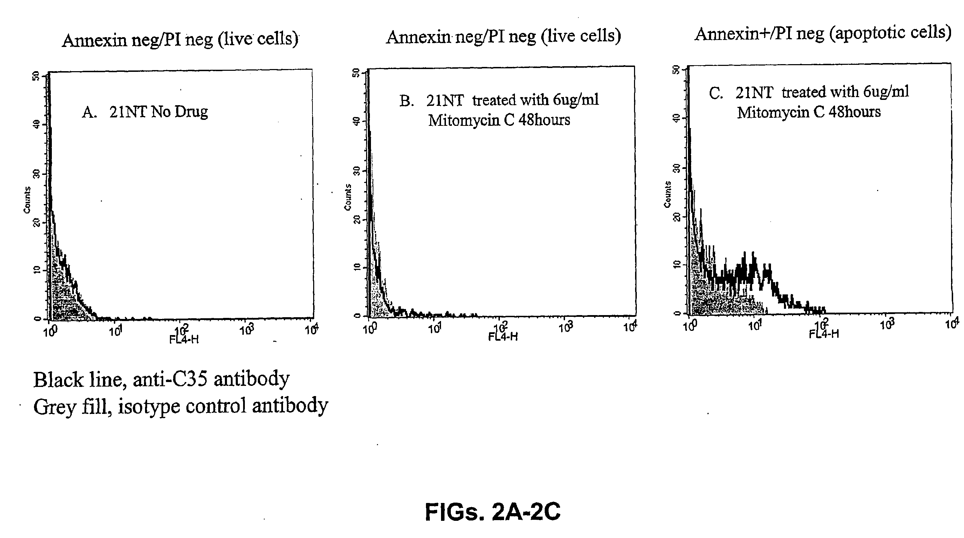

Figure 2 shows C35 surface staining of breast tumor cells following mitomycin C drug induced apoptosis. Fig. 2A shows that untreated live cells (PI negative), that are not undergoing apoptosis (Annexin V negative), do not express C35 on the surface membrane as evidenced by absence of differential staining with anti-C35 antibody and the isotype control antibody. Fig. 2B shows, similarly, that mitomycin C treated tumor cells that remain viable (PI negative) and have not been induced to undergo apoptosis (Annexin V negative) also do not express C35 on the tumor cell surface membrane. Fig. 2C shows, in contrast, that mitomycin C treated tumor cells that are viable (PI negative), but undergoing apoptosis (Annexin V positive), are clearly differentially stained with anti-C35 antibodies as compared to isotype control antibody.

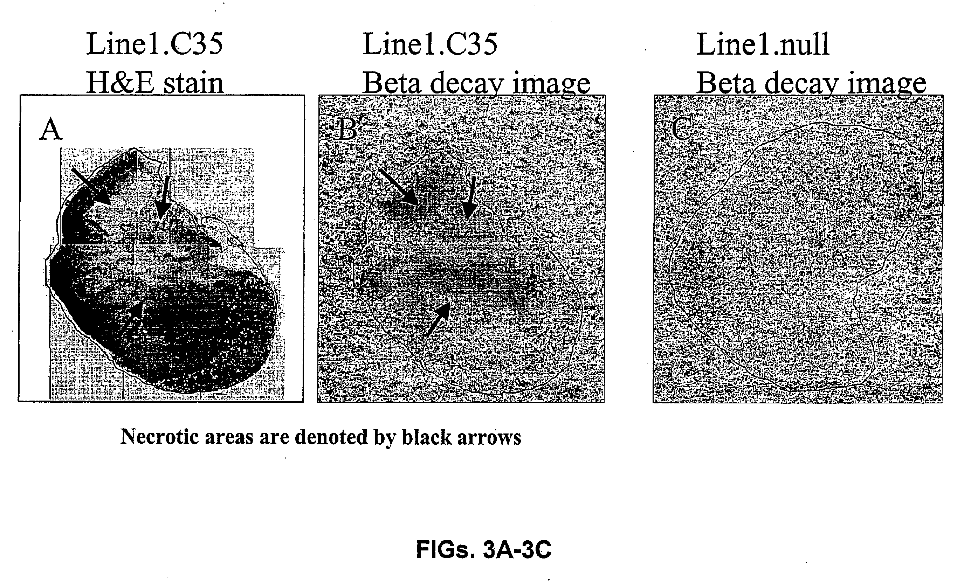

Figures 3A-3C show that Taxol™ induces apoptosis, resulting in exposure of C35 on the surface of apoptotic tumor cells. 24 hours following treatment with 0.5 uM Taxol™, 21MT1 tumor cells were stained with annexin V-FITC, propidium iodide, and with either 100 ng anti-C35 antibody 1F2 (dark line) or isotype control (grey fill) antibody. Both antibodies were directly conjugated to Alexa-647. Histograms were gated on the cells undergoing apoptosis (annexinV positive/PI negative). Antibodies were pre-incubated with PAB buffer (Fig. 3A), 100-fold molar excess recombinant C35 protein (Fig. 3B), or 100-fold molar excess β-galactosidase protein (Fig. 3C).

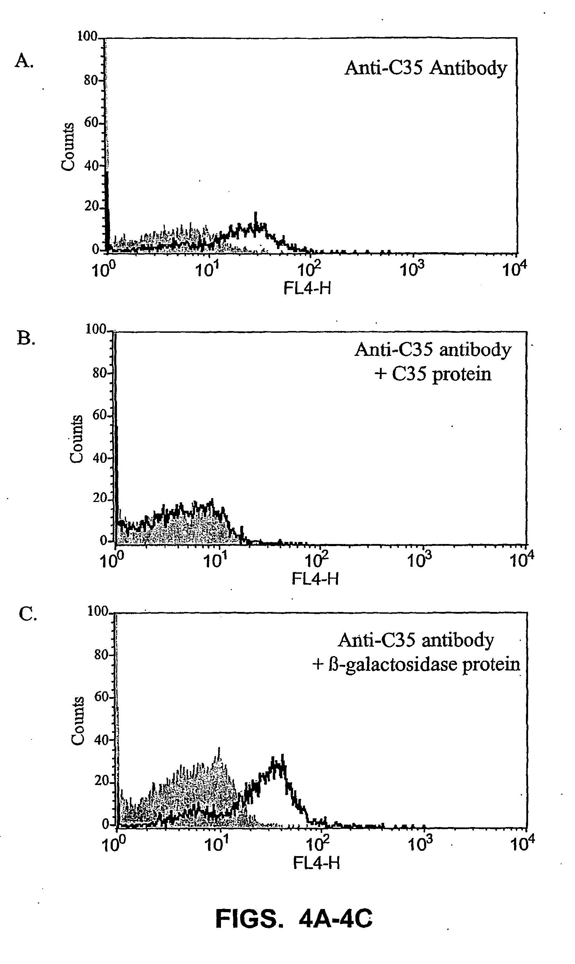

Figure 4 shows that anti-C35 monoclonal antibody localizes to necrotic regions of a C35+ tumor. BALB/c mice were engrafted on opposite flanks with syngeneic non-small cell lung cancer derived Line 1 tumor cells that either had or had not been transfected with human C35. C35 protein expression was confirmed by immunohistochemical staining with anti-C35 antibodies. After 14 days in vivo growth, animals received intravenous injection of 1251-labeled anti-C35 antibody. Animals were sacrificed 120 hrs after injection of radiolabeled antibodies and the concentration of anti-C35 antibodies in C35-positive and C35-negative tumors was determined by exposure of a tumor section to film. Figure 4A shows that radiolabeled anti-C35 antibodies concentrate only in the C35-positive and not the C35-negative tumors. Figures 4B and 4C compare the distribution of label and an H&E stain for intact cells within the tumors, confirming that under these conditions the labeled anti-C35 antibodies concentrated specifically in the necrotic regions of the C35-positive tumor.

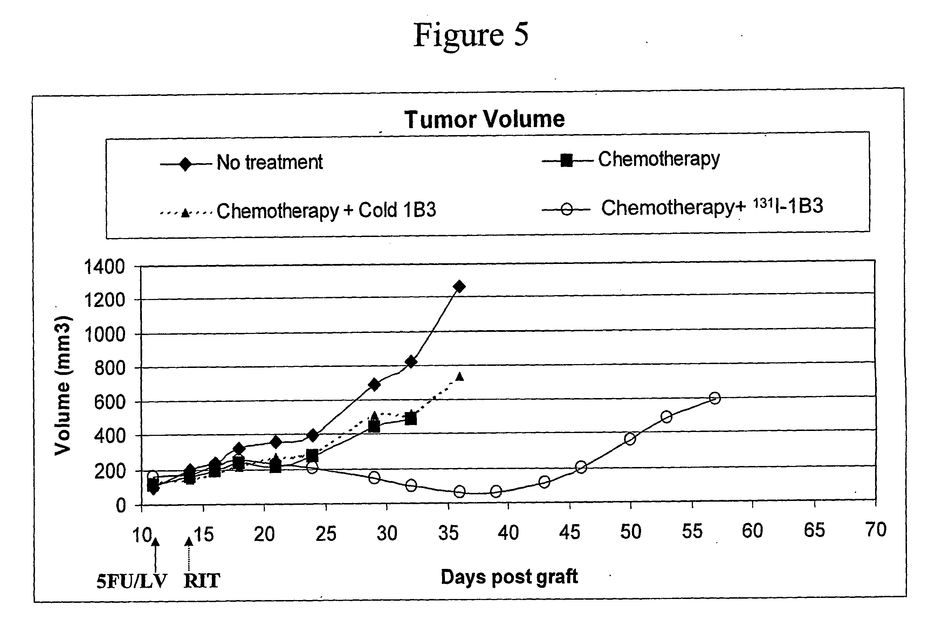

Figure 5 shows the effect on tumor volume of combination radioimmunotherapy with ¹³¹I-labeled 1B3 anti-C35 murine monoclonal antibody and chemotherapy (fluorouracil, 150 mg/kg; leucovorin, 100 mg/kg) in Swiss nude mice grafted with Colau.C35 tumor cells. Chemotherapy was initiated on day 11 after tumor graft and 300 µCi of ¹³¹I-labeled 1B3 anti-C35 antibody was administered on day 14. Tumor growth was followed for up to 8 weeks.

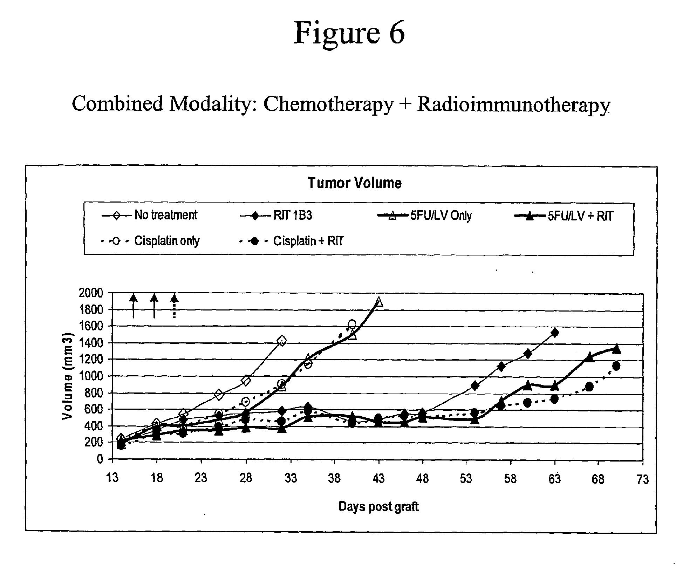

Figure 6 shows the effects on tumor volume of the combined modality treatment of chemotherapy and radioimmunotherapy. Swiss nude mice were grafted with Colau.C35 cells on day 0. Chemotherapy: Cisplatin administered at 2 mg/kg i.v. on days 15 & 18; 5FU/LV administered at 180/120 mg/kg i.v. on day 18. Radioimmunotherapy: 300 µCi (~50 µg) of ¹³¹I-labeled murine 1B3 anti-C35 IgG was administered on day 21.

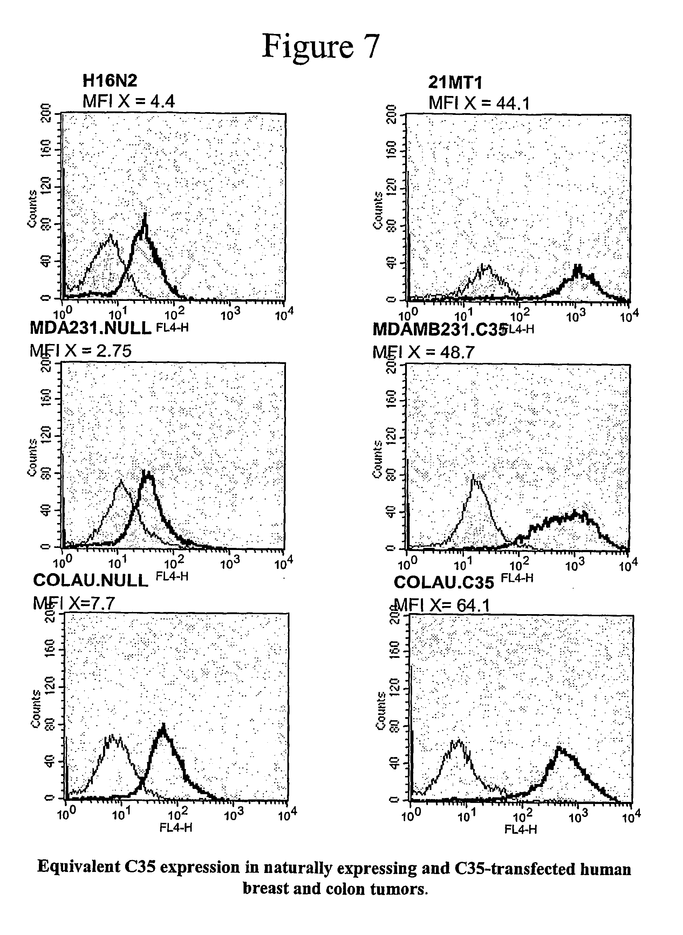

Figure 7 shows equivalent expression in naturally-expressing and C35-transfected human breast and colon tumors. Cells were stained with Alexa-647 conjugated anti-C35 MAb 1F2 or isotype control. MFI X is the ratio of the mean fluorescence intensity of 1F2/mean fluorescence intensity of isotype control. H16N2, derived from normal breast epithelium, and MDAMB231, a breast tumor, and Colau, a colon tumor, express low basal levels of C35. 21MT1, derived from breast carcinoma, naturally expresses high levels of C35. Colau and MDA231 were transduced with empty vector (null) or human C35 recombinant vector. All tumors were grown in vivo, tumors were excised, dissociated and stained.

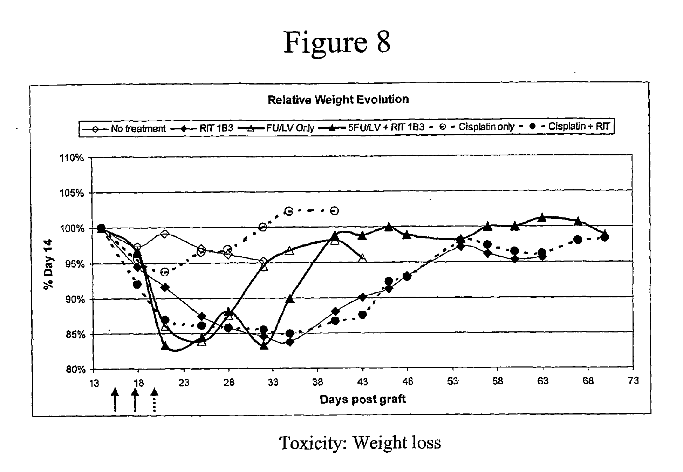

Figure 8 shows toxicity of chemotherapy, radioimmunotherapy, and combination therapy in Swiss nude mice as determined by weight loss.

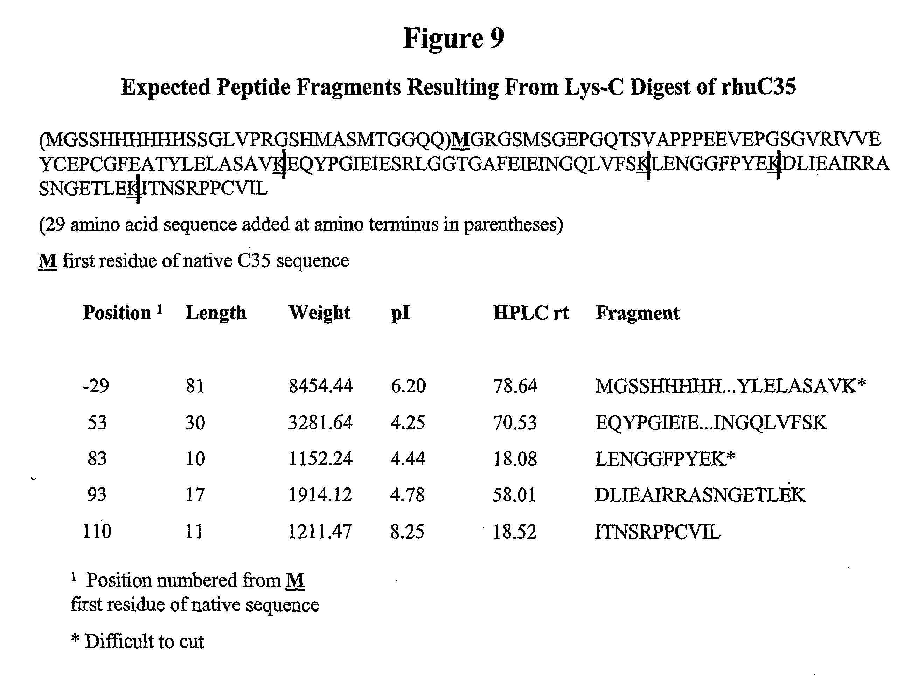

Figure 9 shows the expected peptide fragments following complete digestion of 6x His-tagged recombinant human C35 (rhC35) with Lys-C endoprotease. The full sequence of rhC35, including the amino terminal 6x His tag addition is shown. Amino acid poistions are numbered relative to the amino terminal methionine (bold M) of the native human C35 sequence. The asterisks by the first and third lysine (K) residues indicate that digestion at these positions is inefficient, and some longer fragments may be generated.

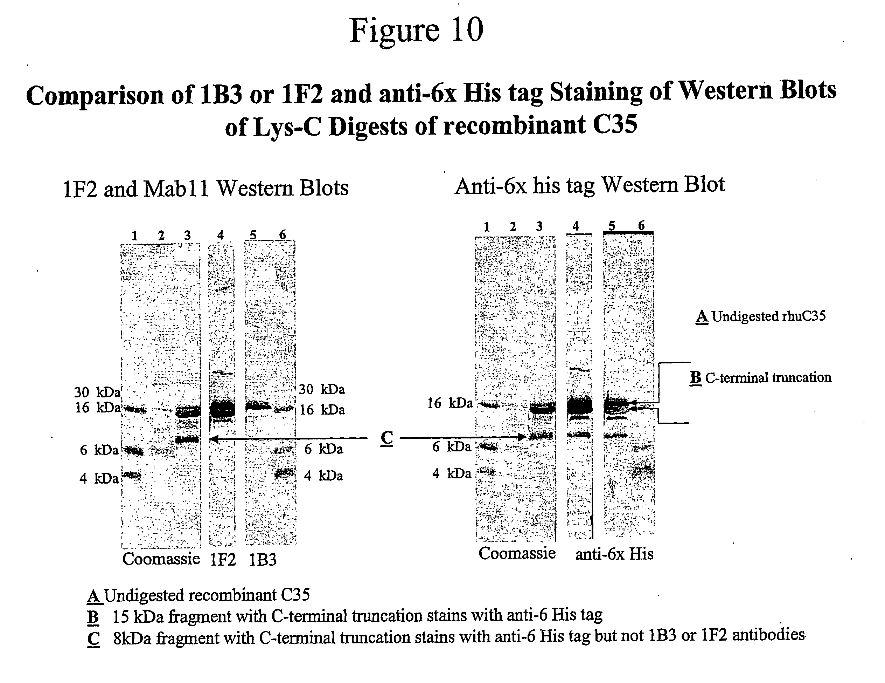

Figure 10 shows a comparison of 1B3 (Mab11) or 1F2 and anti-6x His tag staining of Western blots indicating the fragment of C35 to which each antibody binds.

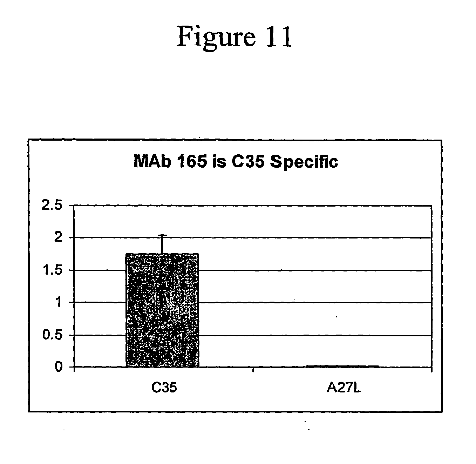

Figure 11 shows that MAb 165 is C35-specific. 141D10 recombinant vaccinia virus was co-infected into HeLa cells with UH8 recombinant vaccinia virus. The resulting secreted antibody was tested for binding to C35 or control protein A27L (vaccinia virus protein) by ELISA.

### DETAILED DESCRIPTION OF THE INVENTION

### OVERVIEW

A number of studies have described alterations in the surface membrane of cells undergoing apoptosis. Prominent among these changes is the early loss of phospholipid asymmetry as reflected in the exposure of phosphatidylserine on the outer leaflet of the surface membrane. It has been reported that this alteration in surface membrane composition facilitates recognition and removal of apoptotic cells by macrophages (Fadok, V.A., et al., J. Immunol. 148:2207-2216 (1992)). A general method has been developed that allows detection of cells undergoing apoptosis by binding of the anticoagulant Annexin V to the exposed phosphatidylserine molecules (Koopman, G., et al., Blood 84:1415-1420 (1994)).

Of more general interest is the possibility that expression and exposure of other surface membrane molecules, in particular proteins, may be altered in apoptotic cells. A number of reports have described apoptosis specific proteins (Grand, R.J.A., et al., Exp. Cell Res. 218:439-451 (1995); U.S. Patent number: 5,972,622, "Method of Detecting Apoptosis Using an Anti-Human GP46 Monoclonal Antibody", Filed: Feb. 6, 1997; Issued, Oct. 26, 1999) that appear to be expressed intracellularly. Of more direct relevance is a report of a monoclonal antibody that detects a 38 kD protein antigen that becomes associated with the surface membrane and mitochondrial membranes of apoptotic cells but is undetectable in normal cells (U.S. Patent number: 5,935,801, "Monoclonal Antibody that Detects Apoptotic Antigen", Filed: Mar. 29, 1996; Issued, Aug. 10, 1999). Other antigens have been described that become differentially exposed on or near the surface of apoptotic keratinocytes (Casciola-Rosen, L.A., et al., J. Exp. Med. 179:1317-1330 (1994)), and in cells undergoing apoptosis during embryonic development (Rotello, R.J., et al., Development 120:1421-1431 (1994)). Three defined protein antigens, CD3, CD69 and CD25 have been shown to be upregulated on the surface membrane of apoptotic thymocytes (Kishimoto, H., et al., J. Exp. Med. 181:649-655 (1995)). In each instance these are surface markers of apoptosis in normal cells and tissues. Although the same markers might also be associated with tumor cells undergoing apoptosis, they do not allow apoptotic tumor cells to be distinguished from normal cells undergoing apoptosis as part of normal tissue turnover. Therefore, they would not be useful as targets for treating cancer.

The present inventors have determined that there is a subset of intracellular tumor-specific or tumor-associated antigens that become exposed on the tumor cell membrane under conditions of chemotherapy or radiation induced apoptosis and could be effective targets for concentrating antibody conjugated radioisotopes or toxins within the tumor. Methods using antibodies against such antigens would be particularly effective because they could enhance the therapeutic benefits of standard apoptosis-inducing chemotherapy and radiation therapy in treating cancer. Disclosed herein are tumor-specific antigens that are associated with internal cell membranes -- in particular, differentially expressed molecules such as the C35 cancer-specific antigen that express a prenylation motifas a class of intracellular tumor antigens that become exposed on the surface membrane of tumor cells that have been induced to undergo apoptosis by radiation and/or chemotherapy.

Disclosed herein is a method that acts in conjunction with the induction of apoptosis (preferably large scale apoptosis) by chemotherapy or radiation therapy to enhance the eradication of tumors. It is based on the novel observation that a class of intracellular markers differentially expressed in tumor cells becomes exposed on the surface of apoptotic cells where it can be targeted by specific antibodies conjugated to a toxic payload. The benefits of this method of treatment are several-fold. For example, this method permits delivery to the tumor environment of a toxic payload that can destroy other non-apoptotic tumor cells in the vicinity of the apoptotic target. Additionally, this method can prevent otherwise viable cells that have initiated the apoptotic process, as evidenced by alterations in surface membrane constituents, from reversing the apoptotic progression and resuming growth (Hammill, A.K., et al., Exp. Cell Res. 251:16-21 (1999)).

The method targeting apoptotic cells should be distinguished from prior inventions targeting necrotic cells (U.S. Patent number: 6,071,491, "Detection of Necrotic Malignant Tissue and Associated Therapy", Filed: Aug. 9, 1999; Issued, Jun. 6, 2000). Necrosis results in release of intracellular contents into the extracellular tumor environment. Some of these intracellular antigens accumulate in that environment and could be targeted by specific antibodies. However, necrosis is associated with hypoxic regions of larger tumors that, because of the absence of oxygen radicals, are relatively resistant to radiation therapy and possibly radio-immunotherapy. Although there may be some increase in necrosis following treatment with chemotherapeutic agents (Desrues B., et al., Br. J. Cancer 72:1076-82, (1995)), the primary action of chemotherapeutic agents is to increase apoptosis. Therefore, necrosis is a less suitable target than apoptosis for immunotherapy of cancer and, in particular, eradication of smaller tumors and micrometastases that are responsible for tumor spread. Thus, methods that are effective at eradicating small tumors and micrometastases are especially useful for treating aggressive cancers.

The method should also be distinguished from the disclosure in patent application publication number US 2002/0052308 A1 (May 2, 2002), which discloses 842 cancer antigens, including an antigen (SEQ ID NO:966) with a large region identical to a portion of C35 (SEQ ID NO:2). US 2002/0052308 A1 generically discloses the administration of antibodies against the 842 cancer antigens "alone or in combination with other types of treatments (e.g., radiation therapy, chemotherapy, hormonal therapy, immunotherapy and anti-tumor agents)", page 205, paragraph [0229]. However, the published application does not specify that to be effective against a C35 related target, C35-specific antibodies conjugated to a toxin should be administered after apoptosis has been induced in tumor cells by administration of an apoptosis inducing agent such as chemotherapy, radiation therapy, or other anti-tumor agents. Indeed, multiple studies of combination chemotherapy and radioimmunotherapy directed at antigens that, in contrast to C35, are naturally expressed on the tumor cell surface membrane have concluded that optimal results are obtained by administration of the radioimmunotherapeutic antibody prior to chemotherapy, that is, before apoptosis has been induced (DeNardo S.J., et al. Anticancer Res. 18:4011-18, (1998); Clarke K., et al., Clin. Cancer Res. 6:3621-28, (2000); Burke P.A., Cancer 94:1320-31(2002); Stein, R. et al., Cancer 94:51-61 (2002); Odonnel R.T., et al., Prostate 50:27-37 (2002)). The discovery that apoptosis results in surface membrane exposure of a class of intracellular antigens including C35, which are prenylated and associated with internal membranes of untreated tumor cells, distinguishes the present disclosure. This disclosure teaches that for optimal effect, radioimmunotherapy directed at this class of target molecules is best administered such that the antibodies accumulate at the tumor site at approximately the same time that apoptosis has been induced in tumor cells by administration of an apoptosis inducing agent, or shortly thereafter. US 2002/0052308 A1 does not describe the subcellular location of the C35 related cancer antigen, nor does it describe how antibodies to this antigen should be administered for therapeutic effect.

### Methods Of Killing Cancer Cells

Disclosed herein is a method of killing cancer cells (also referred to herein as tumor cells) by first administering an effective amount of an apoptosis-inducing therapy (e.g., a chemotherapeutic agent and/or radiation), and subsequently administering an effective amount of an antibody conjugate or antibody complex that binds a cancer-associated antigen which is expressed intracellularly in tumor cells, but which becomes exposed on the cell surface in tumor cells that are undergoing apoptosis. The antibody is conjugated to or complexed with a toxin, as described below. The toxin insures that the cell to which the antibody binds will be killed, and/or kills surrounding cells that are also exposed to the toxin.

In one embodiment, the method involves the combined use of chemotherapy and radioimmunotherapy. Prior to the present disclosure, combination chemotherapy and radioimmunotherapy posed a problem due to cumulative dose-limiting bone marrow toxicity. The method provides for administration of such combination therapy in that chemotherapy results in exposure of an intracellular antigen to antibodies. Optimal timing of administration can be precisely determined employing the methods described herein and in the Examples.

In another embodiment, the method involves administering an antibody that is not conjugated to or complexed with a toxin, and cells which bind the antibody are killed. In this embodiment, a toxin is not needed to be conjugated to or complexed with the antibody in order to kill the cells that bind the antibody. Binding of the antibody itself kills the cell or insures that it dies. In this embodiment, apoptosis is preferably induced in most or nearly all of the cancer cells, e.g., most or nearly all of the cells of a tumor or metastasis.

The timing for administering the antibody, antibody conjugate or antibody complex after the apoptosis-inducing therapy can vary, however it must be within a certain window of time during which the tumor cells are undergoing apoptosis. Thus, the antibody, conjugated antibody or complexed antibody is administered within the time period during which apoptosis is being induced or is ongoing in the tumor cells that have been treated with the apoptosis-inducing therapy (e.g., chemotherapeutic agent and/or radiation). Chemotherapeutic agents generally induce apoptosis 24-72 hours after administration. Antibodies (e.g., complexed and conjugated antibodies) against cancer-associated antigens generally accumulate at the site of a tumor(s) 24-48 hours after administration. Whole antibodies generally take longer to accumulate than antibody fragments. Therefore, in general, antibodies (e.g., complexed and conjugated antibodies) against cancer-associated intracellular antigens should be administered 0-48 hours after administration of a chemotherapeutic agent (for antibody fragments), and from 24 hours before, to 24 hours after, administration of a chemotherapeutic agent (for whole antibodies) in the method of the invention. In a preferred embodiment, the antibody, or conjugated or complexed antibody is administered 0-6 hours, or 6-12 hours, or 6-24 hours, or 6-36 hours, or 6-48 hours, or 6-72 hours, or 6-96 hours, or 6-120 hours, or 12-24 hours, or 12-36 hours, or 12-48 hours, or 12-72 hours, or 12-96 hours, or 12-120 hours, or 24-36 hours, or 24-48 hours, or 24-72 hours, or 24-96 hours, or 24-120 hours, or 36-48 hours, or 36-72 hours, or 36-96 hours, or 36-120 hours or 0, 1, 2, 3, 4, 5, 6, 7, 8, 9, 10, 11, 12, 13, 14, 15*,* 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 hours after the apoptosis-inducing therapy is administered. In another embodiment, the antibody (e.g., complexed and conjugated antibodies) is administered prior to the apoptosis-inducing therapy, for example, 0-6 hours, 0-12, 0-24, 6-12, 6-24, 12-24, 24, 23, 22, 21, 20, 19, 18, 17, 16, 15, 14, 13, 12 10, 9, 8, 7, 6, 5, 4, 3, 2 hours, or 1 hour before the apoptosis-inducing therapy is administered. In another embodiment, the antibody (e.g., complexed and conjugated antibodies) is administered simultaneously with the apoptosis-inducing therapy.

Apoptosis-inducing therapies useful in the method are discussed below. By "tumor" or "cancer" or "hyperproliferative disease" is meant all neoplastic cell growth and proliferation, whether malignant or benign, incuding all transformed cells and tissues and all cancerous cells and tissues.

Examples of cancer include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. Other examples of cancer are listed under "Hyperproliferative Diseases," below.

By "surrounding" cancer cells is meant cancer cell(s) sufficiently close as to be killed by the toxin conjugated to or complexed with the antibody.

The method may be performed in vitro or in vivo, and may be used as a therapeutic in a patient, including a mammal such as a human.

### Cancer-Associated, Intracellular Antigens

The method is directed to the administration of antibodies (e.g., complexed and conjugated antibodies) against cancer-associated, intracellular antigens at a time such that the antibodies accumulate at the cancer site during or after apoptosis has been induced by the administration of an apoptosis-inducing therapy.

The terms "antigen" and "epitope," are well understood in the art and refer to the portion of a macromolecule which is specifically recognized by a component of the immune system, e.g., an antibody or a T-cell antigen receptor. The term epitope includes any protein determinant capable of specific binding to an immunoglobulin. Epitopic determinants usually consist of chemically active surface groupings of molecules such as amino acids or sugar side chains and usually have specific three dimensional structural characteristics, as well as specific charge characteristics.

By "cancer-associated antigen" or ''tumor-associated antigen" is meant an antigen that is expressed preferentially by cancer cells relative to non-cancerous cells of the same cell type or non-cancerous cells from the same tissue. Cancer-associated antigens may not be exclusively expressed by cancer cells (i.e., other normal cells may still express these antigens). However, the expression of cancer-associated antigens is generally consistently upregulated in cancers of a particular type or types. Cancer-associated antigens are antigens, preferably proteins, that may elicit specific immune responses in animals having particular types of cancer and thus, include cancer-associated antigens and fragments of cancer-associated antigens. The terms include not only complete tumor-associated antigens, but also epitope-comprising portions (fragments) thereof. A tumor-associated antigen (TAA) may be one found in nature, or may be a synthetic version of a TAA found in nature, or may be a variant of a naturally-occurring TAA, e.g., a variant that has enhanced immunogenic properties.

Many cancer-associated antigens are known in the art, and routine methods for determining whether a newly identified gene or protein is associated with a type of cancer or tumor are known. Such methods include northern blot analysis, differential display, SAGE, two dimensional protein gel electrophoresis or tandem mass spectrometry, Southern blot analysis, and other methods to detect an increased level of mRNA or protein expression or specific gene amplification in cancer cells from a particular type of cancer or tumor, in comparison with normal (non-cancerous or non-transformed) cells from the tissue of origin of the cancer or tumor, or in comparison to normal cells from other tissues. Other methods include analysis by tandem mass spectrometry of peptides differentially expressed in association with MHC molecules of tumor vs. normal cells.

By an "intracellular" cancer-associated antigen is meant a cancer-associated antigen that is expressed on an intracellular membrane of a tumor cell (a cancerous or transformed cell) that is not undergoing apoptosis. Internal membranes on which "intracellular" cancer-associated antigens may be expressed include the endoplasmic reticulum (ER), other cytoplasmic membrane bound vesicles including endosomal and lysosomal vesicles, mitochondrial membranes, and the nuclear membrane Preferred "internally expressed" cancer-associated antigens include prenylated proteins expressed on the endoplasmic reticulum and/or-membranes of endosomal or lysosomal vesicles. Examples of prenylated cancer-associated proteins are listed in Table 1. Prenylated cancer-associated proteins may also exclude C35 or any individual protein listed in Table 1, or any combination thereof. For example, prenylated cancer-associated proteins may exclude CENP-F kinetochore protein, CAAX box protein 1, DnaJ homolog subfamily A member 1 or 2, or Guanine nucleotide-binding protein G(I)/G(S)/G(O) gamma-5 subunit.

For a newly identified cancer-associated antigen, e.g., a cancer-associated protein, routine methods to determine whether it is prenylated include searching for a string of nucleotides at the 3' end of the gene/protein sequence that corresponds to a prenylation motif. A number of eukaryotic proteins are post-translationally modified by the attachment of either a farnesyl or a geranyl-geranyl group to a Cysteine residue (Glomset, J.A., et al., Trends Biochem. Sci. 15:139-142 (1990); Lowy, D.R., and Willumsen, B.M., Nature 341:384-385 (1989); Imagee, A.I, Biochem. Soc. Trans. 17:875-876 (1989); Powers, S., Curr. Biol. 1:114-116 (1991)). The modification occurs on cysteine residues that are three residues away from the C-terminal extremity; the two residues that separate this cysteine from the C-terminal residue are generally aliphatic (Ali). This Cys-Ali-Ali-X pattern is generally known as the CAAX box. Aliphatic amino acids include isoleucine, valine, leucine, alanine, and proline. The last four amino acids of C35 are CVIL. The leucine at the terminal position indicates that this is a substrate for the prenyltransferase GGTase I which results in addition of a geranyl-geranyl group (Moomaw and Casey, J. Biol. Chem. 267, 17438-17443 (1992)).

Thus, the method also provides for determining whether a cancer-associated protein is an intracellular protein which becomes exposed at the cell surface in cells undergoing apoptosis, prior to administering the antibody (e.g., complexed and conjugated antibodies) or apoptosis-inducing agent. Such a determination may encompass analyzing the amino acid sequence of a candidate protein for the CAAX box at its C-terminus by computer or manually, and/or performing assays to determine whether the candidate protein is expressed extracellularly after induction of apoptosis in cells expressing that protein. Such assays are know in the art and described herein (see, e.g. Examples 1 and 2) and include inducing apoptosis in cells expressing a candidate protein and using antibodies (e.g., labelled antibodies) specific for the candidate protein to detect the protein on the cell surface of the apoptotic cells.

**Table 1. Genes Encoding Cancer-Associated Prenylated Proteins**

| **Gene Name** | **GenBank Accession No**. | **Tumor Types** | **Protein Name** |
|---|---|---|---|
| CENPF | P49454 | Colon, kidney,liver, nervous, skin | CENP-F kinetochore protein (Centromere protein F) (Mitosin) (AH antigen) |
| CXX1 | 015255 | Breast, pancreas | CAAX box protein 1 (Cerebral protein-5) (hucep-5) |
| DNAJA1 OR HSJ2 OR HSPF4 OR DNAJ2 OR HDJ2 | P31689 | GI tract, pancreas, stomach, prostate | DnaJ homolog subfamily A member 1 (Heat shock 40 kDa protein 4) (DnaJ protein homolog 2) (HSJ-2) (HSDJ) |
| DNAJA2 OR HIRIP4 | 060884 | Colon, stomach | DnaJ homolog subfamily A member 2 (HIRA interacting protein 4) (Cell cycle progression restoration gene 3 protein) (Dnj3) |
| GNG5 OR GNGT5 | P30670 | Brain | Guanine nucleotide-binding protein G(I)/G(S)/G(O) gamma-5 subunit |
| GNG10 OR GNGT10 | P50151 | Genitourinary, breast | Guanine nucleotide-binding protein G(I)/G(S)/G(O) gamma-10 subunit |
| GNG12 | Q9UBI6 | Colon, cartilage, genitourinary | Guanine nucleotide-binding protein G(I)/G(S)/G(O) gamma-12 subunit |
| LMNB1 OR LMN2 OR LMNB | P20700 | Colon, genitourinary, breast | Lamin B 1 |
| LMNB2 OR LMN2 | Q03252 | Brain, colon, breast, prostate, stomach, uterus | Lamin B2 |
| LMNA OR LMN1 | P02545 | Pancreas, skin, stomach, uterus | Lamin A/C (70 kDa lamin) |
| PPP1R16A | Q96134 | Stomach | Protein phosphatase 1 regulatory inhibitor subunit 16A |
| PXF OR HK33 OR PEX19 | P40855 | Ovary | Peroxisomal farnesylated protein (33 kDa housekeeping protein) (Peroxin 19) |
| RAB11B OR YPT3 | Q15907 | Prostate, skin, uterus, kidney | Ras-related protein Rab-11B (GTP-binding protein YPT3) |
| RAB22A | Q9UL26 | Hepatocellular | Ras-related protein Rab-22A (Rab-22) |
| RAB7 | P51149 | Colon, head and neck, stomach | Ras-related protein Rab-7 |
| MEL OR RAB8 | P24407 | Nervous, ovary, pancreas, lung, uterus | Ras-related protein Rab-8 (Rab-8A) (Oncogene c-mel). |
| RAC1 | P15154 | Ovarian, colon | Ras-related C3 botulinum toxin substrate 1 (p21-Rac1) (Ras-like protein TC25) |
| RAC2 | P15153 | Kidney, liver, lung, nervous | Ras-related C3 botulinum toxin substrate 2 (p21-Rac2) (Small G protein) (GX) |
| RAC3 | 014658 | Breast, prostate | Ras-related C3 botulinum toxin substrate 3 (p21-Rac3) |
| RAP2A | P10114 | Prostate | Ras-related protein Rap-2a |
| RAP1B | P09526 | Skin | Ras-related protein Rap-lb (GTP-binding protein smg p21B) |
| HRAS OR HRAS1 | P01112 | Bladder, thyroid | Transforming protein p21/H-Ras-1 (c-H-ras) |
| KRAS2 OR RASK2 | P01116 | Colon, ovary | Transforming protein p21A (K-Ras 2A) (Ki-Ras) (c-K-ras). |
| NRAS | P01111 | Myeloma, melanoma, leukemia | Transforming protein N-Ras |
| RAB10 | 088386 | Colon, pancreas | Ras-related protein Rab-10 |
| RAB13 | P51153 | Brain, liver, breast | Ras-related protein Rab-13 |
| RAB1A OR RAB1 | P11476 | Colon, ovary, pancreas | Ras-related protein Rab-1A (YPT1-related protein). |
| RAB1B | Q9H0U4 | Breast, prostate, pancreas | Ras-related protein Rab-1B |
| RAB25 OR CATX8 | P57735 | Head and neck, ovary, pancreas | Ras-related protein Rab-25 (CATX-8). |
| RAB27A OR RAB27 | P51159 | Breast | Ras-related protein Rab-27A (Rab-27) (GTP-binding protein Ram). |
| RAB30 | Q15771 | Ovary, lung | Ras-related protein Rab-30 |
| RAB31 OR RAB22B | Q13636 | Pancreas | Ras-related protein Rab-31 (Rab-22B). |
| RAB32 | Q13637 | Brain | Ras-related protein Rab-32 |
| RAB35 OR RAB1C OR RAY | Q15286 | Prostate | Ras-related protein Rab-35 (Rab-1C) (GTP-binding protein RAY). |
| RAB36 | 095755 | Nasopharyngeal | Ras-related protein Rab-36 |
| RAB38 | P57729 | Melanoma | Ras-related protein Rab-38 (Antigen NY-MEL-1). |
| RAB3A | P20336 | Insulinoma | Ras-related protein Rab-3A |
| RAB3D OR RAB16 | 095716 | Ovary, prostate, breast | Ras-related protein Rab-3D |
| RAB4A OR RAB4 | P20338 | Stomach | Ras-related protein Rab-4A |
| RAB5C | P51148 | Skin, breast | Ras-related protein Rab-5C (RAB5L) (L1880). |
| RAB6A OR RAB6 | P20340 | Colon | Ras-related protein Rab-6A (Rab-6). |
| ARHA OR ARH12 OR RHOA OR RHO12 | P06749 | Breast | Transforming protein RhoA (H12). |
| ARHB OR ARH6 OR RHOB | P01121 | Breast | Transforming protein RhoB (H6). |
| ARHC OR ARH9 OR RHOC | P08134 | Pancreas | Transforming protein RhoC (H9). |
| ARHD OR RHOD | 000212 | Breast, pancreas, stomach | Rho-related GTP-binding protein RhoD (Rho-related protein HP1) (RhoHP1). |
| ARHG OR RHOG | P35238 | Lymphoreticular, pancreas, skin | Rho-related GTP-binding protein RhoG (Sid10750). |
| ARHH OR TTF | Q15669 | AIDS-associated NHL, NHL, primary lymphoma | Rho-related GTP-binding protein RhoH (GTP-binding protein TTF). |
| RRAS2 OR TC21 | P17082 | Ovary, breast | Ras-related protein R-Ras2 (Ras-like protein TC21) (Teratocarcinoma oncogene). |
| RRAS | P10301 | Pancreas | Ras-related protein R-Ras (p23). |

In the various embodiments , prenylated proteins are treated as a single class with closely related properties relative to translocation to the surface exposed membrane of cells undergoing apoptosis.

### Method Of Treatment

The method described above for killing cancer cells can be used in vivo, as a method of treating a mammalian subject.

By "subject" or "individual" or "patient" or "mammal" which terms are used interchangeably herein, is meant any subject, particularly a mammalian subject, for whom diagnosis or therapy is desired. Mammalian subjects include humans, domestic animals, farm animals, and zoo, sports, or pet animals such as dogs, cats, guinea pigs, rabbits, rats, mice, horses, cattle, cows, and so on.

The term "treat" or "treatment" refer to both therapeutic treatment and prophylactic or preventative measures, wherein the object is to prevent or slow down (lessen) an undesired physiological change or disorder, such as the development or spread of cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented. Any of these treatment types or types of patients may also be excluded.

Preferred in the method are the combinations shown in Table 1, in which an antibody (e.g., complexed and conjugated antibodies) specific for a listed prenylated protein, is used to treat a cancer type listed for that prenylated protein. For example, an antibody specific for CENP-F kinetochore may be used in the method to treat colon, kidney, liver, nervous, and skin cancers; an antibody specific for CAAX box protein 1 may be used to treat breast and pancreatic cancers; an antibody specific for DNAJ homolog subfamily A member 1 may be used to treat gastrointestinal tract, pancreatic, stomach, and prostate cancers, and so on. In addition, antibodies specific for C35 protein may be used to treat, diagnose, or detect, breast cancer, ovarian cancer, bladder cancer, lung cancer, prostate cancer, pancreatic cancer, colon cancer, and melanoma.

Also preferred in the method are a combination chemotherapy/radioimmunotherapy for treating cancer comprising administering to a mammal, preferably a human, an effective amount of a chemotherapeutic, followed by or simultaneous with an antibody of the invention that is conjugated to a toxin, preferably a radioactive material.

In another embodiment, an antibody that is conjugated with a toxin, preferably a radioactive material, is administered alone.

### Antibodies

As used herein, the term "antibodies" or "immunoglobulins" refers to antibodies comprised of two immunoglobulin heavy chains and two immunoglobulin light chains as well as a variety of forms besides antibodies; including, for example, Fv, Fab, and F(ab')2 as well as bifunctional hybrid antibodies (e.g., Lanzavecchia et al., Eur. J. Immunol. 17, 105 (1987)) and single chains (e.g., Huston et al., Proc. Natl. Acad. Sci. U.S.A., 85, 5879-5883 (1988) and Bird et al., Science 242, 423-426 (1988). (See, generally, Hood et al., Immunology, Benjamin, N.Y., 2ND ed. (1984), Harlow and Lane, Antibodies. A Laboratory Manual, Cold Spring Harbor Laboratory (1988) and Hunkapiller and Hood, Nature, 323, 15-16 (1986).

Antibodies include, but are not limited to, polyclonal, monoclonal, multispecific, human, humanized or chimeric antibodies, single chain antibodies, Fab fragments, F(ab')2 fragments, fragments produced by a Fab expression library, domain-deleted antibodies (including, e.g., CH2 domain-deleted antibodies), anti-idiotypic (anti-Id) antibodies (including, e.g., anti-Id antibodies to antibodies of the invention), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules can be of any type (e.g., IgG, IgE, IgM, IgD, IgA and IgY), class (e.g., IgG1, IgG2, IgG3, IgG4, IgA1 and IgA2) or subclass of immunoglobulin molecule.

Antibodies also include, but are not limited to, engineered forms of antibodies and antibody fragments such as diabodies, triabodies, tetrabodies, and higher multimers of scFvs, as well as minibodies, such as two scFv fragments joined by two constant (C) domains. *See, e.g.,* Hudson, P.J. and Couriau, C., Nature Med. 9: 129-134 (2003); U.S. Publication No. 20030148409; U.S. Patent No. 5,837,242.

The antibodies may be from any animal origin including birds and mammals. Preferably, the antibodies are human, murine (e.g., mouse and rat), donkey, ship rabbit, goat, guinea pig, camel, horse, or chicken. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example in, U.S. Pat. No. 5,939,598 by Kucherlapati et al.

The phrase "substantially identical," in the context of two nucleic acids or polypeptides (e.g., DNAs encoding a C35 antibody or the amino acid sequence of the C35 antibody) refers to two or more sequences or subsequences that have at least about 80%, most preferably 90-95% or higher nucleotide or amino acid residue identity, when compared and aligned for maximum correspondence, as measured using the following sequence comparison method and/or by visual inspection. Such "substantially identical" sequences are typically considered to be homologous. Preferably, the "substantial identity" exists over a region of the sequences that is at least about 50 residues in length, more preferably over a region of at least about 100 residues, and most preferably the sequences are substantially identical over at least about 150 residues, or over the full length of the two sequences to be compared. As described below, any two antibody sequences can only be aligned in one way, by using the numbering scheme in Kabat. Therefore, for antibodies, percent identity has a unique and well-defined meaning.

Amino acids from the variable regions of the mature heavy and light chains of immunoglobulins are designated Hx and Lx respectively, where x is a number designating the position of an amino acid according to the scheme of Kabat, Sequences of Proteins of Immunological Interest (National Institutes of Health, Bethesda, Md., 1987 and 1991). Kabat lists many amino acid sequences for antibodies for each subgroup, and lists the most commonly occurring amino acid for each residue position in that subgroup to generate a consensus sequence. Kabat uses a method for assigning a residue number to each amino acid in a listed sequence, and this method for assigning residue numbers has become standard in the field. Kabat's scheme is extendible to other antibodies not included in his compendium by aligning the antibody in question with one of the consensus sequences in Kabat by reference to conserved amino acids. The use of the Kabat numbering system readily identifies amino acids at equivalent positions in different antibodies. For example, an amino acid at the L50 position of a human antibody occupies the equivalent position to an amino acid position L50 of a mouse antibody.

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. The variable regions of each light/heavy chain pair form the antibody binding site. Thus, an intact antibody has two binding sites.

Light chains are classified as either kappa or lambda. Heavy chains are classified as gamma, mu, alpha, delta, or epsilon, and define the antibody's isotype as IgG, IgM, IgA, IgD and IgE, respectively. Within light and heavy chains, the variable and constant regions are joined by a "J" region of about 12 or more amino acids, with the heavy chain also including a "D" region of about 10 more amino acids. (See generally, Fundamental Immunology, Paul, W., ed., 3rd ed. Raven Press, N.Y., 1993, SH. 9).

From N-terminal to C-terminal, both light and heavy chain variable regions comprise alternating framework and complementarity determining regions (CDRs): FR, CDR, FR, CDR, FR, CDR and FR. The assignment of amino acids to each region is in accordance with the definitions of Kabat (1987) and (1991), supra, and/or Chothia & Lesk, J. Mol. Biol. 196:901-917 (1987); Chothia et al., Nature 342:878-883 (1989).

As used herein, the term "framework region" refers to those portions of antibody light and heavy chain variable regions that are relatively conserved (i.e., other than the CDRs) among different immunoglobulins in a single species, as defined by Kabat, et al., op. cit. As used herein, a "human framework region" is a framework region that is substantially identical (about 85% or more) to the framework region of a naturally occurring human antibody.

Most preferably for use in humans, the antibodies are human or humanized antigen-binding antibody fragments and include, but are not limited to, Fab, Fab' and F(ab')2, Fd, single-chain Fvs (scFv), diabodies, triabodies, tetrabodies, minibodies, domain-deleted antibodies, single-chain antibodies, disulfide-linked Fvs (sdFv) and fragments comprising either a VL or VH region. Antigen-binding antibody fragments, including single-chain antibodies, may comprise the variable region(s) alone or in combination with the entirety or a portion of the following: hinge region, CHI, CH2, and CH3 domains. Also included are antigen-binding fragments also comprising any combination of variable region(s) with a hinge region, CH1, CH2, and CH3 domains.

Preferred antibodies in the therapeutic methods are those containing a deletion of the CH2 domain.

As used herein, the term "humanized" immunoglobulin or "humanized" antibody refers to an immunoglobulin comprising a human framework, at least one CDR from a non-human antibody, and in which any constant region present is substantially identical to a human immunoglobulin constant region, i.e., at least about 85-90%, preferably at least 95% identical. Hence, all parts of a humanized immunoglobulin, except possibly the CDRs, are substantially identical to corresponding parts of one or more native human immunoglobulin sequences. For example, a humanized immunoglobulin would not encompass a chimeric mouse variable region/human constant region antibody.

As used herein, the term "chimeric" antibody refers to an antibody whose heavy and light chains have been constructed, typically by genetic engineering, from immunoglobulin gene segments belonging to different species. For example, the variable (V) segments of the genes from a mouse monoclonal antibody may be joined to human constant (C) segments, such as gamma1 and/or gamma4. A typical therapeutic or diagnostic chimeric antibody is thus a hybrid protein comprising at least one V region (e.g., VH or VL) or the entire antigen-binding domain (i.e., VH and VL) from a mouse antibody and at least one C (effector) region (e.g., CH (CH1, CH2, CH3, or CH4) or CL (CL1, CL2, CL3, or CL4)) or the entire C domain (i.e., CH and CL) from a human antibody, although other mammalian species may be used. In some embodiments, especially for use in the therapeutic methods , chimeric antibodies contain no CH2 domain.

The antibodies may be monospecific, bispecific, trispecific or of greater multispecificity. Multispecific antibodies may be specific for different epitopes of a polypeptide or may be specific for both a polypeptide of the present invention as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Pat..Nos. 4,474,893; 4,714,681; 4,925,648; 5,573,920; 5,601,819; Kostelny et al., J. Immunol. 148:1547-1553 (1992).

The description in this section applies to C35 antibodies and to other antibodies useful in the method Such antibodies may be conjugated to or complexed with a toxin, as described herein, or may be unconjugated or uncomplexed.

### C35 Antibodies

C35 is an antigen differentially expressed in breast cancer and certain other tumor types including melanoma, colon carcinoma, ovarian cancer, and pancreatic cancer. The C35 protein has been shown to be prenylated and to associate with internal cell membranes but is not detectable on the surface membrane of viable tumor cells. The inventors have produced a number of antibodies, including mouse monoclonal antibodies and human antibodies, that immunospecifically recognize C35 epitopes. The inventors have also demonstrated that induction of apoptosis in tumor cells by treatment either with a chemotherapeutic agent or irradiation results in surface membrane exposure of C35 that permits intact tumor cells to be recognized by C35-specific antibodies.

C35 Polynucleotide and amino acid sequences (SEQ ID NOs:1 and 2)

Thus, disclosed herein are antibodies against C35, polynucleotides encoding such antibodies, methods of treating C35-associated cancers using C35 antibodies and polynucleotides, and methods of detection and diagnosis using C35 antibodies and polynucleotides. Also provided are vectors and host cells comprising C35 antibody polynucleotides, and methods of producing C35 antibodies. As described in more detail herein, disclosed herein methods using C35 antibodies for cancer treatment, detection, and diagnosis. The description above under the "Antibodies" section also applies to C35 antibodies described herein.

Disclosed herein are antibody-based treatment methods which involve administering C35 antibodies of the invention to a subject, preferably a mammal, and most preferably a human, for treating one or more C35 cancers. Therapeutic compounds include, but are not limited to, antibodies (including fragments, analogs and derivatives thereof as described herein) and nucleic acids encoding antibodies (including fragments, analogs and derivatives thereof as described herein). The antibodies can be used to treat, detect or diagnose C35-associated cancers, including breast, ovarian, colon, pancreatic, and bladder cancers, and melanoma. C35 antibodies may be provided in pharmaceutically acceptable compositions as known in the art or as described herein.

Antibodies include, but are not limited to, polyclonal, monoclonal, multispecific, human, humanized or chimeric antibodies, single chain antibodies, scFvs, diabodies, triabodies, tetrabodies, minibodies, domain-deleted antibodies, Fab fragments, F(ab')2 fragments, fragments produced by a Fab expression library, anti-idiotypic (anti-Id) antibodies (including, e.g., anti-Id antibodies to antibodies
), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules, can be of any type (e.g., IgG, IgE, IgM, IgD, IgA and IgY), class (e.g., IgG1, IgG2, IgG3, IgG4, IgA1 and IgA2) or subclass of immunoglobulin molecule.

Hybridoma cell lines 1F2.4.1 and 1B3.6.1, specific for C35 polypeptides, were prepared using hybridoma technology. (Kohler et al., Nature 256:495 (1975); Kohler et al., Eur. J. Immunol. 6:511 (1976); Kohler et al., Eur. J. Immunol. 6:292 (1976); Hammerling et al., in: Monoclonal Antibodies and T-Cell Hybridomas, Elsevier, N.Y., pp. 571-681 (1981)). Briefly, hybridoma cell lines were generated using standard PEG fusion to the non-secreting myeloma cell line NS-1 (P3/NS1/1-AG4-1, ATCC #TIB-18) of splenocytes from BALB/c mice immunized with syngeneic BCA34 fibroblast tumor cells transduced to overexpress C35. Following PEG fusion to NS-1, the hybridomas were grown in methylcellulose semi-solid media. Approximately 2 weeks later, hybridoma colonies were isolated into 96 well plates and individual supernatants were tested for reactivity with C35 by ELISA, "Western blot, and immunohistochemistry. Positive hybridomas colonies were subcloned and screened for reactivity twice to ensure clonality. Antibodies were isolated from hybridoma supernatants by protein G affinity purification using standard methods. Antibodies from two hybridoma cell lines, 1F2 and 1B3, specifically bind recombinant C35 protein in ELISA and Western Blot assays. Antibodies from hybridoma cell line 1F2 also specifically stain formalin fixed, paraffin embedded C35 positive tumors and cell lines by immunohistochemistry. In addition, we have developed intracellular staining flow cytometry assays for quantitative analysis using antibodies from hybridoma cell line 1F2 conjugated to Alexa-647 flourochrome. Each of these antibodies is distinct, yet both are specific for C35 protein. It is possible to immunoprecipitate C35 protein from cell lysates with either of these antibodies and detect with the other. Competitive binding ELISA assays suggest that the monoclonal antibodies produced by hybridoma cell lines 1F2 and 1B3 bind different epitopes of the C35 protein.

C35 antibodies include antibodies which immunospecifically bind a C35 polypeptide, polypeptide fragment, or variant of SEQ ID NO:2, and/or an epitope, (as determined by immunoassays well known in the art for assaying specific antibody-antigen binding).

As used herein the term "isolated" is meant to describe a compound of interest (e.g., a C35 antibody) that is in an environment different from that in which the compound naturally occurs. "Isolated" is meant to include compounds that are within samples that are substantially enriched for the compound of interest and/or in which the compound of interest is partially or substantially purified.

As used herein, the terms "substantially enriched" and "substantially purified" refers to a compound that is removed from its natural environment and is at least 60% free, preferably 75% free, and most preferably 90% free from other components with which it is naturally associated. As used here, an antibody having the "same specificity" as a reference antibody means the antibody binds the same epitope as the reference antibody. The determination of whether an antibody binds the same epitope as a reference antibody may be performed using the assays described in the "Assays For Antibody Binding" section below.

The antibodies derived from mouse hybridoma cell lines discussed herein are 1F2 and 1B3. Polynucleotides encoding the VL and VH regions of these antibodies were cloned into TOPO vectors as described in Example 6, which were deposited with the American Type Culture Collection ("ATCC") on the date listed in Table 2, and given ATCC Deposit Numbers listed in Table 2. The ATCC is located at 10801 University Boulevard, Manassas, VA 20110-2209, USA. The ATCC deposits were made pursuant to the terms of the Budapest Treaty on the international recognition of the deposit of microorganisms for purposes of patent procedure.

Clone 1F2G was deposited at the ATCC on November 11, 2003 and given ATCC Deposit Number PTA-5639. Clone 1F2K was deposited at the ATCC on November 11, 2003 and given ATCC Deposit Number PTA-5640. Clone 1B3G was deposited at the ATCC on November 11, 2003 and given ATCC Deposit Number PTA-5637. Clone 1B3K was deposited at the ATCC on November 11, 2003 and given ATCC Deposit Number PTA-5638.

**Table 2. Deposited Polynucleotide Clones Encoding Mouse Anti-C35 Variable Regions**

| Polynucleotide Clone | ATCC Accession No. | Deposit Date |
|---|---|---|
| 1F2G | PTA-5639 | November 11, 2003 |
| 1F2K | PTA-5640 | November 11, 2003 |
| 1B3G | PTA-5637 | November 11, 2003 |
| 1B3K | PTA-5638 | November 11, 2003 |

The sequences of the mouse variable region genes and part of the vector of the deposited clones are set forth below.
*Italics* = Topo vector sequence (included in deposited clone)
dotted underline = EcoR1 cloning site of Topo vector
Lowercase = 5'untranslated region including generacer primer
ATG = Murine signal peptide begin
**bold = Frame work** regions (FWR)
double underline = CDR1, CDR2, or CDR3
underline = 5' portion of mouse IgG1 or kappa constant region

1F2 murine anti-C35 Vgamma1 gene polynucleotide sequence (from clone 1F2G) SIGNAL PEPTIDE = 18 AA
FR1 = 30 AA
CDR1 = 6AA
FR2 = 14 AA
CDR2=16AA
FR3 = 32AA
CDR3 = 7 AA
FR4 = 11AA

1F2 VH amino acid sequence (encoded by clone 1F2G)

1F2 murine anti-C35 kappa V gene polynucleotide sequence (from clone 1F2K) SIGNAL PEPTIDE = 22 AA
FR1 = 23 AA
CDR 1 = 10 AA
FR 2 = 15 AA
CDR 2 = 7 AA
FR 3 = 32 AA
CDR 3 = 9 AA
FR 4 = 10 AA

1F2-VK amino acid sequence (encoded by clone 1F2K)

1B3 murine anti-C35 Vgamma V-gene (encoded by clone 1B3G) (NC1-A7 V139-D-J1 (VH36-60) M13281) SIG PEP = 18 AA
FR1 = 30AA
CDR 1 = 5 AA
FR2 = 14 AA
CDR2 = 16 AA
FR3=32AA
CDR 3 = 14 AA
FR 4 = 11 AA

1B3 VH amino acid sequence (encoded by clone 1B3G)

1B3 murine anti-C35 kappa V-gene (from clone 1B3K) SP = 20AA
FR1=23 aa
CDR1 = 11 aa
FR2 = 15 aa
CDR2 = 7 AA
FR3 = 32 aa
CDR 3 = 9 AA
FR 4 = 10 AA
1B3 VK amino acid sequence (encoded by clone 1B3K)

The present inventors have also produced two C35 antibodies, MAb 165 and MAb 171, using the method disclosed in US 2002 0123057 A1, published 5 September 2002. The heavy chain variable regions of MAb 165 and MAb 171 comprise the same CDR3 region as the the 1B3 antibody heavy chain variable region described above. The remainders of MAbs 165 and 171 are of human origin. Disclosed herein are
antibodies that immunospecifically bind C35 polypeptides, comprising any one of the VH or VL regions of SEQ ID NO:56, SEQ ID NO:58, or SEQ ID NO:60, or a combination of either VH region encoded by SEQ ID NO:56 or SEQ ID NO:60 and the VL region encoded by SEQ ID NO:58, and preferably the C35-specific antibodies MAb 165 or MAb 171. Both MAb 165 and MAb 171 comprise the same kappa light chain, UH8 VK L120.

The sequences of the heavy and light chain variable regions of MAb 165 and MAb 171 are set forth below.
UNDERLINE = CDR1, CDR2, or CDR3

MAb 165 VH (141D10 VH H732) nucleotide sequence:

MAb 165 VH (141D10 VH H732) amino acid sequence:

MAb 171 VH (MSH3 VH H835) nucleotide sequence:

MAb 171 VH (141D10 VH H732) amino acid sequence:

UH8 VK L120 nucleotide sequence:

UH8 VK L120 amino acid sequence:

The present inventors have also produced a human C35 antibody, MAbc009, using the method disclosed in US 2002 0123057 A1.
Disclosed herein are antibodies that immunospecifically bind C35 polypeptides, comprising the VH and VL regions encoded by the polynucleotide clones that are listed in Table 3, preferably the fully human C35-specific antibody MAbc009. Polynucleotides encoding the VL and VH regions of this antibody were cloned into TOPO vectors as described in Example 6, which were deposited with the American Type Culture Collection ("ATCC") on the date listed in Table 3, and given ATCC Deposit Numbers listed in Table 3. The ATCC is located at 10801 University Boulevard, Manassas, VA 20110-2209, USA. The ATCC deposit was made pursuant to the terms of the Budapest Treaty on the international recognition of the deposit of microorganisms for purposes of patent procedure.

Clone H0009 was deposited at the ATCC on November 11, 2003 and given ATCC Deposit Number PTA-5641. Clone L0010 was deposited at the ATCC on November 11, 2003 and given ATCC Deposit Number PTA-5542.

**Table 3. Deposited Polynucleotide Clones Encoding Human Anti-C35 Variable Regions**

| Polynucleotide Clone | Encoded Antibody Region | ATCC Accession No. | Deposit Date | |
|---|---|---|---|---|
| H0009 | VH of MAbc009 | PTA-5641 | Nov. 11, 2003 | |
| L0010 | VL of MAbc009 | PTA-5642 | Nov. 11, 2003 | |

The sequences of the human variable region genes and part of the vector of the deposited clones are set forth below.
DOTTED UNDERLINE = EcoR1 Cloning Site Of Topo Vector
**ATG** = human signal peptide begin
BOLD = FRAME WORK REGIONS
DOUBLE UNDERLINE = CDR1, CDR2, OR CDR3
UNDERLINE = Human IgG1GS or Kappa Constant Region

MAbc0009 VH NUCLEOTIDE SEQUENCE (from clone H0009)

MAbc0009 VH AMINO ACID SEQUENCE (encoded by clone H0009) MAbc0009 VK NUCLEOTIDE SEQUENCE (from clone L0010)

MAbc0009 VK AMINO ACID SEQUENCE (encoded by clone L0010)

The mouse C35 antibodies have heavy and light chain variable regions designated SEQ ID Nos:3-10. The mouse antibodies 1F2 and 1B3 have gamma1 isotype and kappa light chains. The antibodies MAb 165 and MAb 171 that have the same heavy chain variable region CDR3 as 1B3 mouse antibody have heavy and light chain variable regions designated SEQ ID NOs:56-60. The antibodies MAb 165 and MAb 171 have kappa light chains. The human antibody MAbc009 has heavy and light chain variable regions designated SEQ ID Nos:11-14. The human antibody MAbc009 has gamma1 isotype and kappa light chains.

Disclosed herein are antibodies (including molecules comprising, or alternatively consisting of, antibody fragments or variants thereof) that immunospecifically bind to a C35 polypeptide or a fragment, variant, or fusion protein thereof. A C35 polypeptide includes, but is not limited to, the C35 polypeptide of SEQ ID NO:2. C35 polypeptides may be produced through recombinant expression of nucleic acids encoding the polypeptide of SEQ ID NO:2. (See WO 01/74859 for epitope-containing fragments of C35.)

Preferably, analogs of exemplified antibodies differ from exemplified antibodies by conservative amino acid substitutions. For purposes of classifying amino acids substitutions as conservative or nonconservative, amino acids may be grouped as follows: Group I (hydrophobic sidechains): met, ala, val, leu, ile; Group H (neutral hydrophilic side chains): cys, ser, thr; Group III (acidic side chains): asp, glu; Group IV (basic side chains): asn, gln, his, lys, arg; Group V (residues influencing chain orientation): gly, pro; and Group VI (aromatic side chains): trp, tyr, phe. Conservative substitutions involve substitutions between amino acids in the same class. Non-conservative substitutions constitute exchanging a member of one of these classes for a member of another.

In one embodiment, antibodies that immunospecifically bind to a C35 polypeptide or a fragment or variant thereof, comprise a polypeptide having the amino acid sequence of SEQ ID NO:57 or 61, or any one of the VH regions encoded by at least one of the polynucleotides referred to in Tables 2 or 3 and/or SEQ ID NO:59 or any one of the VL regions encoded by at least one of the polynucleotides referred to in Tables 2 or 3. In further embodiments, antibodies comprise the amino acid sequence of SEQ ID NO:57 or SEQ ID NO:61 and the amino acid sequence of SEQ ID NO:59. In preferred embodiments, antibodies comprise the amino acid sequence of a VH region encoded by clone H0009 and a VL region encoded by clone L0010, referred to in Table 3. In preferred embodiments, antibodies comprise the amino acid sequence of a VH region encoded by clone 1F2G and a VL region encoded by clone 1F2K, or a VH region encoded by clone 1B3G and a VL region encoded by clone 1B3K of Table 2. In other preferred embodiments, antibodies comprise the amino acid sequence of a VH region encoded by clone H0009 of Table 3, the amino acid sequence of SEQ ID NO:57, or the amino acid sequence of SEQ ID NO:61 and a VL region encoded by clone 1F2K or 1B3K of Table 2; or a VH region encoded by clone 1F2G or 1B3G of Table 2, the amino acid sequence of SEQ ID NO:57, or the amino acid sequence of SEQ ID NO:61 and a VL region encoded by clone L0010 of Table 3; or a VH region encoded by clone 1F2G or 1B3G of Table 2 or clone H009 of Table 3 and the amino acid sequence of SEQ ID NO:59. In other preferred embodiments, antibodies comprise the amino acid sequence of a VH region encoded by clone IF2G and a VL region encoded by clone 1B3K of Table 2, or a VH region encoded by clone 1B3G and a VL region encoded by clone 1F2K of Table 2. Molecules comprising, or alternatively consisting of, antibody fragments or variants of the VH and/or VL regions encoded by at least one of the polynucleotides referred to in Tables 2 or 3 that immunospecifically bind to a C35 polypeptide are also disclosed , as are nucleic acid molecules encoding these VH and VL regions, molecules, fragments and/or variants.

Disclosed herein are antibodies that immunospecifically bind to a polypeptide, or polypeptide fragment or variant of a C35 polypeptide, wherein said antibodies comprise, or alternatively consist of, a polypeptide having an amino acid sequence of any one, two, three, or more of the VH CDRs contained in VH regions encoded by one or more polynucleotides of SEQ ID NOs:56 or 60 or referred to in Tables 2 or 3. In particular, disclosed herein are antibodies that immunospecifically bind a C35 poypeptide, comprising, or alternatively consisting of, a polypeptide having the amino acid sequence of a VH CDR1 contained in a VH region encoded by one or more polynucleotides of SEQ ID NOs:56 or 60 or referred to in Tables 2 or 3. In another embodiment, antibodies that immunospecifically bind a C35 polypeptide, comprise, or alternatively consist of, a polypeptide having the amino acid sequence of a VH CDR2 contained in a VH region encoded by one or more polynucleotides of SEQ ID NOs:56 or 60 or referred to in Tables 2 or 3. In a preferred embodiment, antibodies that immunospecifically bind a C35 polypeptide, comprise, or alternatively consist of a polypeptide having the amino acid sequence of a VH CDR3 contained in a VH region encoded by one or more polynucleotides of SEQ ID NOs:56 or 60 or referred to in Tables 2 or 3. Molecules comprising, or alternatively consisting of, these antibodies, or antibody fragments or variants thereof, that immunospecifically bind to C35 polypeptide or a C35 polypeptide fragment or variant thereof are also disclosed, as are nucleic acid molecules encoding these antibodies, molecules, fragments and/or variants.

Disclosed herein are antibodies that immunospecifically bind to a polypeptide, or polypeptide fragment or variant of a C35 polypeptide, wherein said antibodies comprise, or alternatively consist of, a polypeptide having an amino acid sequence of any one, two, three, or more of the VL CDRs contained in a VL region encoded by one or more polynucleotides of SEQ ID NO:58 or referred to in Tables 2 or 3. In particular, disclosed herein are antibodies that immunospecifically bind a C35 polypeptide, comprising, or alternatively consisting of, a polypeptide having the amino acid sequence of a VL CDR1 contained in a VL region encoded by one or more polynucleotides of SEQ ID NO:58 or referred to in Tables 2 or 3. In another embodiment, antibodies that immunospecifically bind a C35 polypeptide, comprise, or alternatively consist of, a polypeptide having the amino acid sequence of a VL CDR2 contained in a VL region encoded by one or more polynucleotides of SEQ ID NO:58 or referred to in Tables 2 or 3. In a preferred embodiment, antibodies that immunospecifically bind a C35 polypeptide, comprise, or alternatively consist of a polypeptide having the amino acid sequence of a VL CDR3 contained in a VL region encoded by one or more polynucleotides of SEQ ID NO:58 or referred to in Tables 2 or 3. Molecules comprising, or alternatively consisting of, these antibodies, or antibody fragments or variants thereof, that immunospecifically bind to C35 polypeptide or a C35 polypeptide fragment or variant thereof are also disclosed, as are nucleic acid molecules encoding these antibodies, molecules, fragments and/or variants.

Disclosed herein are antibodies (including molecules comprising, or alternatively consisting of, antibody fragments or variants) that immunospecifically bind to a C35 polypeptide or polypeptide fragment or variant of a C35 polypeptide, wherein said antibodies comprise, or alternatively consist of, one, two, three, or more VH CDRs and one, two, three or more VL CDRs, as contained in a VH region or VL region encoded by one or more polypeptides of SEQ ID NOs:57, 59, or 61 or referred to in Tables 2 or 3. In particular, disclosed herein are antibodies that immunospecifically bind to a polypeptide or polypeptide fragment or variant of a C35 polypeptide, wherein said antibodies comprise, or alternatively consist of, a VH CDR1 and a VL CDR1, a VH CDR1 and a VL CDR2, a VH CDR1 and a VL CDR3, a VH CDR2 and a VL CDR1, VH CDR2 and VL CDR2, a VH CDR2 and a VL CDR3, a VH CDR3 and a VH CDR1, a VH CDR3 and a VL CDR2, a VH CDR3 and a VL CDR3, or any combination thereof, of the VH CDRs and VL CDRs contained in a VH region or VL region encoded by one or more polynucleotides of SEQ ID NOs:56, 58, or 60 or referred to in Tables 2 or 3. The one, two, three, or more VH CDRs and one, two, three, or more VL CDRs may be from clones H0009 and L0010, clones H0009 and 1F2K, clones H0009 and 1B3K, clone H009 and SEQ ID NO:58, clones 1F2G and 1F2K, clones 1F2G and 1B3K, clones 1F2G and L0010, clone 1F2G and SEQ ID NO:58, clones 1B3G and 1B3K, clones 1B3G and 1F2K, clones 1B3G and L0010, clone 1B3G and SEQ ID NO:58, SEQ ID NO:56 and SEQ ID NO:58, SEQ ID NO:56 and clone L0010, SEQ ID NO:56 and clone 1F2K, SEQ ID NO:56 and clone 1B3K, SEQ ID NO:60 and SEQ ID NO:58, SEQ ID NO:60 and clone L0010, SEQ ID NO:60 and clone 1F2K, or SEQ ID NO:60 and clone 1B3K. Molecules comprising, or alternatively consisting of, fragments or variants of these antibodies, that immunospecifically bind to C35 polypeptide are also disclosed , as are nucleic acid molecules encoding these antibodies, molecules, fragments or variants.

Most preferably the antibodies are human, chimeric (e.g., human.mouse chimeric), or humanized antibodies or antigen-binding antibody fragments , including, but not limited to, Fab, Fab' and F(ab')2, Fd, single-chain Fvs (scFv), diabodies, triabodies, tetrabodies, minibodies, single-chain antibodies, disulfide-linked Fvs (sdFv), and intrabodies, and fragments comprising either a VL or VH region. Antigen-binding antibody fragments, including single-chain antibodies, may comprise the variable region(s) alone or in combination with the entirety or a portion of the following: hinge region, CHI, CH2, and CH3 domains. Also included are antigen-binding fragments also comprising any combination of variable region(s) with a hinge region, CH1, CH2, and CH3 domains. Preferred C35 antibodies in the therapeutic methods are those containing a deletion of the CH2 domain.

Antibodies may be described or specified in terms of the epitope(s) or portion(s) of a polypeptide which they recognize or specifically bind. The epitope(s) or polypeptide portion(s) may be specified as described herein, e.g., by N-terminal and C-terminal positions, or by size in contiguous amino acid residues. Antibodies which specifically bind any epitope or polypeptide may also be excluded. Therefore disclosed herein are antibodies that specifically bind polypeptides , and allows for the exclusion of the same.

Antibodies may also be described or specified in terms of their binding affinity to a polypeptide. Preferred binding affinities include those with a dissociation constant or Kd less than 5 X 10(-7) M, 10(-7) M, 5 X 10(-8) M, 10(-8) M, 5 X 10(-9) M, 10(-9) M, 5 X 10(-10) M, 10(-10) M, 5 X 10(-11) M, 10(-11) M, 5 X 10(-12) M, 10(-12) M, 5 X 10(-13) M, 10(-13) M, 5 X 10(-14) M, 10(-14) M, 5 X 10(-15) M, or 10(-15) M.

Antibodies have an affinity for C35 the same as or similar to the affinity of the antibodies 1F2, 1B3, MAb 165, MAb 171, or MAbc009. Preferably, the antibodies have an affinity for C35 that is higher than the affinity of the antibodies 1F2, 1B3, MAb 165, MAb 171, or MAbc009.

Disclosed herein are antibodies that competitively inhibit binding of an antibody to a C35 epitope as determined by any method known in the art for determining competitive binding, for example, the immmoassays and antibody binding assays described herein. In preferred embodiments, the antibody competitively inhibits binding to the epitope by at least 95%, at least 90%, at least 85%, at least 80%, at least 75%, at least 70%, at least 60%, or at least 50%.

Antibodies may also be described or specified in terms of their cross-reactivity. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide are also included. In specific embodiments, antibodies cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide are also included . In a specific embodiment, the above-described cross-reactivity is with respect to any single specific antigenic or immunogenic polypeptide, or combination(s) of 2, 3, 4, 5, or more of the specific antigenic and/or immunogenic polypeptides disclosed herein. Further included are antibodies which bind polypeptides encoded by polynucleotides which hybridize to a polynucleotide of the present invention under stringent hybridization conditions (as described herein).

Antibodies may be described or specified in terms of the epitope(s) or portion(s) of a polypeptide of the present invention which they recognize or specifically bind. The epitope(s) or polypeptide portion(s) may be specified as described herein, e.g., by N-terminal and C-terminal positions, by size in contiguous amino acid residues, or listed in the Tables and Figures. Antibodies which specifically bind any epitope or polypeptide may also be excluded. Therefore, disclosed herein are antibodies that specifically bind polypeptides , and allows for the exclusion of the same. Excluded are antibodies against C35 disclosed in US 2002/0052308 and/or WO 01/74859, and/or antibodies that specifically bind an epitope disclosed therein.

In a specific embodiment, antibodies bind to an epitope contained within the fragment represented by residues 105 to 115 of the native C35 sequence. In another embodiment, antibodies bind to an epitope contained within the fragment represesnted by residues 53-104 of the native C35 sequence.

Antibodies may also be described or specified in terms of their cross-reactivity, or lack thereof. Antibodies that do not bind any other analog, ortholog, or homolog of a polypeptide of the present invention are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide are also included. In specific embodiments, antibodies cross-react with murine, monkey, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide are also included. In a specific embodiment, the above-described cross-reactivity is with respect to any single specific antigenic or immunogenic polypeptide, or combination(s) of 2, 3, 4, 5, or more of the specific antigenic and/or immunogenic polypeptides disclosed herein. Further included are antibodies which bind polypeptides encoded by polynucleotides which hybridize to a polynucleotide under stringent hybridization conditions (as described herein).

In preferred embodiments, the antibodies (including molecules comprising, or alternatively consisting of, antibody fragments or variants thereof), immunospecifically bind to C35 polypeptide and do not cross-react with any other antigens.

In a preferred embodiment, antibodies preferentially bind C35 polypeptide (SEQ ID NO:2), or fragments and variants thereof relative to their ability to bind other antigens.

By way of non-limiting example, an antibody may be considered to bind a first antigen preferentially if it binds said first antigen with a dissociation constant (KD) that is less than the antibody's KD for the second antigen. In another non-limiting embodiment, an antibody may be considered to bind a first antigen preferentially if it binds said first antigen with an affinity that is at least one order of magnitude less than the antibody's KD for the second antigen. In another non-limiting embodiment, an antibody may be considered to bind a first antigen preferentially if it binds said first antigen with an affinity that is at least two orders of magnitude less than the antibody's KD for the second antigen.

In another non-limiting embodiment, an antibody may be considered to bind a first antigen preferentially if it binds said first antigen with an off rate (k(off)) that is less than the antibody's k(off) for the second antigen. In another non-limiting embodiment, an antibody may be considered to bind a first antigen preferentially if it binds said first antigen with an affinity that is at least one order of magnitude less than the antibody's k(off) for the second antigen. In another non-limiting embodiment, an antibody may be considered to bind a first antigen preferentially if it binds said first antigen with an affinity that is at least two orders of magnitude less than the antibody's k(off) for the second antigen.

In specific embodiments, antibodies bind C35 polypeptides or fragments or variants thereof with an off rate (k(off)) of less than or equal to 5 X 10(-2) sec-1, 10(-2) sec-1, 5 X 10(-3) sec-1 or 10(-3) sec-1. More preferably, antibodies of the invention bind C35 polypeptides or fragments or variants thereof with an off rate (k(off)) less than or equal to 5 X 10(-4) sec-1, 10(-4) sec-1, 5 X 10(-5) sec-1, or 10(-5) sec-1 5 X 10(-6) sec-1, 10(-6) sec-1, 5 X 10(-7) sec-1 or 10(-7) sec-1.

In other embodiments, antibodies bind C35 polypeptides or fragments or variants thereof with an on rate (k(on)) of greater than or equal to 10(3) M-1 sec-1, 5 X 10(3) M-1 sec-1, 10(4) M-1 sec-1 or 5 X 10(4) M-1 sec-1. More preferably, antibodies bind C35 polypeptides or fragments or variants thereof with an on rate (k(on)) greater than or equal to 10(5) M-1 sec-1, 5 X 10(5) M-1 sec-1, 10(6) M-1 sec-1, or 5 X 10(6) M-1 sec-1 or 10(7) M-1 sec-1.

Disclosed herein are antibodies that comprise, or alternatively consist of, variants (including derivatives) of the antibody molecules (e.g., the VH regions and/or VL regions) described herein, which antibodies immunospecifically bind to a C35 polypeptide or fragment or variant thereof. Standard techniques known to those of skill in the art can be used to introduce mutations in the nucleotide sequence encoding a molecule of the invention, including, for example, site-directed mutagenesis and PCR-mediated mutagenesis which result in amino acid substitutions. Preferably, the variants (including derivatives) encode less than 50 amino acid substitutions, less than 40 amino acid subsitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference VH region, VHCDR1, VHCDR2, VHCDR3, VL region, VLCDR1, VLCDR2, or VLCDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity (e.g., the ability to bind a C35 polypeptide).

For example, it is possible to introduce mutations only in framework regions or only in CDR regions of an antibody molecule. Introduced mutations may be silent or neutral missense mutations, i.e., have no, or little, effect on an antibody's ability to bind antigen. These types of mutations may be useful to optimize codon usage, or improve a hybridoma's antibody production. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen binding activity or alteration in binding activity (e.g., improvements in antigen binding activity or change in antibody specificity). Following mutagenesis, the encoded protein may routinely be expressed and the functional and/or biological activity of the encoded protein, (e.g., ability to immunospecifically bind a C35 polypeptide) can be determined using techniques described herein or by routinely modifying techniques known in the art.

In a specific embodiment, an antibody (including a molecule comprising, or alternatively consisting of, an antibody fragment or variant thereof), that immunospecifically binds C35 polypeptides or fragments or variants thereof, comprises, or alternatively consists of, an amino acid sequence encoded by a nucleotide sequence that hybridizes to a nucleotide sequence that is complementary to that encoding one of the VH or VL regions encoded by one or more of the nucleic acids of SEQ ID NOs:56, 58, or 60 or referred to in Tables 2 or 3 under stringent conditions, e.g., hybridization to filter-bound DNA in 6X sodium chloride/sodium citrate (SSC) at about 45° C followed by one or more washes in 0.2xSSC/0.1% SDS at about 50-65° C, under highly stringent conditions, e.g., hybridization to filter-bound nucleic acid in 6xSSC at about 45° C followed by one or more washes in 0.1xSSC/0.2% SDS at about 68° C, or under other stringent hybridization conditions which are know to those of skill in the art (see, for example, Ausubel, F.M. et al., eds. , 1989, Current Protocols in Molecular Biology, Vol. I, Green Publishing Associates, Inc. and John Wiley & Sons, Inc., New York at pages 6.3.1-6.3.6 and 2.10.3). Nucleic acid molecules encoding these antibodies are also disclosed herein.

It is well known within the art that polypeptides, or fragments or variants thereof, with similar amino acid sequences often have similar structure and many of the same biological activities. Thus, in one embodiment, an antibody (including a molecule comprising, or alternatively consisting of, an antibody fragment or variant thereof), that immunospecifically binds to a C35 polypeptide or fragments or variants of a C35 polypeptide, comprises, or alternatively consists of, a VH region having an amino acid sequence that is at least 35%, at least 40%, at least 45%, at least 50%, at least 55%, at least 60%, at least 65%, at least 70%, at least 75%, at least 80%, at least 85%, at least 90%, at least 95%, or at least 99% identical, to the amino acid sequence of a VH region encoded by a nucleic acid of SEQ ID NOs:56 or 60 or referred to in Tables 2 or 3.

In another embodiment, an antibody (including a molecule comprising, or alternatively consisting of, an antibody fragment or variant thereof), that immunospecifically binds to a C35 polypeptide or fragments or variants of a C35 polypeptide, comprises, or alternatively consists of, a VL region having an amino acid sequence that is at least 35%, at least 40%, at least 45%, at least 50%, at least 55%, at least 60%, at least 65%, at least 70%, at least 75%, at least 80%, at least 85%, at least 90%, at least 95%, or at least 99% identical, to the amino acid sequence of a VL region encoded by a nucleic acid of SEQ ID NO:58 or referred to in Tables 2 or 3.

Disclosed herein are antibodies (including molecules comprising, or alternatively consisting of, antibody fragments or variants thereof) that have one or more of the same biological characteristics as one or more of the antibodies described herein. By "biological characteristics" is meant, the in vitro or in vivo activities or properties of the antibodies, such as, for example, the ability to bind to C35 polypeptide (e.g., C35 polypeptide expressed on a cell surface during apoptosis); the ability to substantially inhibit or abolish C35 polypeptide mediated biological activity; the ability to kill C35-associated cancer cells (e.g., treat or diagnose C35-associated cancer), or detect C35; the ability to inhibit or abolish C35 polypeptide mediated biological activity. Optionally, the antibodies of the invention will bind to the same epitope as at least one of the antibodies specifically referred to herein. Such epitope binding can be routinely determined using assays known in the art.

The rules described below for producing humanized antibodies derived from mouse VH and VL regions encoded by the nucleic acids referred to in Table 2 may also be used to produce antibody variants comprising the human VH and/or VL regions encoded by SEQ ID NOs: 56, 58, or 60 or by the nucleic acids referred to in Table 3.

Humanized immunoglobulins and human antibody variants of the invention have variable framework regions substantially from a human immunoglobulin (termed an acceptor immunoglobulin), and CDRs substantially from the mouse C35 VH and VL regions encoded by the clones in Table 2 or from the human C35 VH and VL regions encoded by the clones in Table 3 (referred to as the donor immunoglobulin). The constant region(s), if present, are also substantially from a human immunoglobulin. The humanized antibodies and human antibody variants exhibit a specific binding affinity for C35 of at least 10(2), 10(3), 10(4), 10(5), 10(6), 10(7), 10(8), 10(9), or 10(10) M(-1). Usually the upper limit of binding affinity of the humanized antibodies and human antibody variants for human C35 is within a factor of 3, 4, 5 or 10 of that of the mouse antibodies 1F2 or 1B3 or the human antibody MAbc009, or of antibodies MAb 165 or MAb 171. Often the lower limit of binding affinity is also within a factor of 3, 4, 5 or 10 of that of the mouse antibodies in 1F2 or 1B3 or human antibody MAbc009, or of antibodies MAb 165 or MAb 171. Preferred humanized immunoglobulins and human antibody variants compete with the mouse antibodies 1F2 or 1B3 or human antibody MAbc009, or antibodies MAb 165 or MAb 171 for binding to C35 and prevent C35 from binding to the respective mouse or human antibody.

The heavy and light chain variable regions of possible human acceptor antibodies are described by Kabat, Sequences of Proteins of Immunological Interest (National Institutes of Health, Bethesda, Md., 1987 and 1991). The human acceptor antibody is chosen such that its variable regions exhibit a high degree of sequence identity with those of the mouse C35 antibody. The heavy and light chain variable framework regions can be derived from the same or different human antibody sequences. The human antibody sequences can be the sequences of naturally occurring human antibodies or can be consensus sequences of several human antibodies.

The design of humanized immunoglobulins can be carried out as follows. When an amino acid falls under the following category, the framework amino acid of a human immunoglobulin to be used (acceptor immunoglobulin) is replaced by a framework amino acid from a CDR-providing non-human immunoglobulin (donor immunoglobulin):

(a) the amino acid in the human framework region of the acceptor immunoglobulin is unusual for human immunoglobulins at that position, whereas the corresponding amino acid in the donor immunoglobulin is typical for human immunoglobulins in that position;

(b) the position of the amino acid is immediately adjacent to one of the CDRs; or

(c) the amino acid is capable of interacting with the CDRs (see, Queen et al. WO 92/11018., and Co et al., Proc. Natl. Acad. Sci. USA 88, 2869 (1991), respectively, both of which are incorporated herein by reference). For a detailed description of the production of humanized immunoglobulins see, Queen et al. and Co et al.

Usually the CDR regions in humanized antibodies and human antibody variants are substantially identical, and more usually, identical to the corresponding CDR regions in the mouse or human antibody from which they were derived. Although not usually desirable, it is sometimes possible to make one or more conservative amino acid substitutions of CDR residues without appreciably affecting the binding affinity of the resulting humanized immunoglobulin or human antibody variant. Occasionally, substitutions of CDR regions can enhance binding affinity.

Other than for the specific amino acid substitutions discussed above, the framework regions of humanized immunoglobulins and human antibody variants are usually substantially identical, and more usually, identical to the framework regions of the human antibodies from which they were derived (acceptor immunoglobulin). Of course, many of the amino acids in the framework region make little or no direct contribution to the specificity or affinity of an antibody. Thus, many individual conservative substitutions of framework residues can be tolerated without appreciable change of the specificity or affinity of the resulting humanized immunoglobulin or human antibody variants.

For example, analogs of HuZAF show substantial amino acid sequence identity with HuZAF. Heavy and light chain variable regions of analogs are encoded by nucleic acid sequences that hybridize with the nucleic acids encoding the heavy or light chain variable regions of HuZAF, or degenerate forms thereof, under stringent conditions. Phage-display technology offers powerful techniques for selecting such analogs while retaining binding affinity and specificity (see, e.g., Dower et al., WO 91/17271; McCafferty et al., WO 92/01047; and Huse, WO 92/06204 (each of which is incorporated by reference in its entirety for all purposes).

The VH and VL genes in the nucleic acid clones in Tables 2 or 3 or SEQ ID NO:s 56, 58, or 60 can be employed to select fully human antibodies specific for C35 according to the method taught by US 2002 0123057A1, "In vitro methods of producing and identifying immunoglobulin molecules in eukaryotic cells," published 5 September 2002. Briefly, the mouse (or human) VH linked to a human CH is employed to select fully human immunoglobulin light chains from a library of such light chains that when paired with the mouse (or human) VH confers specificity for C35. The selected fully human immunoglobulin light chains are then employed to select fully human immunoglobulin heavy chains from a library of such heavy chains that when paired with the fully human light chain confer specificity for C35. Similarly, the mouse (or human) VL linked to a human CL may be employed to select fully human immunoglobulin heavy chains from a library of such heavy chains that when paired with the mouse (or human) VL confers specificity for C35. The selected fully human immunoglobulin heavy chains are then employed to select fully human immunoglobulin light chains from a library of such light chains that when paired with the fully human heavy chain confer specificity for C35. Frequently, the fully human antibody selected in this fashion has epitope specificity that is identical or closely related to that of the original mouse (or human) C35-specific antibody.

The method of US 2002 0123057 A1 may also be used with a library of heavy or light chains of which all members have one or more non-human (e.g., mouse) CDRs. In one example, each member of the library comprises a CDR3 region derived from an isolated murine monoclonal antibody specific for C35, e.g., 1F2 or 1B3.

All fully human antibodies or antibodies having one or more non-human (e.g., mouse) CDRs (including molecules comprising, or alternatively consisting of, antibody fragments or variants thereof) selected through use of the method of US 2002 0123057 A1 starting with immunoglobulin heavy or light chain variable regions encoded by the nucleic acids of SEQ ID NO:s 56, 58, or 60 or referred to in Tables 2 or 3 are encompassed in the present invention.

The variable segments of humanized antibodies or human antibody variants produced as described supra are typically linked to at least a portion of an immunoglobulin constant region (Fc), typically that of a human immunoglobulin. Human constant region DNA sequences can be isolated in accordance with well-known procedures from a variety of human cells, such as immortalized B-cells (see Kabat et al., supra, and WO 87/02671). The antibody may contain both light chain and heavy chain constant regions. The heavy chain constant region may include CH1, hinge, CH2, CH3, and, sometimes, CH4 regions. For therapeutic purposes, the CH2 domain may be deleted or omitted.

The humanized antibody or human antibody variants include antibodies having all types of constant regions, including IgM, IgG, IgD, IgA and IgE, and any isotype, including IgG1, IgG2, IgG3 and IgG4. When it is desired that the humanized antibody or human antibody variants exhibit cytotoxic activity, the constant domain is usually a complement-fixing constant domain and the class is typically IgG1. When such cytotoxic activity is not desirable, the constant domain can be of the IgG2 class. The humanized antibody or human antibody variants may comprise sequences from more than one class or isotype.

Chimeric antibodies are also disclosed herein. Such antibodies may comprise the VH region and/or VL region encoded by the nucleic acids of SEQ ID NOs:56, 58, or 60 or in Tables 2 or 3 fused to the CH region and/or CL region of a another species, such as human or mouse or horse, etc. In preferred embodiments, a chimeric antibody comprises the VH and/or VL region encoded by the nucleic acids of Table 2 fused to human C regions. The human CH2 domain may be deleted when antibodies are used in therapeutic purposes. Chimeric antibodies encompass antibody fragments, as described above.

The variable segments of chimeric antibodies are typically linked to at least a portion of an immunoglobulin constant region (Fc), typically that of a human immunoglobulin. Human constant region DNA sequences can be isolated in accordance with well-known procedures from a variety of human cells, such as immortalized B-cells (see Kabat et al., supra, and WO 87/02671). The antibody may contain both light chain and heavy chain constant regions. The heavy chain constant region may include CH1, hinge, CH2, CH3, and, sometimes, CH4 regions. For therapeutic purposes, the CH2 domain may be deleted or omitted.

The variable segments of chimeric antibodies produced as described supra are typically linked to at least a portion of an immunoglobulin constant region (Fc), typically that of a human immunoglobulin. Human constant region DNA sequences can be isolated in accordance with well-known procedures from a variety of human cells, such as immortalized B-cells (see Kabat et aL, supra, and WO 87/02671). The antibody may contain both light chain and heavy chain constant regions. The heavy chain constant region may include CH1, hinge, CH2, CH3, and, sometimes, CH4 regions. For therapeutic purposes, the CH2 domain may be deleted or omitted.

Chimeric antibodies include antibodies having all types of constant regions, including IgM, IgG, IgD, IgA and IgE, and any isotype, including IgG1, IgG2, IgG3 and IgG4. When it is desired that the chimeric antibody exhibit cytotoxic activity, the constant domain is usually a complement-fixing constant domain and the class is typically IgG1. When such cytotoxic activity is not desirable, the constant domain can be of the IgG2 class. The chimeric antibody may comprise sequences from more than one class or isotype.

A variety of methods are available for producing such immunoglobulins. Because of the degeneracy of the genetic code, a variety of nucleic acid sequences encode each immunoglobulin amino acid sequence. The desired nucleic acid sequences can be produced by de novo solid-phase DNA synthesis or by PCR mutagenesis of an earlier prepared variant of the desired polynucleotide. All nucleic acids encoding the antibodies described in this application are disclosed herein.

Once expressed, the whole antibodies, their dinners, individual light and heavy chains, or other immunoglobulin forms can be purified according to standard procedures in the art, including ammonium sulfate precipitation, affinity columns, column chromatography, gel electrophoresis and the like (see, generally, Scopes, R, Protein Purification, Springer-Verlag, N.Y. (1982) ). Substantially pure immunoglobulins of at least about 90 to 95% homogeneity are preferred, and 98 to 99% or more homogeneity most preferred, for pharmaceutical uses. Once purified, partially or to homogeneity as desired, the polypeptides may then be used therapeutically (including extracorporeally), in developing and performing assay procedures, immunofluorescent stainings, and the like. (See, generally, Immunological Methods, Vols. I and II, Lefkovits and Pernis, eds., Academic Press, New York, N.Y. (1979 and 1981), or detect C35 or diagnose a C35-associated cancer.

Disclosed herein are fusion proteins comprising, or alternatively consisting of, an antibody (including molecules comprising, or alternatively consisting of, antibody fragments or variants thereof), that immunospecifically binds to C35 polypeptide, and a heterologous polypeptide. Preferably, the heterologous polypeptide to which the antibody is fused is useful for function or is useful to target the C35 polypeptide expressing cells, including but not limited to breast, ovarian, bladder, colon, and pancreatic cancer cells, and melanoma cells. In an alternative preferred embodiment, the heterologous polypeptide to which the antibody is fused is useful for T cell, macrophage, and/or monocyte cell function or is useful to target the antibody to a T cell, macrophage, or monocyte. In one embodiment, a fusion protein, comprises, or alternatively consists of, a polypeptide having the amino acid sequence of any one or more of the VH regions of an antibody or the amino acid sequence of any one or more of the VL regions of an antibody or fragments or variants thereof, and a heterologous polypeptide sequence. In another embodiment, a fusion protein comprises, or alternatively consists of, a polypeptide having the amino acid sequence of any one, two, three, or more of the VH CDRs of an antibody, or the amino acid sequence of any one, two, three, or more of the VL CDRs of an antibody, or fragments or variants thereof, and a heterologous polypeptide sequence. In a preferred embodiment, the fusion protein comprises, or alternatively consists of a polypeptide having the amino acid sequence of a VH CDR3 of an antibody , or fragment or variant thereof, and a heterologous polypeptide sequence, which fusion protein immunospecifically binds to C35 polypeptide. In another embodiment, a fusion protein comprises, or alternatively consists of a polypeptide having the amino acid sequence of at least one VH region of an antibody and the amino acid sequence of at least one VL region of an antibody or fragments or variants thereof, and a heterologous polypeptide sequence. Preferably, the VH and VL regions of the fusion protein correspond to a single antibody (or scFv or Fab fragment). In yet another embodiment, a fusion protein comprises, or alternatively consists of a polypeptide having the amino acid sequence of any one, two, three or more of the VH CDRs of an antibody and the amino acid sequence of any one, two, three or more of the VL CDRs of an antibody or fragments or variants thereof, and a heterologous polypeptide sequence. Preferably, two, three, four, five, six, or more of the VHCDR(s) or VLCDR(s) correspond to single antibody (or scFv or Fab fragment) Nucleic acid molecules encoding these fusion proteins are also disclosed herein.

As discussed in more detail below, the antibodies may be used either alone or in combination with other compositions. The antibodies may further be recombinantly fused to a heterologous polypeptide at the N- or C-terminus or chemically conjugated (including covalent and non-covalent conjugations) to polypeptides or other compositions. For example, antibodies may be recombinantly fused or conjugated to molecules useful as labels in detection assays and effector molecules such as heterologous polypeptides, drugs, radionuclides, or toxins. See, e.g., PCT publications WO 92/08495; WO 91/14438; WO 89/12624; U.S. Patent No. 5,314,995; and EP 396,387.

By way of another non-limiting example, antibodies may be administered to individuals as a form of passive immunization. Alternatively, antibodies may be used for epitope mapping to identify the epitope(s) bound by the antibody. Epitopes identified in this way may, in turn, for example, be used as vaccine candidates, i.e., to immunize an individual to elicit antibodies against the naturally occurring forms of C35 for therapeutic methods.

Antibodies may act as agonists or antagonists of the C35 polypeptides.

Antibodies may be used, for example, but not limited to, to purify, detect, and target the polypeptides including both in vitro and in vivo diagnostic and therapeutic methods. For example, the antibodies have use in immunoassays for qualitatively and quantitatively measuring levels of the polypeptides in biological samples. See, e.g., Harlow et al., Antibodies: A Laboratory Manual, (Cold Spring Harbor Laboratory Press, 2nd ed. 1988).

The antibodies include derivatives that are modified, i.e, by the covalent attachment of any type of molecule to the antibody such that covalent attachment does not prevent the antibody from generating an anti-idiotypic response or binding C35. For example, but not by way of limitation, the antibody derivatives include antibodies that have been modified, e.g., by glycosylation, acetylation, pegylation, phosphylation, phosphorylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

Antibodies can be composed of amino acids joined to each other by peptide bonds or modified peptide bonds, i.e., peptide isosteres, and may contain amino acids other than the 20 gene-encoded amino acids. The C35 antibodies may be modified by natural processes, such as posttranslational processing, or by chemical modification techniques which are well known in the art. Such modifications are well described in basic texts and in more detailed monographs, as well as in a voluminous research literature. Modifications can occur anywhere in the C35 antibody, including the peptide backbone, the amino acid side-chains and the amino or carboxyl termini. It will be appreciated that the same type of modification may be present in the same or varying degrees at several sites in a given C35 antibody. Also, a given C35 antibody may contain many types of modifications. C35 antibodies may be branched, for example, as a result of ubiquitination, and they may be cyclic, with or without branching. Cyclic, branched, and branched cyclic C35 antibodies may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

Disclosed herein is a polypeptide which comprises the amino acid sequence of a C35 antibody sequence having an amino acid sequence which contains at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions. Of course, in order of ever-increasing preference, it is highly preferable for a polypeptide to have an amino acid sequence which comprises a C35 antibody sequence, which contains at least one, but not more than 10, 9, 8, 7, 6, 5, 4, 3, 2 or 1 amino acid substitutions. In specific embodiments, the number of additions, substitutions, and/or deletions in the C35 antibody sequence is 1-5, 5-10, 5-25, 5-50, 10-50 or 50-150. For substitutions, conservative amino acid substitutions are preferable. The substitutions may be within the framework regions or the CDRs or both.

The description in this section applies to C35 antibodies and to other antibodies useful in the method . Such antibodies may be conjugated to or complexed with a toxin, as described herein, or may be unconjugated or uncomplexed.

### Polynucleotides Encoding C35 Antibodies

Disclosed herein are nucleic acid molecules encoding a C35 antibody (including molecules comprising, or alternatively consisting of, antibody fragments or variants thereof).

Disclosed herein are polynucleotides that hybridize under stringent or alternatively, under lower stringency hybridization conditions, e.g., as defined supra, to polynucleotides that encode an antibody, preferably, that specifically binds to a C35 polypeptide.

In a specific embodiment, a nucleic acid molecule encodes an antibody (including molecules comprising, or alternatively consisting of, antibody fragments or variants thereof), comprising, or alternatively consisting of, a VH region having an amino acid sequence of any one of the VH regions encoded by a nucleic acid and a VL region having an amino acid sequence of any one of the VL regions encoded by a nucleic acid In another embodiment, a nucleic acid molecule encodes an antibody (including molecules comprising, or alternatively consisting of, antibody fragments or variants thereof), comprising, or alternatively consisting of, a VH region having an amino acid sequence of any one of the VH regions encoded by a nucleic acid of the invention or a VL region having an amino acid sequence of any one of the VL regions encoded by a nucleic acid

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably poly A+RNA, isolated from, any tissue or cells expressing the antibody, such as hybridoma cells selected to express an antibody) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, N.Y. and Ausubel et al., eds., 1998, Current Protocols in Molecular Biology, John Wiley & Sons, NY,), to generate antibodies having a different amino acid sequence, for example to create amino acid substitutions, deletions, and/or insertions.

In a specific embodiment, the amino acid sequence of the heavy and/or light chain variable domains may be inspected to identify the sequences of the complementarity determining regions (CDRs) by methods that are well know in the art, e.g., by comparison to known amino acid sequences of other heavy and light chain variable regions to determine the regions of sequence hypervariability. Using routine recombinant DNA techniques, one or more of the CDRs may be inserted within framework regions, e.g., into human framework regions to humanize a non-human antibody, as described supra. The framework regions may be naturally occurring or consensus framework regions, and preferably human framework regions (see, e.g., Chothia et al., J. Mol. Biol. 278: 457-479 (1998) for a listing of human framework regions). Preferably, the polynucleotide generated by the combination of the framework regions and CDRs encodes an antibody that specifically binds C35. Preferably, as discussed supra, one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are within the skill of the art.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Pat. No. 4,946,778; Bird, Science 242:423-42 (1988); Huston et al., Proc. Natl. Acad. Sci. USA 85:5879-5883 (1988); and Ward et al., Nature 334:544-54 (1989)) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in *E. coli* may also be used (Skerra et al., Science 242:1038-1041 (1988)).

Disclosed herein are nucleic acid molecules encoding an antibody (including molecules comprising, or alternatively consisting of, antibody fragments or variants thereof). In a specific embodiment, a nucleic acid molecule encodes an antibody (including molecules comprising, or alternatively consisting of, antibody fragments or variants thereof), comprising, or alternatively consisting of, a VH region having an amino acid sequence of any one of the VH regions encoded by a nucleic acid of SEQ ID NO:s 56 or 60 or referred to in Table 2 or 3 and a VL region having an amino acid sequence of any one of the VL regions encoded by a nucleic acid of SEQ ID NO:58 or referred to in Table 2 or 3. In another embodiment, a nucleic acid molecule encodes an antibody (including molecules comprising, or alternatively consisting of, antibody fragments or variants thereof), comprising, or alternatively consisting of, a VH region having an amino acid sequence of any one of the VH regions encoded by a nucleic acid of SEQ ID NOs:56 or 60 or referred to in Table 2 or 3 or a VL region having an amino acid sequence of any one of the VL regions encoded by a nucleic acid of SEQ ID NO:58 or referred to in Table 2 or 3.

Disclosed herein are antibodies that comprise, or alternatively consist of, variants (including derivatives) of the antibody molecules (e.g., the VH regions and/or VL regions) described herein, which antibodies immunospecifically bind to a C35 polypeptide or fragment or variant thereof. Standard techniques known to those of skill in the art can be used to introduce mutations in the nucleotide sequence , including, for example, site-directed mutagenesis and PCR-mediated mutagenesis which result in amino acid substitutions. Preferably, the variants (including derivatives) encode less than 50 amino acid substitutions, less than 40 amino acid subsitutions, less than 30 amino acid substitutions, less than 25 amino acid substitutions, less than 20 amino acid substitutions, less than 15 amino acid substitutions, less than 10 amino acid substitutions, less than 5 amino acid substitutions, less than 4 amino acid substitutions, less than 3 amino acid substitutions, or less than 2 amino acid substitutions relative to the reference VH region, VHCDR1, VHCDR2, VHCDR3, and/or VL region, VLCDR1, VLCDR2, or VLCDR3. A "conservative amino acid substitution" is one in which the amino acid residue is replaced with an amino acid residue having a side chain with a similar charge. Families of amino acid residues having side chains with similar charges have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity (e.g., the ability to bind a C35 polypeptide).

For example, it is possible to introduce mutations only in framework regions or only in CDR regions, or both regions, of an antibody molecule. Introduced mutations may be silent or neutral missense mutations, Le., have no, or little, effect on an antibody's ability to bind antigen. These types of mutations may be useful to optimize codon usage, or improve a hybridoma's antibody production. Alternatively, non-neutral missense mutations may alter an antibody's ability to bind antigen. The location of most silent and neutral missense mutations is likely to be in the framework regions, while the location of most non-neutral missense mutations is likely to be in CDR, though this is not an absolute requirement. One of skill in the art would be able to design and test mutant molecules with desired properties such as no alteration in antigen binding activity or alteration in binding activity (e.g., improvements in antigen binding activity or change in antibody specificity). Following mutagenesis, the encoded protein may routinely be expressed and the functional and/or biological activity of the encoded protein, (e.g., ability to immunospecifically bind a C35 polypeptide) can be determined using techniques described herein or by routinely modifying techniques known in the art.

In a specific embodiment, an antibody (including a molecule comprising, or alternatively consisting of, an antibody fragment or variant thereof), that immunospecifically binds C35 polypeptides or fragments or variants thereof, comprises, or alternatively consists of, an amino acid sequence encoded by a nucleotide sequence that hybridizes to a nucleotide sequence that is complementary to that encoding one of the VH or VL regions encoded by a nucleic acid of SEQ ID NO:s 56, 58, or 60 or referred to in Table 2 or 3 under stringent conditions, e.g., hybridization to filter-bound DNA in 6X sodium chloride/sodium citrate (SSC) at about 45° C followed by one or more washes in 0.2xSSC/0.1% SDS at about 50-65° C, under highly stringent conditions, e.g., hybridization to filter-bound nucleic acid in 6xSSC at about 45° C followed by one or more washes in 0.1xSSC/0.2% SDS at about 68° C, or under other stringent hybridization conditions which are known to those of skill in the art (see, for example, Ausubel, F.M. et al., eds. , 1989, Current Protocols in Molecular Biology, Vol. I, Green Publishing Associates, Inc. and John Wiley & Sons, Inc., New York at pages 6.3.1-6.3.6 and 2.10.3). Nucleic acid molecules encoding these antibodies are disclosed herein.

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences, or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone generated using oligo dT as a primer).

The C35 antibody polynucleotide can be composed of any polyribonucleotide or polydeoxribonucleotide, which may be unmodified RNA or DNA or modified RNA or DNA. For example, C35 antibody polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the C35 antibody polynucleotides can be composed of triple-stranded regions comprising RNA or DNA or both RNA and DNA. C35 antibody polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

Antibodies (including antibody fragments or variants) can be produced by any method known in the art. For example, it will be appreciated that antibodies can be expressed in cell lines other than hybridoma cell lines. Sequences encoding the cDNAs or genomic clones for the particular antibodies can be used for transformation of a suitable mammalian or nonmammalian host cells or to generate phage display libraries, for example. Additionally, polypeptide antibodies may be chemically synthesized or produced through the use of recombinant expression systems.

One way to produce the antibodies would be to clone the VH and/or VL regions encoded by any one or more of the nucleic acids of SEQ ID NOs: 56, 58, or 60 or referred to in Table 2 or 3. In order to isolate the VH and VL regions from the hybridoma cell lines or nucleic acids, PCR primers including VH or VL nucleotide sequences, may be used to amplify the VH and VL sequences contained in the vectors containing the nucleic acids of SEQ ID NOs: 56, 58, or 60 or Table 2 or 3. The PCR products may then be cloned using vectors, for example, which have a PCR product cloning site consisting of a 5' and 3' single T nucleotide overhang, that is complementary to the overhanging single adenine nucleotide added onto the 5' and 3' end of PCR products by many DNA polymerases used for PCR reactions. The VH and VL regions can then be sequenced using conventional methods known in the art.

The cloned VH and VL genes may be placed into one or more suitable expression vectors. By way of non-limiting example, PCR primers including VH or VL nucleotide sequences, a restriction site, and a flanking sequence to protect the restriction site may be used to amplify the VH or VL sequences. Utilizing cloning techniques known to those of skill in the art, the PCR amplified VH regions may be cloned into vectors expressing the appropriate immunoglobulin constant region, e.g., the human IgG1 or IgG4 constant region for VH regions, and the human kappa or lambda constant regions for kappa and lambda VL regions, respectively. Preferably, the vectors for expressing the VH or VL regions comprise a promoter suitable to direct expression of the heavy and light chains in the chosen expression system, a secretion signal, a cloning site for the immunoglobulin variable domain, immunoglobulin constant domains, and a selection marker such as neomycin. The VH and VL regions may also be cloned into a single vector expressing the necessary constant regions. The heavy chain conversion vectors and light chain conversion vectors are then co-transfected into cell lines to generate stable or transient cell lines that express full-length antibodies, e.g., IgG, using techniques known to those of skill in the art (See, for example, Guo et al., J. Clin. Endocrinol. Metab. 82:925-31 (1997), and Ames et al., J. Immunol. Methods 184:177-86 (1995)).

Disclosed herein are polynucleotides comprising a nucleotide sequence encoding an antibody of the invention and fragments thereof. Disclosed herein are polynucleotides that hybridize under stringent or lower stringency hybridization conditions, e.g., as defined supra, to polynucleotides that encode an antibody, preferably, that specifically binds to a polypeptide preferably, an antibody that binds to a polypeptide having the amino acid sequence of SEQ ID NO:2 or a polypeptide encoded by the deposited clone.

For some uses, such as for in vitro affinity maturation of an antibody , it may be useful to express the VH and VL regions of the heavy and light chains of one or more antibodies as single chain antibodies or Fab fragments in a phage display library. For example, the cDNAs encoding the VH and VL regions of one or more antibodies may be expressed in all possible combinations using a phage display library, allowing for the selection of VH/VL combinations that bind a C35 polypeptides with preferred binding characteristics such as improved affinity or improved off rates. Additionally, VH and VL segments, the CDR regions of the VH and VL regions of one or more antibodies, in particular, may be mutated in vitro. Expression of VH and VL regions with "mutant" CDRs in a phage display library allows for the selection of VH/VL combinations that bind a C35 polypeptide receptor polypeptides with preferred binding characteristics such as improved affinity or improved off rates.

In phage display methods, functional antibody domains are displayed on the surface of phage particles which carry the polynucleotide sequences encoding them. In particular, DNA sequences encoding VH and VL regions are amplified from animal cDNA libraries (e.g., human or murine cDNA libraries of lymphoid tissues) or synthetic cDNA libraries. The DNA encoding the VH and VL regions are joined together by an scFv linker by PCR and cloned into a phagemid vector (e.g., p CANTAB 6 or pComb 3 HSS). The vector is electroporated in E. coli and the E. coli is infected with helper phage. Phage used in these methods are typically filamentous phage including fd and M13 and the VH and VL regions are usually recombinantly fused to either the phage gene III or gene VIII. Phage expressing an antigen binding domain that binds to an antigen of interest (i.e., a C35 polypeptide or a fragment thereof) can be selected or identified with antigen, e.g., using labeled antigen or antigen bound or captured to a solid surface or bead. Examples of phage display methods that can be used to make the antibodies include, but are not limited to, those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280(1994); PCT application No. PCT/GB91/O1 134; PCT publications WO 90/02809; WO 91/10737; WO 92/01047; WO 92/18719; WO 93/1 1236; WO 95/15982; WO 95/20401; WO97/13844; and U.S. Patent Nos. 5,698,426; 5,223,409; 5,403,484; 5,580,717; 5,427,908; 5,750,753; 5,821,047; 5,571,698; 5,427,908; 5,516,717; 5,780,225; 5,658,727; 5,735,743 and 5,969,108.

Alternatively, a preferred method for increasing the affinity of antibodies is disclosed in US 2002 0123057 A1.

In addition, techniques developed for the production of "chimeric antibodies" (Morrison et al., Proc. Natl. Acad. Sci. 81:851-855 (1984); Neuberger et al., Nature 312:604-608 (1984); Takeda et al., Nature 314:452-454 (1985)) by splicing genes from a mouse antibody molecule of appropriate antigen specificity together with genes from a human antibody molecule of appropriate biological activity can be used. As described supra, a chimeric antibody is a molecule in which different portions are derived from different animal species, such as those having a variable region derived from a murine mAb and a human immunoglobulin constant region, e.g., humanized antibodies.

Alternatively, techniques described for the production of single chain antibodies (U.S. Patent No. 4,946,778; Bird, Science 242:423- 42 (1988); Huston et al., Proc. Natl. Acad. Sci. USA. 85:5879-5883 (1988); and Ward et al., Nature 334:544-54 (1989)) can be adapted to produce single chain antibodies. Single chain antibodies are formed by linking the heavy and light chain fragments of the Fv region via an amino acid bridge, resulting in a single chain polypeptide. Techniques for the assembly of functional Fv fragments in E. coli may also be used (Skerra et al., Science 242:1038- 1041 (1988)).

### Assays For Antibody Binding

The antibodies may be assayed for immunospecific binding by any method known in the art. The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al, eds, 1994, Current Protocols in Molecular Biology, Vol. 1, John Wiley & Sons, Inc., New York.

### Exemplary immunoassays are described briefly below (but are not intended by way of limitation).

Immunoprecipitation protocols generally comprise lysing a population of cells in a lysis buffer such as RIPA buffer (1% NP-40 or Triton X-100, 1% sodium deoxycholate, 0.1% SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4.degree. C., adding protein A and/or protein G sepharose beads to the cell lysate, incubating for about an hour or more at 4.degree. C., washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8%-20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., 3H or 1251) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e.g., 3H or 1251) in the presence of increasing amounts of an unlabeled second antibody.

Characterization of the affinity and kinetics of monoclonal antibody (Mab) interactions with a specific antigen can be used to probe the structure and function of either the antibody or the antigen. Kinetic measurements are also important in the selection of appropriate reagents for immunoassay.

There are a variety of methods available for measuring the affinity of an antibody-antigen interaction, but relatively few for determining rate constants. Most of the methods rely on either labeling antibody or antigen, which inevitably complicates routine measurements and introduces uncertainties in the measured quantities.

Surface plasmon reasonance (SPR) as performed on BIAcore offers a number of advantages over conventional methods of measuring the affinity of antibody-antigen interactions: (i) no requirement to label either antibody or antigen; (ii) antibodies do not need to be purified in advance, cell culture supernatant can be used directly; (iii) real-time measurements, allowing rapid semi-quantitative comparison of different monoclonal antibody interactions, are enabled and are sufficient for many evaluation purposes; (iv) biospecific surface can be regenerated so that a series of different monoclonal antibodies can easily be compared under identical conditions; (v) analytical procedures are fully automated, and extensive series of measurements can be performed without user intervention. BIAapplications Handbook, version AB (reprinted 1998), BIACORE code No. BR-1001-86; BIAtechnology Handbook, version AB (reprinted 1998), BIACORE code No. BR-1001-84.

SPR based binding studies require that one member of a binding pair be immobilized on a sensor surface. The binding partner immobilized is referred to as the ligand. The binding partner in solution is referred to as the analyte. In some cases, the ligand is attached indirectly to the surface through binding to another immobilized molecule, which is referred as the capturing molecule. SPR response reflects a change in mass concentration at the detector surface as analytes bind or dissociate.

Based on SPR, real-time BIAcore measurements monitor interactions directly as they happen. The technique is well suited to determination of kinetic parameters. Comparative affinity ranking is extremely simple to perform, and both kinetic and affinity constants can be derived from the sensorgram data.

When analyte is injected in a discrete pulse across a ligand surface, the resulting sensorgram can be divided into three essential phases: (i) Association of analyte with ligand during sample injection; (ii) Equilibrium or steady state during sample injection, where the rate of analyte binding is balanced by dissociation from the complex; (iii) Dissociation of analyte from the surface during buffer flow.

The association and dissociation phases provide information on the kinetics of analyte-ligand interaction (kₐ and k_{d}, the rates of complex formation and dissociation, k_{d}/kₐ = K_{D}). The equilibrium phase provides information on the affinity of the analyte-ligand interaction (K_{D}).

BIAevaluation software provides comprehensive facilities for curve fitting using both numerical integration and global fitting algorithms. With suitable analysis of the data, separate rate and affinity constants for interaction can be obtained from simple BIAcore investigations. The range of affinities measurable by this technique is very broad ranging from mM to pM.

Epitope specificity is an important characteristic of a monoclonal antibody. Epitope mapping with BIAcore, in contrast to conventional techniques using radioimmunoassay, ELISA or other surface adsorption methods, does not require labeling or purified antibodies, and allows multi-site specificity tests using a sequence of several monoclonal antibodies. Additionally, large numbers of analyses can be processed automatically.

Pair-wise binding experiments test the ability of two MAbs to bind simultaneously to the same antigen. MAbs directed against separate epitopes will bind independently, whereas MAbs directed against closely related epitopes will interfere with each other's binding. These binding experiments with BIAcore are straightforward to carry out.

For example, one can use a capture molecule to bind the first Mab, followed by addition of antigen and second MAb sequentially. The sensorgrams will reveal: 1. how much of the antigen binds to first Mab, 2. to what extent the second MAb binds to the surface-attached antigen, 3. if the second MAb does not bind, whether reversing the order of the pair-wise test alters the results.

Peptide inhibition is another technique used for epitope mapping. This method can complement pair-wise antibody binding studies, and can relate functional epitopes to structural features when the primary sequence of the antigen is known. Peptides or antigen fragments are tested for inhibition of binding of different MAbs to immobilized antigen. Peptides which interfere with binding of a given MAb are assumed to be structurally related to the epitope defined by that MAb.

### Methods Of Producing Antibodies

The antibodies can be produced by any method known in the art for the synthesis of antibodies, in particular, by chemical synthesis or preferably, by recombinant expression techniques.

Recombinant expression of an antibody, or fragment, derivative or analog thereof, (e.g., a heavy or light chain of an antibody or a single chain antibody), may require construction of an expression vector containing a polynucleotide that encodes the antibody. Once a polynucleotide encoding an antibody molecule or a heavy or light chain of an antibody, or portion thereof (preferably containing the heavy or light chain variable domain), has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. Disclosed herein are replicable vectors comprising a nucleotide sequence encoding an antibody molecule , or a heavy or light chain thereof, or a heavy or light chain variable domain, operably linked to a promoter. Such vectors may include the nucleotide sequence encoding the constant region of the antibody molecule (see, e.g., PCT Publication WO 86/05807; PCT Publication WO 89/01036; and U.S. Pat. No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody Thus, disclosed herein are host cells containing a polynucleotide encoding an antibody , or a heavy or light chain thereof, or a single chain antibody , operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules . Such host-expression systems represent vehicles by which the coding sequences of interest may be produced and subsequently purified, but also represent cells which may, when transformed or transfected with the appropriate nucleotide coding sequences, express an antibody molecule in situ. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BIK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as Escherichia coli, and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett et al., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2:1791 (1983)), in which the antibody coding sequence may be ligated individually into the vector in frame with the lac Z coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa califomica nuclear polyhedrosis virus (AcNPV) is used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non- essential region of the viral genome (e.g., region E1 or E3) will result in a recombinant virus that is viable and capable of expressing the antibody molecule in infected hosts. (e.g., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., Methods in Enzymol. 153:51-544 (1987)).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, and in particular, breast cancer cell lines such as, for example, BT483, Hs578T, HTB2, BT20 and T47D, and normal mammary gland cell line such as, for example, CRL7030 and Hs578Bst.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compounds that interact directly or indirectly with the antibody molecule.

As described above, a preferred method for producing monoclonal antibodies is disclosed in US 2002 0123057 A1.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 (1980)) genes can be employed in tk-, hgprt- or aprt- cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, 1993, TIB TECH 11(5):155-215); and hygro, which confers resistance to hygromycin (Santerre et al., Gene 30:147 (1984)). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., J. Mol. Biol. 150:1 (1981).

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., Mol. Cell. Biol. 3:257 (1983)).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

Antibodies can be developed against the entire C35 polypeptide or portions thereof, or sites that are prenylated, glycosylated, phosphorylated, myristoylated, or amidated.

Fragments which function as epitopes may be produced by any conventional means. (See, e.g., Houghten, Proc. Natl. Acad. Sci. USA 82:5131-5135 (1985), further described in U.S. Patent No. 4,631,211).

Similarly, immunogenic epitopes can be used to induce antibodies according to methods well known in the art. (See, for instance, Sutcliffe et al., supra; Wilson *et al*., *supra*; Chow et al., Proc. Natl. Acad. Sci. USA 82:910-914; and Bittle et al., J. Gen. Virol. 66:2347-2354 (1985). Preferred immunogenic epitopes include the immunogenic epitopes disclosed herein, as well as any combination of two, three, four, five or more of these immunogenic epitopes. The polypeptides comprising one or more immunogenic epitopes may be presented for eliciting an antibody response together with a carrier protein, such as an albumin, to an animal system (such as rabbit or mouse), or, if the polypeptide is of sufficient length (at least about 25 amino acids), the polypeptide may be presented without a carrier. However, immunogenic epitopes comprising as few as 8 to 10 amino acids have been shown to be sufficient to raise antibodies capable of binding to, at the very least, linear epitopes in a denatured polypeptide (e.g., in Western blotting). In a specific embodiment, epitopes include those set forth in Table 11, infra.

Epitope-bearing polypeptides may be used to induce antibodies according to methods well known in the art including, but not limited to, in vivo immunization, in vitro immunization, and phage display methods. *See*, *e.g*., Sutcliffe *et al*., *supra*; Wilson *et al*., *supra*, and Bittle et al., J. Gen. Virol., 66:2347-2354 (1985). If in vivo immunization is used, animals may be immunized with free peptide; however, anti-peptide antibody titer may be boosted by coupling the peptide to a macromolecular carrier, such as keyhole limpet hemacyanin (KLH) or tetanus toxoid. For instance, peptides containing cysteine residues may be coupled to a carrier using a linker such as maleimidobenzoyl- N-hydroxysuccinimide ester (MBS), while other peptides may be coupled to carriers using a more general linking agent such as glutaraldehyde.

Epitope bearing peptide may also be synthesized as multiple antigen peptides (MAPs), first described by J. P. Tam in Proc. Natl. Acad. Sci. U.S.A. 85:5409.
MAPs consist of multiple copies of a specific peptide attached to a non-immunogenic lysine core. Map peptides usually contain four or eight copies of the peptide often referred to as MAP-4 or MAP-8 peptides. By way of non-limiting example, MAPs may be synthesized onto a lysine core matrix attached to a polyethylene glycol-polystyrene (PEG-PS) support. The peptide of interest is synthesized onto the lysine residues using 9-fluorenylmethoxycarbonyl (Fmoc) chemistry. For example, Applied Biosystems (Foster City, CA) offers MAP resins, such as, for example, the Fmoc Resin 4 Branch and the Fmoc Resin 8 Branch which can be used to synthesize MAPs. Cleavage of MAPs from the resin is performed with standard trifloroacetic acid (TFA)-based cocktails known in the art. Purification of MAPs, except for desalting, is not necessary. MAP peptides may be used as an immunizing vaccine which elicits antibodies that recognize both the MAP and the native protein from which the peptide was derived.

Epitope bearing peptides may also be incorporated into a coat protein of a virus which can then be used as an immunogen or a vaccine with which to immunize animals, including humans, in order encourage the production of anti-epitope antibodies. For example, the V3 loop of the gp120 glycoprotein of the human immunodeficiency virus type 1 (HIV-1) has been engineered to be expressed on the surface of rhinovirus. Immunization with this rhinovirus displacing the V3 loop peptide yielded apparently effective mimics of the HIV-1 immunogens (as measured by their ability to be neutralized by anti-HIV-1 antibodies as well as their ability to elicit the production of antibodies capable of neutralizing HIV-1 in cell culture). This techniques of using engineered viral particles as an immunogen is described in more detail in Smith et al., Behring Inst Mitt Feb;(98):229-39 (1997), Smith et al, J Virol 72:651-9 (1998), and Zhang et al., Biol Chem 380:365-74 (1999).

Animals such as rabbits, rats and mice are immunized with either free or carrier- coupled peptides or MAP peptides, for instance, by intraperitoneal and/or intradermal injection of emulsions containing about 100 µg of peptide or carrier protein and Freund's adjuvant or any other adjuvant known for stimulating an immune response. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

Once an antibody molecule has been produced by an animal, chemically synthesized, or recombinantly expressed, it may be purified by any method known in the art for purification of an immunoglobulin molecule, for example, by chromatography (e.g., ion exchange, affinity, particularly by affinity for the specific antigen after Protein A, and sizing column chromatography), centrifugation, differential solubility, or by any other standard technique for the purification of proteins. In addition, the antibodies - or fragments thereof can be fused to heterologous polypeptide sequences described herein or otherwise known in the art, to facilitate purification.

The antibodies may be generated by any suitable method known in the art. Polyclonal antibodies to an antigen-of-interest can be produced by various procedures well known in the art. For example, a polypeptide can be administered to various host animals including, but not limited to, rabbits, mice, rats, etc. to induce the production of sera containing polyclonal antibodies specific for the antigen. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art and are discussed in detail in the Examples. In a non-limiting example, mice can be immunized with a polypeptide or a cell expressing such peptide. Once an immune response is detected, e.g., antibodies specific for the antigen are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide . Ascites fluid, which generally contains high levels of antibodies, can be generated by immunizing mice with positive hybridoma clones.

Accordingly, disclosed herein are methods of generating monoclonal antibodies as well as antibodies produced by the method comprising culturing a hybridoma cell secreting an antibody wherein, preferably, the hybridoma is generated by fusing splenocytes isolated from a mouse immunized with an antigen with myeloma cells and then screening the hybridomas resulting from the fusion for hybridoma clones that secrete an antibody able to bind a polypeptide.

Antibody fragments which recognize specific epitopes may be generated by known techniques. For example, Fab and F(ab')2 fragments may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain.

For example, the antibodies can also be generated using various phage display methods known in the art. In phage display methods, functional antibody domains are displayed on the surface of phage particles which carry the polynucleotide sequences encoding them. In a particular embodiment, such phage can be utilized to display antigen binding domains expressed from a repertoire or combinatorial antibody library (e.g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994); PCT application No. PCT/GB91/01134; PCT publications WO 90/02809; WO 91/10737; WO 92/01047; WO 92/18619; WO 93/11236; WO 95/15982; WO 95/20401; and U.S. Pat. Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including Mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in PCT publication WO 92/22324; Mullinax et al., BioTechniques 12(6):864-869 (1992); and Sawai et al., AJRI 34:26-34 (1995); and Better et al., Science 240:1041-1043 (1988).

Examples of techniques which can be used to produce single-chain Fvs and antibodies, as well as diabodies, triabodies, and tetrabodies, include those described in U.S. Pat. Nos. 4,946,778 and 5,258,498; Huston et al., Methods in Enzymology 203:46-88 (1991); Shu et al., PNAS 90:7995-7999 (1993); Skerra et al., Science 240:1038-1040 (1988); U.S. Publication No. 20020018749 and U.S. Pat. No. 5,837,242. For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. *See e.g*., Morrison, Science 229:1202 (1985); Oi et al., BioTechniques 4:214 (1986); Gillies et al., J. Immunol. Methods 125:191-202 (1989); U.S. Pat. Nos. 5,807,715; 4,816,567; and 4,816397.
Humanized antibodies are antibody molecules from non-human species antibody that binds the desired antigen having one or more complementarity determining regions (CDRs) from the non-human species and a framework regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. (*See, e.g*., Queen et al., U.S. Pat. No. 5,585,089; Riechmann et al., Nature 332:323 (1988), which are incorporated herein by reference in their entireties.) Antibodies can be humanized using a variety of techniques known in the art including, for example, CDR-grafting (EP 239,400; PCT publication WO 91/09967; U.S. Pat. Nos. 5,225,539; 5,530,101; and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska. et al., PNAS 91:969-973 (1994)), and chain shuffling (U.S. Pat. No. 5,565,332).

Completely human antibodies are particularly desirable for therapeutic treatment of human patients. Human antibodies can be made by a variety of methods known in the art including phage display methods described above using antibody libraries derived from human immunoglobulin sequences, and the methods disclosed in US 2002 0123057 A1. See also, U.S. Pat. Nos. 4,444,887 and 4,716,111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, e.g., all or a portion of a polypeptide. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar, Int. Rev. Immunol. 13:65-93 (1995). For a detailed discussion of this technology for producing human antibodies and human monoclonal antibodies and protocols for producing such antibodies, see, e.g., PCT publications WO 98/24893; WO 92/01047; WO 96/34096; WO 96/33735; European Patent No. 0 598 877; U.S. Pat. Nos. 5,413,923; 5,625,126; 5,633,425; 5,569,825; 5,661,016; 5,545,806; 5,814,318; 5,885,793; 5,916,771; and 5,939,598.
In addition, companies such as Abgenix, Inc. (Freemont, Calif.) and Genpharm (San Jose, Calif.) can be engaged to provide human antibodies directed against a selected antigen using technology similar to that described above.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Bio/technology 12:899-903 (1988)).

Further, antibodies to the polypeptides can, in turn, be utilized to generate anti-idiotype antibodies that "mimic" polypeptides using techniques well known to those skilled in the art. (*See, e.g*., Greenspan & Bona, FASEB J. 7(5):437-444; (1989) and Nissinoff, J. Immunol. 147(8):2429-2438 (1991)). For example, antibodies which bind to and competitively inhibit polypeptide multimerization and/or binding of a polypeptide to a ligand can be used to generate anti-idiotypes that "mimic" the polypeptide multimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide and/or to bind its ligands/receptors, and thereby block its biological activity.

### Antibody Conjugates

Disclosed herein is a method for the specific destruction of cells (e.g., the destruction of aberrant cells, including, but not limited to, tumor cells) by administering antibodies against cancer-associated, intracellular antigens in association with toxins or cytotoxic prodrugs.

By "toxin" is meant compounds that bind and activate endogenous cytotoxic effector systems, radioisotopes, holotoxins, modified toxins, catalytic subunits of toxins, cytotoxins (cytotoxic agents), or any molecules or enzymes not normally present in or on the surface of a cell that under defined conditions cause the cell's death. Toxins that may be used according to the methods include, but are not limited to, radioisotopes known in the art, compounds such as, for example, antibodies (or complement fixing containing portions thereof) that bind an inherent or induced endogenous cytotoxic effector system, thymidine kinase, endonuclease, RNAse, alpha toxin, ricin, abrin, Pseudomonas exotoxin A, diphtheria toxin, saporin, momordin, gelonin, pokeweed antiviral protein, alpha-sarcin and cholera toxin. "Toxin" also includes a cytostatic or cytocidal agent, a therapeutic agent or a radioactive metal ion, e.g., alpha-emitters such as, for example, ²¹³Bi, or other radioisotopes such as, for example, ¹⁰³Pd, ¹³³Xe, ¹³¹I, ⁶⁸Ge, ⁵⁷Co, ⁶⁵Zn, ⁸⁵Sr, ³²P, ³⁵S, ⁹⁰Y, ¹⁵³Sm, ¹⁵³Gd, ¹⁶⁹Yb, ⁵¹Cr, ⁵⁴Mn, ⁷⁵Se, ¹¹³Sn, ⁹⁰Yttrium, ¹¹⁷Tin, ¹⁸⁶Rhenium, ¹⁶⁶Holminium, and ¹⁸⁸Rhenium and those listed in the "Complex" section below; luminescent labels, such as luminol; and fluorescent labels, such as fluorescein and rhodamine, and biotin. In a specific embodiment, antibodies (including fragments or variants thereof) are conjugated with a radioisotope, e.g., ¹³¹I. In a preferred embodiment, the radioisotope-conjugated antibody is adminstered for radioimmunotherapy in combination with chemotherapy.

A cytotoxin or cytotoxic agent includes any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Cytotoxin or cytotoxic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis- dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

By "cytotoxic prodrug" is meant a non-toxic compound that is converted by an enzyme, normally present in the cell, into a cytotoxic compound. Cytotoxic prodrugs that may be used according to the methods of the invention include, but are not limited to, glutamyl derivatives of benzoic acid mustard alkylating agent, phosphate derivatives of etoposide or mitomycin C, cytosine arabinoside, daunorubisin, and phenoxyacetamide derivatives of doxorubicin.

Disclosed herein are antibodies recombinantly fused or chemically conjugated (including both covalently and non-covalently conjugations) to a polypeptide (or portion thereof, preferably at least 10, 20, 30, 40, 50, 60, 70, 80, 90 or 100 amino acids of the polypeptide) to generate fusion proteins. The fusion does not necessarily need to be direct, but may occur through linker sequences. The antibodies may be specific for antigens other than C35 polypeptides (or portion thereof, preferably at least 10, 20, 30, 40, 50, 60, 70, 80, 90 or 100 amino acids of the polypeptide). In particular, antibodies may be specific for other cancer-associated, intracellular antigens. In a preferred embodiment, such intracellular antigens are prenylated proteins.

Antibodies (including fragments or variants thereof) may be fused to either the N- or C-terminal end of the heterologous protein (e.g., immunoglobulin Fc polypeptide or human serum albumin polypeptide). Antibodies may also be fused to albumin (including but not limited to recombinant human serum albumin (see, e.g., U.S. Patent No. 5,876,969, issued March 2, 1999, EP Patent 0 413 622, and U.S. Patent No. 5,766,883, issued June 16, 1998, ), resulting in chimeric polypeptides. In a preferred embodiment, antibodies (including fragments or variants thereof) are fused with the mature form of human serum albumin (i.e., amino acids 1 - 585 of human serum albumin as shown in Figures 1 and 2 of EP Patent 0 322 094) . In another preferred embodiment, antibodies (including fragments or variants thereof) are fused with polypeptide fragments comprising, or alternatively consisting of, amino acid residues 1-z of human serum albumin, where z is an integer from 369 to 419, as described in U.S. Patent 5,766,883.

Polynucleotides encoding fusion proteins are also disclosed herein. Such fusion proteins may, for example, facilitate purification and may increase half-life in vivo. Antibodies fused or conjugated to polypeptides may also be used in in vitro immunoassays and purification methods using methods known in the art. *See e.g*., Harbor *et al*., *supra*, and PCT publication WO 93/21232; EP 439,095; Naramura et al., Immunol. Lett. 39:91-99 (1994); U.S. Patent 5,474,981; Gillies et al., PNAS 89:1428-1432 (1992); Fell et al., J. Immunol. 146:2446-2452 (1991).

Moreover, the antibodies or fragments thereof can be fused to marker sequences, such as a peptide to facilitate purification. In preferred embodiments, the marker amino acid sequence is a hexa-histidine peptide, such as the tag provided in a pQE vector (QIAGEN, Inc., 9259 Eton Avenue, Chatsworth, Calif., 91311), among others, many of which are commercially available. As described in Gentz et al., Proc. Natl. Acad. Sci. USA 86:821-824 (1989), for instance, hexa-histidine provides for convenient purification of the fusion protein. Other peptide tags useful for purification include, but are not limited to, the "HA" tag, which corresponds to an epitope derived from the influenza hemagglutinin protein (Wilson et al., Cell 37:767 (1984)) and the "flag" tag.

Disclosed herein are antibodies, such as C35 antibodies or fragments thereof, conjugated to a diagnostic or therapeutic agent. The antibodies can be used diagnostically to, for example, monitor the development or progression of a tumor as part of a clinical testing procedure to, e.g., determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive materials, positron emitting metals using various positron emission tomographies, and nonradioactive paramagnetic metal ions. The detectable substance may be coupled or conjugated either directly to the antibody (or fragment thereof) or indirectly, through an intermediate (such as, for example, a linker known in the art) using techniques known in the art. See, for example, U.S. Pat. No. 4,741,900 for metal ions which can be conjugated to antibodies for use as diagnostics . Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include 125I, 131I, 111In or 99Tc.

The therapeutic agent is not to be construed as limited to classical chemical therapeutic agents. For example, the therapeutic agent may be a protein or polypeptide possessing a desired biological activity. Such proteins may include, for example, a toxin such as abrin, ricin A, pseudomonas exotoxin, or diphtheria toxin; a protein such as tumor necrosis factor, alpha-interferon, beta-interferon, nerve growth factor, platelet derived growth factor, tissue plasminogen activator, an apoptotic agent, e.g., TNF-alpha, TNF-beta, AIM I (See, International Publication No. WO 97/33899), AIM II (See, International Publication No. WO 97134911), Fas Ligand (Takahashi et al., Int. Immunol., 6:1567-1574 (1994)), VEGI (See, International Publication No. WO 99/23105), a thrombotic agent or an anti- angiogenic agent, e.g., angiostatin or endostatin; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

Techniques for conjugating such toxins and therapeutic agents to antibodies are well known, see, e.g., Amon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev. 62:119-58 (1982).

Alternatively, an antibody can be conjugated to a second antibody to form an antibody heteroconjugate as described by Segal in U.S. Pat. No. 4,676,980,

An antibody, with or without a toxin or therapeutic agent conjugated to it, administered alone or in combination with cytotoxic factor(s) and/or cytokine(s) can be used as a therapeutic.

Techniques known in the art may be applied to label antibodies of the invention. Such techniques include, but are not limited to, the use of bifunctional conjugating agents (see e.g., U.S. Pat. Nos. 5,756,065; 5,714,631; 5,696,239; 5,652,361; 5,505,93.1; 5,489,425; 5,435,990; 5,428,139; 5,342,604; 5,274,119; 4,994,560; and 5,808,003.

Chelator molecules are known in the art. For example, see Subramanian, R. and Meares, C.F., "Bifunctional Chelating Agents for Radiometal-labeled monoclonal Antibodies," in Cancer Imaging with Radiolabeled Antibodies (D. M. Goldenberg, Ed.) Kluwer Academic Publications, Boston; Saji, H., "Targeted delivery of radiolabeled imaging and therapeutic agents: bifunctional radiopharmaceuticals." Crit. Rev. Ther. Drug Carrier Syst. 16:209-244 (1999); Srivastava S.C. and Mease R.C., "Progress in research on ligands, nuclides and techniques for labeling monoclonal antibodies." Int. J. Rad. Appl. Instrum. B 18:589-603 (1991); and Liu, S. and Edwards, D.S., "Bifunctional chelators for therapeutic lanthanide radiopharmaceuticals." Bioconjug. Chem. 12:7-34 (2001). Any chelator which can be covalently bound to said antibody may be used
The chelator may further comprise a linker moiety that connects the chelating moiety to the antibody.

Bifunctional chelators based on macrocyclic ligands in which conjugation is via an activated arm attached to the carbon backbone of the ligand can be employed as described by M. Moi et al., J. Amer. Chem. Soc. 49:2639 (1989) (2-p-nitrobenzyl-1,4,7,10-tetraazacyclododecane-N,N',N",N"'-tetraacetic acid); S. V. Deshpande et al., J. Nucl. Med. 31:473 (1990); G. Ruser et al., Bioconj. Chem. 1:345 (1990); C. J. Broan et al., J. C. S. Chem. Comm. 23:1739 (1990); and C. J. Anderson, et al., J. Nucl. Med. 36:850 (1995).

In one embodiment, the chelator is a macrocyclic chelator, such as polyazamacrocyclic chelators, optionally containing one or more carboxy, phosphonate, or phosphate groups. In another embodiment, the chelator is a chelator selected from the group consisting of DOTA, analogues of DOTA, and derivatives of DOTA.

In one embodiment, suitable chelator molecules include DOXA (1-oxa-4,7,10-triazacyclododecanetriacetic acid), NOTA (1,4,7-triazacyclononanetriacetic acid), TETA (1,4,8,11-tetraazacyclotetradecanetetraacetic acid), and THT (4'-(3-amino-4-methoxyphenyl)-6,6"-bis(N',N'-dicarboxymethyl-N-methylhydra zino)-2,2':6',2"-terpyridine), and analogs and derivatives thereof. *See, e.g*., Ohmono et al., J. Med. Chem. 35: 157-162 (1992); Kung et al., J. Nucl. Med. 25: 326-332 (1984); Jurisson et al., Chem. Rev. 93:1137-1156 (1993); and U.S. Patent No. 5,367,080. Other suitable chelators include chelating agents disclosed in U.S. Patent Nos. 4,647,447; 4,687,659; 4,885,363; EP-A-71564; WO89/00557; and EP-A-232751.

In another embodiment, suitable macrocyclic carboxylic acid chelators which can be used include 1,4,7,10-tetranacyclododecane-N,N',N",N"'-tetraacetic acid (DOTA); 1,4,8,12-tetraazacyclopentadecane- N,N',N",N"'-tetraacetic acid (15N4); 1,4,7-triazacyclononane-N,N',N"-triacetic acid (9N3); 1,5,9-triazacyclododecane-N,N,N'-triacetic acid (12N3); and 6-bromoacetamido-benzyl-1,4,8,11-tetraazacyclotetadecane- N,N'N",N'"-tetraacetic acid (BAT).

The antibody conjugate may contain more than an average of one chelator molecule. In one embodiment, the antibody conjugate contains about 1, 2, 3, 4, 5, 6,7,8,9,10,15,20,25,30, or 35 chelator molecules. In another embodiment, the antibody conjugate contains about 1, 2, 3, 4, 5, 6, 7, 8, 9, or 10 chelator molecules. In another embodiment, the antibody conjugate contains about 1,2,3,4, or 5 chelator molecules. In another embodiment, the antibody conjugate contains an average of about 1 chelator molecule.

The antibody conjugate, as described above, comprises an antibody and a chelator molecule covalently bonded together. The covalent bond between the protein and chelator is formed between an atom of the protein and an atom of the chelator. The atom of the protein that forms the covalent bond can be any suitable atom of the protein, such as a carbon, nitrogen, oxygen, and sulfur. Certain functional groups on the protein are preferred to form the covalent bond with the chelator. Such groups include an amino group at the terminus of the protein; a carboxy group at the terminus of the protein; an amino group on a lysine residue; a carboxy group on an aspartame or glutamate residue; a guanidino group on an arginine residue; a thiol group on a cysteine residue; a hydroxy group on a serine residue or threonine residue; a imidazole group of a histidine residue; a hydroxy group of a tyrosine; a hydroxy group of a tryptophan group; and an amide group of an asparagine or glutamine residue.

### Methods Of Preparing Antibody Conjugates

Disclosed herein is a method of preparing an antibody conjugate. The general procedure for preparing an antibody conjugate comprises reacting an antibody with a chelator. A suitable chelator will be able to react with the antibody to form a covalent bond between the chelator and the antibody.

A chelator that can form part of the conjugate is known in the art, and methods of preparing such a chelator are known. Any chelator which is able to form a covalent bond with the antibody may be used to form the antibody conjugate. Such chelators are described below.

In one embodiment, a chelator to be used in the method is an activated chelator. The activated chelator contains a functional group that will readily react with a functional group on the protein, thereby forming a covalent bond between the protein and the chelator. Such functional groups are well known in the art.

A suitable reactive functional group is a group that will react directly with carboxy, aldehyde, amine, alcohol, or sulfhydryl group on the antibody. Such groups include, for example, active halogen containing groups including, for example, chloromethylphenyl groups and chloroacetyl (C1CH2C(=O)-) groups; activated 2-(leaving group substituted)-ethylsulfonyl and ethylcarbonyl groups such as 2-chloroethylsulfonyl and 2-chloroethylcarbonyl; vinylsulfonyl; vinylcarbonyl; epoxy; isocyanato; isothiocyanato; aldehyde; aziridine; succinimidoxycarbonyl; activated acyl groups such as carboxylic acid halides; and mixed anhydrides.

A chelator which can be used in the present method includes any specific chelator as described herein for the antibody conjugate. Moreover, in another embodiment, the chelator used in the method is a chelator which can be used to prepare any specific antibody conjugate as described above. In one embodiment, the chelator is an activated chelator selected from the group consisting of DOTA, analogues of DOTA, and derivatives of DOTA, wherein each of said DOTA, analogues of DOTA, and derivatives of DOTA contains a suitable activating group which enables the chelator molecule to be covalently bonded to the antibody.

In another embodiment, the activated chelator for use in preparing a conjugate is a-(5-isothiocyanato- 2-methoxyphenyl)-1,4,7,10-tetraazacyclododecane-1,4,7,10-tetraacetic acid, which is also known as MeO-DOTA-NCS. A salt or ester of a-(5-isothiocyanato- 2-methoxyphenyl)- 1,4,7,10-tetraazacyclododecane-1,4,7,10-tetraacetic acid may also be used.

In one embodiment, the method of preparing an antibody conjugate comprises mixing, agitating, or preparing a solution, said solution comprising an antibody and a chelator. In an additional embodiment, the method of preparing an antibody conjugate comprises mixing, agitating, or preparing a solution comprising an antibody, a chelator, citrate buffer, HEPES buffer, and sterile water. In an additional embodiment, the solution further comprises NaOH. In an additional embodiment, the solution has a pH of about 8.5.

In an additional embodiment, the method of preparing the conjugate comprises a) mixing, agitating, or preparing a solution comprising an antibody and a chelator, at a temperature of about 0 °C to about 50 °C for about 0.5 hours to about 24 hours, wherein said solution has a pH of about 8.0 to about 9.0; and b) optionally adding a quenching agent.

In one embodiment of the above process, the solution is mixed, agitated or prepared at a temperature of about 0 °C to about 50 °C. In another embodiment of the above process, the solution is mixed, agitated or prepared at a temperature of about 20 °C to about 30 °C. The temperature at which the solution is mixed, agitated, or prepared can be about 0, 5, 10, 15, 20, 25, 20, 35, 40, 45, or 50 °C.

Any suitable chelator as described above may be used in the present process.

The molar ratio of antibody to chelator used in the preparation of the conjugate can vary. The molar ratio of antibody to chelator can be from about 1000:1 to about 1:1000, from about 1 to about 100, from about 5 to about 20, from about 10 to about 15, about 12. The molar ratio of antibody to chelator refers to the ratio of the number of antibody molecules to the number of chelator molecules in the reaction solution.

When preparing an antibody conjugate, the solution can be mixed, agitated, or allowed to stand for a variable number of hours, depending upon a number of variables, such factors including the identity of the chelator, the identity of the antibody, the temperature of the reaction, the pH, the identity of the buffer, the molar ratio of the reactants, the purity of the available reagents, the presence of a catalyst or activator, and other factors which would be evident to one of skill in the art. In one embodiment of the above process, the solution comprising an antibody and a chelator is mixed, agitated, or allowed to stand for about 0.5 hours to about 24 hours. In another embodiment, the solution is mixed, agitated, or allowed to stand for about 1 hour to about 20 hours. In another embodiment, the solution is mixed, agitated, or allowed to stand for about 2 hour to about 10 hours. In another embodiment, the solution is mixed, agitated, or allowed to stand for about 3 hour to about 5 hours. In another embodiment, the solution is mixed, agitated, or allowed to stand for about 4 hours. In other embodiments, the solution is mixed, agitated, or allowed to stand for about 1, 2, 3, 4, 5, 6, 7, 8, 9, 10, 11, 12, 13, 14 ,15, 16, 17, 18, 19, 20, 21, 22, 23, or 24 hours.

The pH of the solution used to prepare the antibody conjugate can vary. In one embodiment, the pH will be about 6, 7, 8, 9, or 10. In another embodiment, said solution has a pH of about 7 to about 10. In another embodiment, said solution has a pH of about 7.5 to about 9.5. In another embodiment, said solution has a pH of about 8 to about 9. In another embodiment, said solution has a pH of about 8.5.

The solution used to prepare the antibody conjugate may further comprise a buffer. Buffers are well-known in the art. Suitable buffers for use in the preparation of an antibody are described below for the method of preparing an antibody conjugate. In one embodiment, the buffer is a citrate buffer or an acetate buffer. In another embodiment, the buffer includes an acetate buffer having a concentration of about 1 to about 50 mM and having a NaCl concentration of about 1 to about 500 mM. In another embodiment, the buffer includes an acetate buffer having a concentration of about about 10 mM and having a NaCl concentration of about about 140 mM. Suitable acetate buffers include acetate buffers havaing a concentration of about 1, 5, 10, 15, 20, 25, 20, 35, 40, 45, or 55 mM. Suitable buffers and solutions include those having a NaCl concentration of about 1, 50, 75, 100, 125, 140, 150, 175, 200, 225, 250, 275, 300, 350, 400, 450, or 500 mM. An additional suitable buffer is a HEPES buffer, in particular a HEPES buffer having a concentration of about 100, 200, 300, 400, 500, 600, 700, 800, 900, or 1000 mM. In an additional embodiment, the solution comprises a PIEPES buffer having a concentration of about 500 mM.

The concentration of the antibody used in the method may vary. In one embodiment, the concentration of antibody in the solution is from about 0.1 mg/mL to about 10 mg/mL. In another embodiment, the antibody concentration is about 0.5 mg/mL to about 5 mg/mL. In other specific embodiments, the antibody concentration is about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, or 2.5 mg/mL. In another specific embodiment, the concentration of antibody in the solution is about 1 mg/mL to about 2 mg/mL.

A reagent which facilitates the formation of the conjugate can also be used. Such reagents typically activate a chelator to react more readily with a protein. Alternatively, the reagent may activate the protein to react more readily with the chelator. Examples of such reagents are known in the art and include dicyclohexylcarbodiimide (DCC), diethylazodicarboxylate (DEAD), and diisopropylazodicarboxylate (DIAD).

According to the method , a quencing agent may optionally be added to the reaction solution after the antibody conjugate is prepared to a sufficient degree. Suitable quencing agents are known in the art. In one embodiment, the quenching agent is glycine. In a specific embodiment, a solution comprising a glycine buffer is used as a quenching example. By way of example, after the reaction solution comprising the antibody and the chelator has been mixed, agitated, or allowed to stand for a sufficient amount of time, e.g., 1-10 hours, 3-5 hours, or 1-3 hours, a solution comprising a glycine buffer is added to the reaction solution to stop the reaction. The reaction solution is then allowed to stand for an additional amount of time, e.g., about 0.25, 0.5, 0.75, 1, or 1.5 hours.

The method of preparing an antibody conjugate may comprise mixing, agitating, or preparing a solution comprising:
an antibody has a concentration of about 1 to about 2 mg/mL;
a MeO-DOTA-NCS in an amount such that the ratio of antibody to MeO-DOTA-NCS is about 1 to 12;
a citrate buffer having a concentration of about 0.5 to about 20 mM;
NaCl haing a concentration of about 1 to about 200 mM;
HEPES buffer having a concentration of about 10 to about 500 mM; and
sterile water.

The method may comprise:
preparing a first solution comprising an antibody and a citrate buffer;
adding a second solution comprising a HEPES buffer to said first solution;
adding a third solution comprising a chelator and NaOH to said first solution;
optionally adjusting the pH of said first solution to about 8.5;
mixing, agitating, or allowing to stand said first solution at about 25 °C for about 3 to about 5 hours; and
optionally adding quencing agent to said first solution.

The method of preparing an antibody conjugate further optionally comprises a process of purifying said conjugate. A number of known methods may be used to purifying said protein conjugate.

In one embodiment, the purifying step uses normal flow filtration or tangential flow filtration. In another embodiment, the process for purifying the conjugate is a diafiltration method.

### Antibody Complexes

Disclosed herein is an antibody complex comprising an antibody conjugate and a metal ion, wherein said metal ion is noncovalently associated with the chelator moiety of said antibody conjugate. Specific embodiments of an antibody complex include a complex comprising an antibody conjugate, as set forth herein, and a metal ion

Any metal ion that noncovalently associates with the chelating moiety of said antibody conjugate may be used. Such a metal ion includes a metal ion selected from the group consisting of Ac, Ag, At, Au, Bi, Ce, Co, Cr, Cu, Dy, Er, Eu, Fe, Ga, Gd, Hg, Ho, In, La, Lu, Mn, Mo, Nd, Ni, Os, Pb, Pd, Pm, Pr, Pt, Rb, Re, Rh, Ru, Sb, Sc, Si, Sm, Sn, Sr, Tb, Tc, Tl, Tm, V, W, Y, and Yb.

In one embodiment, the metal ion of the complex is a radionuclide. Radionuclides useful in the antibody complex are known in the art. The radionuclides useful in the present invention include gamma-emitters, positron-emitters, x-ray emitters, fluorescence-emitters, beta-emitters, alpha-emitters, and electron and neutron-capturing agents. In one embodiment, gamma-emitters, positron-emitters, x-ray emitters, and fluorescence-emitters are suitable for localization and/or therapy, while beta and alpha-emitters and electron and neutron-capturing agents, such as boron and uranium, also can be used for therapy.

The radionuclide used in the complex may be suitable for therapeutic, diagnostic, or both therapeutic and diagnostic purposes. Examples of appropriate metals include Ag, At, Au, Bi, Cu, Ga, Ho, In, Lu, Pb, Pd, Pm, Pr, Rb, Re, Rh, Sc, Sr, Tc, Tl, Y, and Yb. Examples of the radionuclide used for diagnostic purposes are Fe, Gd, 111In, 67Ga, or 68Ga. In another embodiment, the radionuclide used for diagnostic purposes is 111In, or 67Ga. Examples of the radionuclide used for therapeutic purposes are 166Ho, 165Dy, 90Y, 115mIn, 52Fe, or 72Ga. In one embodiment, the radionuclide used for diagnostic purposes is 166Ho or 90Y. Examples of the radionuclides used for both therapeutic and diagnostic purposes include 153Sm, 177Lu, 159Gd, 175Yb, or 47Sc. In one embodiment, the radionuclide is 153Sm, 177Lu, 175Yb, or 159Gd.

Preferred metal radionuclides include 90Y, 99mTc, 111In, 47Sc, 67Ga, 51Cr, 177mSn, 67Cu, 167Tm, 97Ru, 188Re, 177Lu, 199Au, 47Sc, 67Ga, 51Cr, 177mSn, 67Cu, 167Tm, 95Ru, 188Re, 177Lu, 199Au, 203Pb and 141Ce.

In a particular embodiment, the metal ion of the antibody complex is selected from the group consisting of 90Y, 111 In, 177Lu, 166Ho, 215Bi, and 225Ac.

Moreover, gamma-emitting radionuclides, such as 99mTc, 111In, 67Ga, and 169Yb have been approved or under investigation for diagnostic imaging, while complexes of beta-emitters, such as 67Cu, 111Ag, 186Re, and 90Y are useful for the applications in tumor therapy. Also other useful radionuclides include gamma-emitters, such as 99mTc, 111In, 67Ga, and 169Yb, and beta-emitters, such as 67Cu, 111Ag, 186Re, 188Re and 90Y, as well as other radionuclides of interest such as 211At, 212Bi, 177Lu, 86Rb , 105Rh, 153Sm, 198Au, 149Pm, 85Sr, 142Pr, 214Pb, 109Pd, 166Ho, 208T1, and 44Sc.

In another embodiment, paramagnetic metal ions include ions of transition and lanthanide metal, such as metals having atomic numbers of 21-29, 42, 43, 44, or 57-71, in particular ions of Cr, V, Mn, Fe, Co, Ni, Cu, La, Ce, Pr, Nd, Pm, Sm, Eu, Gd, Tb, Dy, Ho, Er, Tm, Yb, and Lu. The paramagnetic metals used in the composition for magnetic resonance imaging include the elements having atomic numbers of 22 to 29, 42, 44 and 58-70.

In another embodiment, fluorescent metal ions include lanthanides, in particular La, Ce, Pr, Nd, Pm, Sm, Eu, Gd, Tb, Dy, Ho, Er, Tm, Yb, and Lu.

In another embodiment, heavy metal-containing reporters may include atoms of Mo, Bi, Si, and W.

In a preferred embodiment, an antibody complex comprises an antibody conjugate and a metal ion, wherein the antibody of said conjugate is a human antibody.

In one embodiment, the chelator of the conjugate is a macrocyclic chelator, such as polyazamacrocyclic chelators, optionally containing one or more carboxy, phosphonate, or phosphate groups. In another embodiment, the chelator of the conjugate of the invention is a chelator selected from the group consisting of DOTA, analogues of DOTA, and derivatives of DOTA.

In one embodiment, suitable chelator molecules include DOXA (1-oxa-4,7,10-triazacyclododecanetriacetic acid), NOTA (1,4,7-triazacyclononanetriacetic acid), TETA (1,4,8,11-tetraazacyclotetradecanetetraacetic acid), and THT (4'-(3-amino-4-methoxyphenyl)-6,6"-bis(N',N'-dicarboxymethyl-N-methylhydra zino)-2,2':6',2"-terpyridine), and analogs and derivatives thereof. See, e.g., Ohmono et al., J. Med. Chem. 35: 157-162 (1992); Kung et al., J. Nucl. Med. 25: 326-332 (1984); Jurisson et al., Chem. Rev. 93:1137-1156 (1993); and U.S. Patent No. 5,367,080. Other suitable chelators include chelating agents disclosed in U.S. Patent Nos. 4,647,447; 4,687,659; 4,885,363; EP-A-71564; WO89/00557; and EP-A-232751.

In another embodiment, suitable macrocyclic carboxylic acid chelators which can be used in the present invention include 1,4,7,10-tetraazacyclododecane-N,N',N",N"'-tetraacetic acid (DOTA); 1,4,8,12-tetraazacyclopentadecane-N,N',N",N'"-tetraacetic acid (15N4); 1,4,7-triazacyclononane-N,N',N"-triacetic acid (9N3); 1,5,9-triazacyclododecane-N,N',N"-triacetic acid (12N3); and 6-bromoacetamido-benzyl-1,4,8,11-tetraazacyclotetadecane- N,N',N",N"'-tetraacetic acid (BAT).

### Methods Of Preparing Antibody Complexes

Disclosed herein is a method of preparing an antibody complex. In one embodiment, an antibody complex may be prepared by reacting an antibody conjugate, as described herein, with a metal ion, such as a radionuclide, which is able to associate noncovalently with the conjugate. In another embodiment, the metal ion associates noncovalently with the chelator moiety of said antibody conjugate. The reaction between the antibody complex and the metal ion may occur in solution, such as in a suitable buffered solution.

In one embodiment, a method for preparing an antibody complex comprises reacting an antibody conjugate with a radionuclide. Such a method comprises mixing, agitating, or preparing a solution comprising an antibody conjugate and a radionuclide. In one embodiment, said solution further comprises a buffer.

In another embodiment, a method for preparing an antibody complex comprises reacting an antibody conjugate with a chelator complexed with a metal ion. In this embodiment, a chelator is first complexed with a metal ion according to known procedures in the art. For example, see U.S. Patent No. 5,654,361. The chelator can be an activated chelator as described above. After the chelator is complexed with the metal ion and thereby forming a chelator-metal ion complex, the antibody is reacted with the chelator-metal ion complex, using a procedure as described above for preparing an antibody conjugate or using another procedure known in the art. According to the method, the solution comprising the antibody and the chelator-metal ion complex is mixed, agitated, or prepared, thereby forming said antibody compex.

When preparing an antibody complex, the solution can be mixed, agitated, or allowed to stand for a variable number of hours, depending upon a number of variables, such factors including the identity of the chelator, the identity of the antibody, the identity of the metal ion, the temperature of the reaction, the pH, the identity of the buffer, the molar ratio of the reactants, the purity of the available reagents, the presence of a catalyst, or activator, and other factors which would be evident to one of skill in the art. The solution comprising an antibody conjugate and a metal ion can be mixed, agitated, or allowed to stand for any amount of time that permits sufficient formation of the antibody complex. In one embodiment of the above process, the solution is mixed, agitated, or allowed to stand for about 0.5 minutes to about about 24 hours. In another embodiment, the solution is mixed, agitated, or allowed to stand for about 1 hour to about 20 hours. In another embodiment, the solution is mixed, agitated, or allowed to stand for about 2 hour to about 10 hours. In another embodiment, the solution is mixed, agitated, or allowed to stand for about 1 minute to about 1 hour. In another embodiment, the solution is mixed, agitated, or allowed to stand for about 1 minute to about 30 minutes. In another embodiment, the solution is mixed, agitated, or allowed to stand for about 1, 2, 3, 4, 5, 6, 7, 8, 9, 10, 15, 20, 25, 30, 35, 40, 45, 50, or 55 minutes.

The pH of the solution used to prepare the antibody complex can vary. In one embodiment, the pH will be about 1, 2, 3, 4, 5, 6, 7, 8, 9, or 10. In another embodiment, said solution has a pH of about 4 to about 8. In another embodiment, said solution has a pH of about 5 to about 7. In another embodiment, said solution has a pH of about 5.5 to about 7. In another embodiment, said solution has a pH of about 6.5.

The solution used to prepare the antibody complex may further comprise a buffer. Buffers are well-known in the art. Suitable buffers for use in the preparation of an antibody complex may include, but are not limited to, citrate, acetate, phosphate, carbonate, diphosphate, glycyl-glycine-piperazine-2HCl-NaOH; MES-NaOH-NaCl; TRIS-malic acid-NaOH; MES-NaOH; ADA-NaOH-NaCl; ACES-NaOH-NaCl; ACES-NaOH-NaCl; BES-NaOH-NaCl; MOPS-NaOH-NaCl; TES-NaOH-NaCl; MOPS-KOH; HEPES-NaOH-NaCl; TRIS-HCl; HEPPSO-NaOH; BICINE-NaOH-NaCl; TAPS-NaOH-NaCl; HEPPS (EPPS)-NaOH; TRICINE-NaOH; BICINE-NaOH; citric acid-disodiumhydrogenphosphate; boric acid-citric acid-potassium dihydrogen phosphate-Diethyl- barbituric acid-NaOH; citric acid-sodium citrate; sodium acetate-acetic acid; potassium hydrogenphthalate-NaOH; cacodylic acid sodium salt-HCl; potassium dihydrogen phosphate-disodium hydrogenphosphate; potassium dihydrogen-phosphate-NaOH; sodium dihydrogen phosphate- disodium hydrogen phosphate; imidazole-HCl; sodium tetraborate-boric acid; 2-amino-2-methyl-1,3 propanediol-HCl; diethanolamine-HCl; potassium chloride-boric acid-NaOH; boric acid-NaOH-KCl; glycine-NaOH; and sodium carbonate-sodium hydrogen carbonate.

The molar ratio of the antibody conjugate to the metal ion may vary. In one embodiment, the ratio of antibody conjugate to metal ion is from about 1:1000 to about 1000:1. In another embodiment, the ratio of antibody conjugate to metal ion is from about 1:100 to about 100:1, in another embodiment, from about 1:10 to about 10:1, in another embodiment, from about 1:5 to about 5:1, in another embodiment about 1:2, in another embodiment about 1:3, in another embodiment about 2:1, in another embodiment about 1:1. In other embodiments, the ratio of antibody conjugate to metal ion is about 1:2, 1:3, 1:4, 1:5, 1:6, 1:7, 1:8, 1:9, or 1:10.

Alternatively, the ratio of the antibody and the chelator-metal ion complex may vary. In one embodiment, the ratio of antibody to chelator-metal ion complex is from about 1:1000 to 1000:1.

Radionuclides are described and listed above and are well known and available commercially. Additionally, several, known methods can be used to prepare the radionuclides In one embodiment, the metal ion is in the form of a salt, for example a chloride salt. Such salts are known in the art. In another embodiment, the metal ion salt is yttrium chloride or yttrium acetate.

When preparing the complex according to one embodiment, other metal ions which could compete for complexation with the chelator are not present in significant amounts in the solution. For example, when preparing an antibody complex comprising 90Y, Fe3+ is not present in the solution in a significant amount or concentration. As used with reference to the one or more competing metal ions in the present process, the term significant amount refers to an amount or concentration which significantly intereferes, retards, delays, inhibits, or prevents preparation of the antibody complex.

The method of preparing an antibody complex may further comprise a step of removing excess metal ion from the reaction solution and/or the antibody complex. In one embodiment, such a step comprises adding a secondary chelating agent which is able to complex with excess metal ion in the solution. Such a secondary chelating agent may include one or more known chelating agents, for example, DTPA, EDTA, and MeO-DOTA-glycine. In one embodiment, MeO-DOTA-glycine is used as a secondary chelating agent.

The method of preparing an antibody complex may further comprise a step of removing excess metal ion from the reaction solution comprising the chelator-metal ion complex. In one embodiment, after the chelator-metal ion complex is formed and is in solution, a secondary chelating agent which is able to complex with excess metal ion is added. The solution is then eluted through a DEAE-cellulose anion exhange resin (Sigma Chemical Co., St. Louis, MO), which has been converted to acetate form to purify the neutral species chelator-metal ion complex from the charged species, i.e., secondary chelator-metal ion complex. The purified chelator-metal ion complex is then used to prepared the antibody complex as described herein.

The secondary chelating agent can be added to the reaction mixture as a solid or as a solution. In one embodiment, the seconary chelating agent is added in a buffered solution. In another embodiment , a buffer comprising an acetate buffer having a concentration of about 1 to about 50 mM, preferably about 10 mM, having a NaCl concentration of about 1 to about 500 mM, preferably about 140 mM, having a HSA concentration of about 1% to about 20%, preferably about 7% to about 8%, more preferably about 7.5%, having a pH of about 3-8, preferably about 6, and having a MeO-DOTA-glycine concentration of about 0.01 mM to about 100 mM, preferably about 1 mM, is added to the reaction solution after the antibody complex is formed to a satisfactory level of completion. In one embodiment, the secondary chelating agent is added about 5 minutes after the formation of the antibody complex. In other embodiments, the secondary chelating agent is added about 1, 2, 3, 4, 5, 6, 7 ,8, 9, 10, 15, 20, 25, 30, 5,40, 45, 50 55, or 60 minutes after formation of the antibody complex.

In another embodiment, the step of removing excess metal ion from the reaction solution and/or the antibody complex comprises subjecting the reaction solution and/or the antibody complex to centrifugation. Subjecting the reaction solution and/or the antibody complex to centrifugation can remove non-chelated metal ions.

In another embodiment, the step of removing excess metal ion from the reaction solution and/or the antibody complex comprises washing the excess metal ion from the reaction solution and/or the antibody complex with a buffered solution. The washing may be repeated one or more times, as is necessary. In one embodiment, the washing is repeated two or three times.

In one embodiment of the present method, two or more methods of removing excess metal ion are used in combination to remove excess metal ion from the step of removing excess metal ion from the reaction solution and/or the antibody complex.

In another embodiment of the present method, for radiopharmaceutical and radiotherapy applications, the antibody complex is prepared from a metal in an oxidation state different from that of the desired complex. In this case, either a reducing agent or an oxidizing agent, depending on the oxidation state of the metal used and the oxidation state of the desired final product, is added to the reaction mixture to bring the metal to the desired oxidation state. The oxidant or reductant can be used to form an intermediate complex in the desired oxidation state but with labile ligands. These labile ligands can then be displaced by the desired chelating moiety. In another embodiment, the labile ligands are added to the reaction mixture along with the reductant or oxidant and the desired ligand to achieve the change to the desired oxidation state and chelation to the desired metal in a single step.

In a further embodiment the method of preparing an antibody complex comprises:
preparing a first solution comprising an antibody conjugate;
adding to said first solution a second solution comprising a metal ion capable of complexing with said antibody conjugate;
mixing, agitating, or allowing to stand said first solution; and
optionally adding a secondary chelating agent, such as EDTA, to complex with uncomplexed metal ion.

In an additional embodiment, the method of preparing an antibody complex comprises:
combining a first solution, second solution, and a third solution,
wherein
said first solution comprises an antibody conjugate,
said second solution comprises an acetate buffer, and
said third solution comprises 90YC13;
mixing, agitating, or allowing to stand the combined solutions; and adding to the combined solution a fourth solution, said fourth solution comprising MeO-DOTA-NCS, human serum albumin (HSA), acetate buffer, and NaCl.

### Immunophenotyping

The antibodies such as the C35 antibodies may be utilized for immunophenotyping of cell lines and biological samples. C35 is useful as a tumor specific marker. Monoclonal antibodies directed against a specific epitope, or combination of epitopes, will allow for the diagnosis of cancer and screening of cellular populations. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (See, e.g., U.S. Pat. No. 5,985,660; and Morrison et al., Cell, 96:737-49 (1999)).

These techniques allow for the screening of particular populations of cells, such as might be found with hematological malignancies (e.g., C35-associated cancers such as breast, ovarian, bladder, and colon cancers, and melanoma).

### Diagnosis And Imaging

Labeled C35 antibodies, and derivatives and analogs thereof, which specifically bind to a polypeptide of interest can be used for diagnostic purposes to detect, diagnose, or monitor diseases, disorders, and/or conditions associated with the aberrant expression and/or activity of a polypeptide of the invention (e.g., C35-associated cancers such as breast, ovarian, bladder, and colon cancers, and melanoma). Disclosed herein is the detection of aberrant expression of a polypeptide of interest, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of aberrant expression.

Disclosed herein is a diagnostic assay for diagnosing a disorder, comprising (a) assaying the expression of the polypeptide of interest in cells or body fluid of an individual using one or more antibodies specific to the polypeptide interest and (b) comparing the level of gene expression with a standard gene expression level, whereby an increase or decrease in the assayed polypeptide gene expression level compared to the standard expression level is indicative of a particular disorder. With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Antibodies can be used to assay protein levels in a biological sample using classical immunohistological methods known to those of skill in the art (e.g., see Jalkanen, et al., J. Cell. Biol. 101:976-985 (1985); Jalkanen, et al., J. Cell Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (125I, 121I), carbon (14C), sulfur (35S), tritium (3H), indium (112In), and technetium (99Tc); luminescent labels, such as luminol; and fluorescent labels, such as fluorescein and rhodamine, and biotin.

Disclosed herein is the detection and diagnosis of a disease or disorder associated with aberrant expression of a polypeptide of interest in an animal, preferably a mammal and most preferably a human. In one embodiment, diagnosis comprises: a) administering (for example, parenterally, subcutaneously, or intraperitoneally) to a subject an effective amount of a labeled molecule which specifically binds to the polypeptide of interest; b) waiting for a time interval following the administering for permitting the labeled molecule to preferentially concentrate at sites in the subject where the polypeptide is expressed (and for unbound labeled molecule to be cleared to background level); c) determining background level; and d) detecting the labeled molecule in the subject, such that detection of labeled molecule above the background level indicates that the subject has a particular disease or disorder associated with aberrant expression of the polypeptide of interest. Background level can be determined by various methods including, comparing the amount of labeled molecule detected to a standard value previously determined for a particular system.

It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S. W. Burchiel and B. A. Rhodes, eds., Masson Publishing Inc. (1982).

Depending on several variables, including the type of label used and the mode of administration, the time interval following the administration for permitting the labeled molecule to preferentially concentrate at sites in the subject and for unbound labeled molecule to be cleared to background level is 6 to 48 hours or 6 to 24 hours or 6 to 12 hours. In another embodiment the time interval following administration is 5 to 20 days or S to 10 days.

In an embodiment, monitoring of the disease or disorder is carried out by repeating the method for diagnosing the disease or disease, for example, one month after initial diagnosis, six months after initial diagnosis, one year after initial diagnosis, etc.

Presence of the labeled molecule can be detected in the patient using methods known in the art for in vivo scanning. These methods depend upon the type of label used. Skilled artisans will be able to determine the appropriate method for detecting a particular label. Methods and devices that may be used in the diagnostic methods of the invention include, but are not limited to, computed tomography (CT), whole body scan such as position emission tomography (PET), magnetic resonance imaging (MRI), and sonography.

In a specific embodiment, the molecule is labeled with a radioisotope and is detected in the patient using a radiation responsive surgical instrument (Thurston et al., U.S. Pat. No. 5,441,050). In another embodiment, the molecule is labeled with a fluorescent compound and is detected in the patient using a fluorescence responsive scanning instrument. In another embodiment, the molecule is labeled with a positron emitting metal and is detected in the patent using positron emission-tomography. In yet another embodiment, the molecule is labeled with a paramagnetic label and is detected in a patient using magnetic resonance imaging (MRI).

The antibodies are useful for diagnosis, treatment, prevention and/or prognosis of cancer disorders in mammals, preferably humans. Such disorders include, but are not limited to, cancer, neoplasms, tumors and/or as described under "Hyperproliferative Disorders" below, especially C35-associated cancers such as breast, ovarian, bladder, and colon cancers, and melanoma.

. In particular, it is believed that certain tissues in mammals with cancer express significantly enhanced or reduced levels of normal or altered cancer antigen expression and mRNA encoding the cancer associated polypeptide when compared to a corresponding "standard" level. Further, it is believed that enhanced or depressed levels of the cancer associated polypeptide can be detected in certain body fluids (e.g., sera, plasma, urine, and spinal fluid) or cells or tissue from mammals with such a cancer when compared to sera from mammals of the same species not having the cancer.

For example, as disclosed herein, C35 expression is associated with certain cancers (e.g., breast, ovarian, bladder, colon, and pancreatic cancers, and melanoma). Accordingly, antibodies (and antibody fragments) directed against C35 may be used to quantitate or qualitate concentrations of cells expressing C35. These antibodies additionally have diagnostic applications in detecting abnormalities in the level of C35 expression, or abnormalities in the structure and/or temporal, tissue, cellular, or subcellular location of C35. These diagnostic assays may be performed in vivo or in vitro, such as, for example, on blood samples, biopsy tissue or autopsy tissue.

Disclosed herein is a diagnostic method useful during diagnosis of a cancers, which involves measuring the expression level of the cancer antigen polypeptide in tissue or other cells or body fluid from an individual and comparing the measured expression level with a standard cancer antigen expression level, whereby an increase in the expression level compared to the standard is indicative of a disorder.

Where a diagnosis of a disorder, including diagnosis of a tumor, has already been made according to conventional methods, the present invention is useful as a prognostic indicator, whereby patients exhibiting enhanced cancer antigen gene expression will experience a worse clinical outcome relative to patients expressing the gene at a level nearer the standard level.

By "assaying the expression level of the cancer associated polypeptide" is intended qualitatively or quantitatively measuring or estimating the level of the cancer antigen polypeptide in a first biological sample either directly (e.g., by determining or estimating absolute protein level) or relatively (e.g., by comparing to the cancer associated polypeptide level in a second biological sample). Preferably, the cancer antigen polypeptide expression level in the first biological sample is measured or estimated and compared to a standard cancer antigen polypeptide level, the standard being taken from a second biological sample obtained from an individual not having the disorder or being determined by averaging levels from a population of individuals not having the disorder. As will be appreciated in the art, once a standard cancer antigen polypeptide level is known, it can be used repeatedly as a standard for comparison.

By "biological sample" is intended any biological sample obtained from an individual, cell line, tissue culture, or other source containing cancer antigen polypeptides (including portions thereof). As indicated, biological samples include body fluids (such as sera, plasma, urine, synovial fluid and spinal fluid) which contain cells expressing cancer antigen polypeptides, and other tissue sources found to express the full length or fragments thereof of a cancer antigen. Methods for obtaining tissue biopsies and body fluids from mammals are well known in the art.

Disclosed herein are diagnostic assays such as quantitative and diagnostic assays for detecting levels of cancer antigen polypeptides, in a biological sample (e.g., cells and tissues), including determination of normal and abnormal levels of polypeptides. Thus, for instance, a diagnostic assay in accordance with the invention for detecting over-expression of cancer antigens compared to normal control tissue samples may be used to detect the presence of tumors. Assay techniques that can be used to determine levels of a polypeptide, such as a cancer antigen polypeptide of the present invention in a sample derived from a host are well-known to those of skill in the art. Such assay methods include radioimmunoassays, competitive-binding assays, Western Blot analysis and ELISA assays. Assaying cancer antigen polypeptide levels in a biological sample can occur using any art-known method.

Cancer antigen polypeptide expression in tissues can also be studied with classical immunohistological methods (Jalkanen et al., J. Cell. Biol. 101:976-985 (1985); Jalkanen, M., et al., J. Cell . Biol. 105:3087-3096 (1987)). Other antibody-based methods useful for detecting cancer antigen polypeptide gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

The tissue or cell type to be analyzed will generally include those which are known, or suspected, to express the cancer antigen gene. The protein isolation methods employed herein may, for example, be such as those described in Harlow and Lane (Harlow, E. and Lane, D., 1988, "Antibodies: A Laboratory Manual", Cold Spring Harbor Laboratory Press, Cold Spring Harbor, New York), The isolated cells can be derived from cell culture or from a patient. The analysis of cells taken from culture may be a necessary step in the assessment of cells that could be used as part of a cell-based gene therapy technique or, alternatively, to test the effect of compounds on the expression of the cancer antigen gene.

In an additional preferred embodiment, antibodies, or fragments of antibodies directed to a conformational epitope of a cancer antigen may be used to quantitatively or qualitatively detect the presence of cancer antigen gene products or conserved variants or peptide fragments thereof. This can be accomplished, for example, by immunofluorescence techniques employing a fluorescently labeled antibody coupled with light microscopic, flow cytometric, or fluorimetric detection.

The antibodies (or fragments thereof), and/or cancer antigen polypeptides of the present invention may, additionally, be employed histologically, as in immunofluorescence, immunoelectron microscopy or non-immunological assays, for in situ detection of cancer antigen gene products or conserved variants or peptide fragments thereof. In situ detection may be accomplished by removing a histological specimen from a patient, and applying thereto a labeled antibody or cancer antigen polypeptide of the present invention. The antibody (or fragment thereof) or cancer antigen polypeptide is preferably applied by overlaying the labeled antibody (or fragment) onto a biological sample. Through the use of such a procedure, it is possible to determine not only the presence of the cancer antigen gene product, or conserved variants or peptide fragments, or cancer antigen polypeptide binding, but also its distribution in the examined tissue. Using the present invention, those of ordinary skill will readily perceive that any of a wide variety of histological methods (such as staining procedures) can be modified in order to achieve such in situ detection.

Immunoassays and non-immunoassays for cancer antigen gene products or conserved variants or peptide fragments thereof will typically comprise incubating a sample, such as a biological fluid, a tissue extract, freshly harvested cells, or lysates of cells which have been incubated in cell culture, in the presence of a detectably labeled antibody capable of binding cancer antigen gene products or conserved variants or peptide fragments thereof, and detecting the bound antibody by any of a number of techniques well-known in the art.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled anti-cancer antigen antibody or detectable cancer antigen polypeptide. The solid phase support may then be washed with the buffer a second time to remove unbound antibody or polypeptide. Optionally the antibody is subsequently labeled. The amount of bound label on solid support may then be detected by conventional means.

By "solid phase support or carrier" is intended any support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of anti-cancer antigen antibody or cancer antigen polypeptide may be determined according to well known methods. Those skilled in the art will be able to determine operative and optimal assay conditions for each determination by employing routine experimentation.

In addition to assaying cancer antigen polypeptide levels or polynucleotide levels in a biological sample obtained from an individual, cancer antigen polypeptide or polynucleotide can also be detected in vivo by imaging. For example, cancer antigen polypeptide and/or anti-cancer antigen antibodies are used to image diseased cells, such as neoplasms. In another embodiment, cancer antigen polynucleotides (e.g., polynucleotides complementary to all or a portion of cancer antigen mRNA) and/or anti-cancer antigen antibodies (e.g., antibodies directed to any one or a combination of the epitopes of cancer antigens, antibodies directed to a conformational epitope of cancer antigens, antibodies directed to the full length polypeptide expressed on the cell surface of a mammalian cell) are used to image diseased or neoplastic cells.

Antibody labels or markers for in vivo imaging of cancer antigen polypeptides include those detectable by X-radiography, NMR, MRI, CAT-scans or ESR For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. Where in vivo imaging is used to detect enhanced levels of cancer antigen polypeptides for diagnosis in humans, it may be preferable to use human antibodies or "humanized" chimeric monoclonal antibodies. Such antibodies can be produced using techniques described herein or otherwise known in the art. For example methods for producing chimeric antibodies are known in the art. See, for review, Morrison, Science 229:1202 (1985); Oi et al., BioTechniques 4:214 (1986); Cabilly et al., U.S. Pat. No. 4,816,567; Taniguchi et al., EP 171496; Morrison et al., EP 173494; Neuberger et al., WO 8601533; Robinson et al., WO 8702671; Boulianne et al., Nature 312:643 (1984); Neuberger et al., Nature 314:268 (1985).

Additionally, any cancer antigen polypeptides whose presence can be detected, can be administered. For example, cancer antigen polypeptides labeled with a radio-opaque or other appropriate compound can be administered and visualized in vivo, as discussed, above for labeled antibodies. Further such cancer antigen polypeptides can be utilized for in vitro diagnostic procedures.

A cancer antigen polypeptide-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously or intraperitoneally) into the mammal to be examined for a disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain cancer antigen protein. In vivo tumor imaging is described in S. W. Burchiel et al., "Immunopharmacokinetics of Radiolabeled Antibodies and Their Fragments" (Chapter 13 in Tumor Imaging: The Radiochemical Detection of Cancer, S. W. Burchiel and B. A. Rhodes, eds., Masson Publishing Inc. (1982)).

With respect to antibodies, one of the ways in which the anti-cancer antigen antibody can be detectably labeled is by linking the same to an enzyme and using the linked product in an enzyme immunoassay (EIA) (Voller, A., "The Enzyme Linked Immunosorbent Assay (ELISA)", 1978, Diagnostic Horizons 2:1-7, Microbiological Associates Quarterly Publication, Walkersville, Md.); Voller et al., J. Clin. Pathol. 31:507-520 (1978); Butler, J. E., Meth. Enrymol. 73:482-523 (1981); Maggio, E. (ed.), 1980, Enzyme Immunoassay, CRC Press, Boca Raton, Fla.,; Ishikawa, E. et al., (eds.), 1981, Enzyme Immunoassay, Kgaku Shoin, Tokyo). The enzyme, which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. Additionally, the detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect cancer antigens through the use of a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by means including, but not limited to, a gamma counter, a scintillation counter, or autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, ophthaldehyde and fluorescamine.

The antibody can also be detectably labeled using fluorescence emitting metals such as 152Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody . Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

In general, cancer may be detected in a patient based on the presence of one or more cancer antigen proteins of the invention and/or polynucleotides encoding such proteins in a biological sample (for example, blood, sera, urine, and/or tumor biopsies) obtained from the patient. In other words, such proteins and/or polynucleotides may be used as markers to indicate the presence or absence of cancer. Cancers that may be diagnosed, and/or prognosed using the compositions of the invention include but are not limited to, colorectal cancer, breast cancer, ovarian cancer, prostate cancer, pancreatic cancer, lung cancer, liver cancer, uterine cancer, and/or skin cancer. The binding agents provided herein generally permit detection of the level of antigen that binds to the agent in the biological sample. Polynucleotide primers and probes may be used to detect the level of MRNA encoding cancer antigen polypeptides, which is also indicative of the presence or absence of cancer. In general, cancer antigen polypeptides should be present at a level that is at least three fold higher in diseased tissue than in normal tissue.

There are a variety of assay formats known to those of ordinary skill in the art for using a binding agent to detect polypeptide markers in a sample. See, e.g., Harlow and Lane, supra. In general, the presence or absence of a disease in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the cancer antigen polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include cancer antigen polypeptides and portions thereof, or antibodies, to which the binding agent binds, as described above.

The solid support may be any material known to those of skill in the art to which cancer antigen polypeptides may be attached. For example, the solid support may be a test well in a microtiter plate or a nitrocellulose or other suitable membrane. Alternatively, the support may be a bead or disc, such as glass fiberglass, latex or a plastic material such as polystyrene or polyvinylchloride. The support may also be a magnetic particle or a fiber optic sensor, such as those disclosed, for example, in U.S. Pat. No. 5,359,681. The binding agent may be immobilized on the solid support using a variety of techniques known to those of skill in the art, which are amply described in the patent and scientific literature.
The term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for the suitable amount of time. The contact time varies with temperature, but is typically between about 1 hour and about 1 day. In general, contacting a well of plastic microtiter plate (such as polystyrene or polyvinylchloride) with an amount of binding agent ranging from about 10 ng to about 10 ug, and preferably about 100 ng to about 1 ug, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (see, e.g., Pierce Immunotechnology Catalog and Handbook, 1991, at A12-A13).

### Therapeutic/Prophylactic Administration And Composition

In one embodiment, the entire antibody dose is provided in a single bolus. Alternatively, the dose can be provided by multiple administrations, such as an extended infusion method or by repeated injections administered over a span of hours or days, for example, a span of about 2 to about 4 days. Also see Examples 5, 9 and 10, and Tables 6-9.

Formulations and methods of administration that can be employed when the compound comprises a nucleic acid or an immunoglobulin are described above; additional appropriate formulations and routes of administration can be selected from among those described herein below.

Various delivery systems are known and can be used to administer a compound , e.g., encapsulation in liposomes, microparticles, microcapsules, recombinant cells capable of expressing the compound, receptor-mediated endocytosis (see, e.g., Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, it may be desirable to introduce the pharmaceutical compounds or compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it may be desirable to administer the pharmaceutical compounds or compositions locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the invention, care must be taken to use materials to which the protein does not absorb.

In another embodiment, the compound or composition can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the compound or composition can be delivered in a controlled release system. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., Macromol. Sci. Rev. Macromol. Chem. 23:61 (1983); see also Levy et al., Science 228:190 (1985); During et al., Ann. Neutral. 25:351 (1989); Howard et al., J.Neurosurg. 71:105 (1989)). In yet another embodiment, a controlled release system can be placed in proximity of the therapeutic target, i.e., the brain, thus requiring only a fraction of the systemic dose (see, e.g., Goodson, in Medical Applications of Controlled Release, supra, vol. 2, pp. 115-138 (1984)).

Other controlled release systems are discussed in the review by Langer (Science 249:1527-1533 (1990)).

In a specific embodiment where the compound is a nucleic acid encoding a protein, the nucleic acid can be administered in vivo to promote expression of its encoded protein, by constructing it as part of an appropriate nucleic acid expression vector and administering it so that it becomes intracellular, e.g., by use of a retroviral vector (see U.S. Pat. No. 4,980,286), or by direct injection, or by use of microparticle bombardment (e.g., a gene gun; Biolistic, Dupont), or coating with lipids or cell-surface receptors or transfecting agents, or by administering it in linkage to a homeobox- like peptide which is known to enter the nucleus (see e.g., Joliot et al., Proc. Natl. Acad. Sci. USA 88:1864-1868 (1991)), etc. Alternatively, a nucleic acid can be introduced intracellularly and incorporated within host cell DNA for expression, by homologous recombination.

Disclosed herein are pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The amount of the compound which will be effective in the treatment, inhibition and prevention of a disease or disorder associated with aberrant expression and/or activity of a polypeptide of the invention can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems.

For antibodies, the dosage administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human antibodies have a longer half-life within the human body than antibodies from other species due to the immune response to the foreign polypeptides. Thus, lower dosages of human antibodies and less frequent administration is often possible. Further, the dosage and frequency of administration of antibodies of the invention may be reduced by enhancing uptake and tissue penetration (e.g., into the brain) of the antibodies by modifications such as, for example, lipidation. Also see Example 5.

Disclosed herein is a pharmaceutical pack or kit comprising one or more containers filled with one or more of the ingredients of the pharmaceutical compositions Optionally associated with such container(s) can be a notice in the form prescribed by a governmental agency regulating the manufacture, use or sale of pharmaceuticals or biological products, which notice reflects approval by the agency of manufacture, use or sale for human administration.

Antibodies can be used to assay levels of polypeptides encoded by polynucleotides of the invention in a biological sample using classical immunohistological methods known to those of skill in the art (e.g., see Jalkanen, et al., J. Cell. Biol. 101:976-985 (1985); Jalkanen, et al., J. Cell. Biol. 105:3087-3096 (1987)). Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase; radioisotopes, such as iodine (¹³¹I, ¹²⁵I, ¹²³I, ¹²¹I), carbon (¹⁴C), sulfur (³⁵S), tritium (³H), indium (¹¹⁵mIn, ¹¹³In, ¹¹²In, ¹¹¹In), and technetium (⁹⁹Tc, ⁹⁹mTc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ⁸⁸Re, ¹⁴²Pr, ¹⁰⁵Rh, ⁹⁷Ru; luminescent labels, such as luminol; and fluorescent labels, such as fluorescein and rhodamine, and biotin.

In addition to assaying levels of polypeptide in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, ¹³¹I, 1¹²In, ⁹⁹mTc, (¹³¹I, ¹²⁵I, ¹²³I, ¹²¹I), carbon (¹⁴C), sulfur (35S), tritium (³H), indium (¹¹⁵mIn, ¹¹³mIn, ¹¹²In, ¹¹¹In), and technetium (⁹⁹Tc, ⁹⁹mTc), thallium (^{20I}Ti), gallium (⁶⁸Ga, ⁶⁷Ga), palladium (¹⁰³Pd), molybdenum (⁹⁹Mo), xenon (¹³³Xe), fluorine (¹⁸F, ¹⁵³Sm, ¹⁷⁷Lu, ⁵⁹Gd, ¹⁴⁹Pm, ¹⁴⁰La, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru), a radio-opaque substance, or a material detectable by nuclear magnetic resonance, is introduced (for example, parenterally, subcutaneously or intraperitoneally) into the mammal to be examined for immune system disorder. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of ⁹⁹mTc. The labeled antibody or antibody fragment will then preferentially accumulate at the location of cells which express the polypeptide encoded by a polynucleotide of the invention. In vivo tumor imaging is described in S.W. Burchiel et al., "Immunopharmacokinetics of Radiolabeled Antibodies and Their Fragments" (Chapter 13 in Tumor Imaging: The Radiochemical Detection of Cancer, S. W. Burchiel and B. A. Rhodes, eds., Masson Publishing Inc. (1982)).

Disclosed herein is a method for the specific delivery of compositions to cells by administering polypeptides (e.g., polypeptides encoded by polynucleotides of the invention and/or antibodies) that are associated with heterologous polypeptides or nucleic acids. In one example, disclosed herein is a method for delivering a therapeutic protein into the targeted cell. In another example, disclosed herein is a method for delivering a single stranded nucleic acid acid (e.g., antisense or ribozymes) or double stranded nucleic acid (e.g., DNA that can integrate into the cell's genome or replicate episomally and that can be transcribed) into the targeted cell.

Techniques known in the art may be applied to label polypeptides of the invention (including antibodies). Such techniques include, but are not limited to, the use of bifunctional conjugating agents (see e.g., U.S. Pat. Nos. 5,756,065; 5,714,631; 5,696,239; 5,652,361; 5,505,931; 5,489,425; 5,435,990; 5,428,139; 5,342,604; 5,274,119; 4,994,560; and 5,808,003,

### Gene Therapy

In a specific embodiment, nucleic acids comprising sequences encoding antibodies such as C35 antibodies, or functional derivatives thereof, are administered to treat, inhibit or prevent a disease or disorder associated with aberrant expression and/or activity of C35, by way of gene therapy. Gene therapy refers to therapy performed by the administration to a subject of an expressed or expressible nucleic acid. In this embodiment, the nucleic acids produce their encoded protein that mediates a therapeutic effect.

Any of the methods for gene therapy available in the art can be used Exemplary methods are described below.

For general reviews of the methods of gene therapy, see Goldspiel et al., Clinical Pharmacy 12:488-505 (1993); Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); May, TIBTECH 11(5):155-215 (1993). Methods commonly known in the art of recombinant DNA technology which can be used are described in Ausubel et al. (eds.), Current Protocols in Molecular Biology, John Wiley & Sons, NY (1993); and Kriegler, Gene Transfer and Expression, A Laboratory Manual, Stockton Press, NY (1990).

In a preferred aspect, the compound comprises nucleic acid sequences encoding an antibody, said nucleic acid sequences being part of expression vectors that express the antibody or fragments or chimeric proteins or heavy or light chains thereof in a suitable host. In particular, such nucleic acid sequences have promoters operably linked to the antibody coding region, said promoter being inducible or constitutive, and, optionally, tissue-specific. In another particular embodiment, nucleic acid molecules are used in which the antibody coding sequences and any other desired sequences are flanked by regions that promote homologous recombination at a desired site in the genome, thus providing for intrachromosomal expression of the antibody encoding nucleic acids (Koller and Smithies, Proc. Natl. Acad. Sci. USA 86:8932-8935 (1989); Zijlstra et al., Nature 342:435-438 (1989). In specific embodiments, the expressed antibody molecule is a single chain antibody; alternatively, the nucleic acid sequences include sequences encoding both the heavy and light chains, or fragments thereof, of the antibody.

Delivery of the nucleic acids into a patient may be either direct, in which case the patient is directly exposed to the nucleic acid or nucleic acid-carrying vectors, or indirect, in which case, cells are first transformed with the nucleic acids in vitro, then transplanted into the patient. These two approaches are know, respectively, as in vivo or ex vivo gene therapy.

In a specific embodiment, the nucleic acid sequences are directly administered in vivo, where it is expressed to produce the encoded product This can be accomplished by any of numerous methods known in the art, e.g., by constructing them as part of an appropriate nucleic acid expression vector and administering it so that they become intracellular, e.g., by infection using defective or attenuated retrovirals or other viral vectors (see U.S. Pat. No. 4,980,286), or by direct injection of naked DNA, or by use of microparticle bombardment (e.g., a gene gun; Biolistic, Dupont), or coating with lipids or cell-surface receptors or transfecting agents, encapsulation in liposomes, microparticles, or microcapsules, or by administering them in linkage to a peptide which is known to enter the nucleus, by administering it in linkage to a ligand subject to receptor-mediated endocytosis (see, e.g., Wu and Wu, J. Biol. Chem. 262:4429-4432 (1987)) (which can be used to target cell types specifically expressing the receptors), etc. In another embodiment, nucleic acid-ligand complexes can be formed in which the ligand comprises a fusogenic viral peptide to disrupt endosomes, allowing the nucleic acid to avoid lysosomal degradation. In yet another embodiment, the nucleic acid can be targeted in vivo for cell specific uptake and expression, by targeting a specific receptor (see, e.g., PCT Publications WO 92/06180; WO 92/22635; WO92/20316; WO93/14188, WO 93/20221). Alternatively, the nucleic acid can be introduced intracellularly and incorporated within host cell DNA for expression, by homologous recombination (Koller and Smithies, Proc. Natl. Acad. Sci. USA 86:8932-8935 (1989); Zijlstra et al., Nature 342:435-438 (1989)).

In a specific embodiment, viral vectors that contains nucleic acid sequences encoding an antibody are used. For example, a retroviral vector can be used (see Miller et al., Meth. Enzymol. 217:581-599 (1993)). These retroviral vectors contain the components necessary for the correct packaging of the viral genome and integration into the host cell DNA. The nucleic acid sequences encoding the antibody to be used in gene therapy are cloned into one or more vectors, which facilitates delivery of the gene into a patient. More detail about retroviral vectors can be found in Boesen et al., Biotherapy 6:291-302 (1994), which describes the use of a retroviral vector to deliver the mdrl gene to hematopoietic stem cells in order to make the stem cells more resistant to chemotherapy. Other references illustrating the use of retroviral vectors in gene therapy are: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993).

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, Current Opinion in Genetics and Development 3:499-503 (1993) present a review of adenovirus-based gene therapy. Bout et al., Human Gene Therapy 5:3-10 (1994) demonstrated the use of adenovirus vectors to transfer genes to the respiratory epithelia of rhesus monkeys. Other instances of the use of adenoviruses in gene therapy can be found in Rosenfeld et al., Science 252:431434 (1991); Rosenfeld et al., Cell 68:143-155 (1992); Mastrangeli et al., J. Clin. Invest. 91:225-234 (1993); PCT Publication WO94/12649; and Wang, et al., Gene Therapy 2:775-783 (1995). In a preferred embodiment, adenovirus vectors are used.

Adeno-associated virus (AAV) has also been proposed for use in gene therapy (Walsh et al., Proc. Soc. Exp. Biol. Med. 204:289-300 (1993); U.S. Pat. No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration in vivo of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler and Behr, Meth. Enzymol. 217:599-618 (1993); Cohen et al., Meth. Enzymol. 217:618-644 (1993); Cline, Pharmac. Ther. 29:69-92m (1985) and may be used , provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. Recombinant blood cells (e.g., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to epithelial cells, endothelial cells, keratinocytes, fibroblasts, muscle cells, hepatocytes; blood cells such as Tlymphocytes, Blymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc.

In a preferred embodiment, the cell used for gene therapy is autologous to the patient.

In an embodiment in which recombinant cells are used in gene therapy, nucleic acid sequences encoding an antibody are introduced into the cells such that they are expressible by the cells or their progeny, and the recombinant cells are then administered in vivo for therapeutic effect. In a specific embodiment, stem or progenitor cells are used. Any stem and/or progenitor cells which can be isolated and maintained in vitro can potentially be used (see e.g. PCT Publication WO 94/08598; Stemple and Anderson, Cell 71:973-985 (1992); Rheinwald, Meth. Cell Bio. 21A:229 (1980); and Pittelkow and Scott, Mayo Clinic Proc. 61:771 (1986)).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

### Hyperproliferative Diseases

The method can be used to treat hyperproliferative diseases, disorders, and/or conditions, including neoplasms.

Examples of hyperproliferative diseases, disorders, and/or conditions that can be treated by the method of the invention include, but are not limited to neoplasms located in the: prostate, colon, abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Other examples of such hyperproliferative disorders include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumors, Breast Cancer, Cancer of the Renal Pelvis and Ureter, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Childhood Cerebellar Astrocytoma, Childhood Cerebral Astrocytoma, Childhood Extracranial Germ Cell Tumors, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumors, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumors, Exocrine Pancreatic Cancer, Extracranial Germ Cell Tumor, Extragonadal Germ Cell Tumor, Extrahepatic Bile Duct Cancer, Eye Cancer, Female Breast Cancer, Gaucher's Disease, Gallbladder Cancer, Gastric Cancer, Gastrointestinal Carcinoid Tumor, Gastrointestinal Tumors, Germ Cell Tumors, Gestational Trophoblastic Tumor, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumor, Ovarian Low Malignant Potential Tumor, Pancreatic Cancer, Paraproteinemias, Purpura, Parathyroid Cancer, Penile Cancer, Pheochromocytoma, Pituitary Tumor, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Ureter Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumors, T-Cell Lymphoma, Testicular Cancer, Thymoma, Thyroid Cancer, Transitional Cell Cancer of the Renal Pelvis and Ureter, Transitional Renal Pelvis and Ureter Cancer, Trophoblastic Tumors, Ureter and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, Wilms' Tumor, and any other hyperproliferative disease, besides neoplasia, located in an organ system listed above.

The method may be used to treat premalignant conditions and to prevent progression to a neoplastic or malignant state, including but not limited to those disorders described above. Such uses are indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins and Angell, 1976, Basic Pathology, 2d Ed., W. B. Saunders Co., Philadelphia, pp, 68-79.)

Hyperplasia is a form of controlled cell proliferation, involving an increase in cell number in a tissue or organ, without significant alteration in structure or function. Hyperplastic disorders which can be treated by the method of the invention include, but are not limited to, angiofollicular mediastinal lymph node hyperplasia, angiolymphoid hyperplasia with eosinophilia, atypical melanocytic hyperplasia, basal cell hyperplasia, benign giant lymph node hyperplasia, cementum hyperplasia, congenital adrenal hyperplasia, congenital sebaceous hyperplasia, cystic hyperplasia, cystic hyperplasia of the breast, denture hyperplasia, ductal hyperplasia, endometrial hyperplasia, fibromuscular hyperplasia, focal epithelial hyperplasia, gingival hyperplasia, inflammatory fibrous hyperplasia, inflammatory papillary hyperplasia, intravascular papillary endothelial hyperplasia, nodular hyperplasia of prostate, nodular regenerative hyperplasia, pseudoepitheliomatous hyperplasia, senile sebaceous hyperplasia, and verrucous hyperplasia.

Metaplasia is a form of controlled cell growth in which one type of adult or fully differentiated cell substitutes for another type of adult cell. Metaplastic disorders which can be treated by the method of the invention include, but are not limited to, agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, and symptomatic myeloid metaplasia.

Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnonnally large, deeply stained nuclei, and exhibit pleomorphism. Dysplasia characteristically occurs where there exists chronic irritation or inflammation. Dysplastic disorders which can be treated by the method of the invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis hemimelia, dysplasia epiphysialis multiplex, dysplasia epiphysialis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia, hidrotic ectodermal dysplasia, hypohidrotic ectodermal dysplasia, lymphopenic thymic dysplasia, mammary dysplasia, mandibulofacial dysplasia, metaphysial dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Additional pre-neoplastic disorders which can be treated by the method include, but are not limited to, benign dysproliferative disorders (e.g., benign tumors, fibrocystic conditions, tissue hypertrophy, intestinal polyps, colon polyps, and esophageal dysplasia), leukoplakia, keratoses, Bowen's disease, Farmer's Skin, solar cheilitis, and solar keratosis.

In preferred embodiments, the method is used to inhibit growth, progression, and/or metastasis of cancers, in particular those listed above.

Additional diseases or conditions associated with increased cell survival that could be treated by the method of the invention, include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, emangioblastoma, acoustic neuroma, oligodendroglioma, menangioma, melanoma, neuroblastoma, and retinoblastoma.

Hyperproliferative diseases and/or disorders that could be treated by the method, include, but are not limited to, neoplasms located in the liver, abdomen, bone, breast, digestive system, pancreas, peritoneum, endocrine glands (adrenal, parathyroid, pituitary, testicles, ovary, thymus, thyroid), eye, head and neck, nervous system (central and peripheral), lymphatic system, pelvis, skin, soft tissue, spleen, thorax, and urogenital tract.

Similarly, other hyperproliferative disorders can also be treated by the method of the invention. Examples of such hyperproliferative disorders include, but are not limited to: hypergammaglobulinemia, lymphoproliferative disorders, paraproteinemias, purpura, sarcoidosis, Sezary Syndrome, Waldenstron's macroglobulinemia, Gaucher's Disease, histiocytosis, and any other hyperproliferative disease, besides neoplasia, located in an organ system listed above.

### Demonstration Of Therapeutic Activity

The methods and antibodies are preferably tested in vitro, and then in vivo for the desired therapeutic or prophylactic activity, prior to use in humans. For example, in vitro assays to demonstrate the therapeutic or prophylactic utility of a compound or pharmaceutical composition include the effect of a compound on a cell line or a patient tissue sample. The effect of the compound or composition on the cell line and/or tissue sample can be determined utilizing techniques known to those of skill in the art including, but not limited to, rosette formation assays and cell lysis assays. In accordance with the invention, in vitro assays which can be used to determine whether administration of a specific compound is indicated, include in vitro cell culture assays in which a patient tissue sample is grown in culture, and exposed to or otherwise administered a compound, and the effect of such compound upon the tissue sample is observed.

### Kits

Disclosed herein are kits that can be used in the above methods. In one embodiment, a kit comprises an antibody preferably a purified antibody, in one or more containers. In a specific embodiment, the kits contain a substantially isolated polypeptide comprising an epitope which is specifically immunoreactive with an antibody included in the kit. Preferably, the kits further comprise a control antibody which does not react with the polypeptide of interest. In another specific embodiment, the kits contain a means for detecting the binding of an antibody to a polypeptide of interest (e.g., the antibody may be conjugated to a detectable substrate such as a fluorescent compound, an enzymatic substrate, a radioactive compound or a luminescent compound, or a second antibody which recognizes the first antibody may be conjugated to a detectable substrate).

In another specific embodiment, the kit is a diagnostic kit for use in screening serum containing antibodies specific against proliferative and/or cancerous polynucleotides and polypeptides. Such a kit may include a control antibody that does not react with the polypeptide of interest. Such a kit may include a substantially isolated polypeptide antigen comprising an epitope which is specifically immunoreactive with at least one anti-polypeptide antigen antibody. Further, such a kit includes means for detecting the binding of said antibody to the antigen (e.g., the antibody may be conjugated to a fluorescent compound such as fluorescein or rhodamine which can be detected by flow cytometry). In specific embodiments, the kit may include a recombinantly produced or chemically synthesized polypeptide antigen. The polypeptide antigen of the kit may also be attached to a solid support.

In a more specific embodiment the detecting means of the above-described kit includes a solid support to which said polypeptide antigen is attached. Such a kit may also include a non-attached reporter-labeled anti-human antibody. In this embodiment, binding of the antibody to the polypeptide antigen can be detected by binding of the said reporter-labeled antibody.

Disclosed herein is a diagnostic kit for use in screening samples containing antigens of the polypeptide.
The diagnostic kit includes a substantially isolated antibody specifically immunoreactive with polypeptide or polynucleotide antigens, and means for detecting the binding of the polynucleotide or polypeptide antigen to the antibody. In one embodiment, the antibody is attached to a solid support. In a specific embodiment, the antibody may be a monoclonal antibody. The detecting means of the kit may include a second, labeled monoclonal antibody. Alternatively, or in addition, the detecting means may include a labeled, competing antigen.

In one diagnostic configuration, test sample is reacted with a solid phase reagent having a surface-bound antigen obtained by the methods of the present invention. After binding with specific antigen antibody to the reagent and removing unbound sample components by washing, the reagent is reacted with reporter-labeled anti-human antibody to bind reporter to the reagent in proportion to the amount of bound anti-antigen antibody on the solid support. The reagent is again washed to remove unbound labeled antibody, and the amount of reporter associated with the reagent is determined. Typically, the reporter is an enzyme which is detected by incubating the solid phase in the presence of a suitable fluorometric, luminescent or calorimetric substrate (Sigma, St. Louis, Mo.).

The solid surface reagent in the above assay is prepared by known techniques for attaching protein material to solid support material, such as polymeric beads, dip sticks, 96-well plate or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

Disclosed herein is an assay system or kit for carrying out this diagnostic method. The kit generally includes a support with surface- bound recombinant antigens, and a reporter-labeled anti-human antibody for detecting surface-bound anti-antigen antibody.

### Apoptosis-Inducing Therapies

Apoptosis-inducing therepies include chemotherapeutic agents (also known as antineoplastic agents), radiation therapy, and combination radiotherapy and chemotherapy.

Exemplary chemotherapeutic agents are vinca alkaloids, epipodophyllotoxins, anthracycline antibiotics, actinomycin D, plicamycin, puromycin, gramicidin D, paclitaxel (Taxol.RTM., Bristol Myers Squibb), colchicine, cytochalasin B, emetine, maytansine, and amsacrine (or "mAMSA"). The vinca alkaloid class is described in Goodman and Gilman's The Pharmacological Basis of Therapeutics (7th ed.), (1985), pp. 1277-1280. Exemplary of vinca alkaloids are vincristine, vinblastine, and vindesine. The epipodophyllotoxin class is described in Goodman and Gilman's The Pharmacological Basis of Therapeutics (7th ed.), (1985), pp. 1280-1281. Exemplary of epipodophyllotoxins are etoposide, etoposide orthoquinone, and teniposide. The anthracycline antibiotic class is described in Goodman and Gilman's The Pharmacological Basis of Therapeutics (7th ed.), (1985), pp. 1283-1285. Exemplary of anthracycline antibiotics are daunorubicin, doxorubicin, mitoxantraone, and bisanthrene. Actinomycin D, also called Dactinomycin, is described in Goodmand and Gilman's The Pharmacological Basis of Therapeutics (7th ed.), (1985), pp. 1281-1283. Plicamycin, also called mithramycin, is described in Goodmand and Gilman's The Pharmacological Basis of Therapeutics (7th ed), (1985), pp.1287-1288. Additional chemotherapeutic agents include cisplatin (Platinol.RTM., Bristol Myers Squibb), carboplatin (Paraplatin.RTM., Bristol Myers Squibb), mitomycin (Mutamycin.RTM., Bristol Myers Squibb), altretamine (Hexalen.RTM., U.S. Bioscience, Inc.), cyclophosphamide (Cytoxan.RTM., Bristol Myers Squibb), lomustine (CCNU) (CeeNU.RTM., Bristol Myers Squibb), carmustine (BCNU) (BiCNU.RTM., Bristol Myers Squibb).

Exemplary chemotherapeutic agents also include aclacinomycin A, aclarubicin, acronine, acronycine, adriamycin, aldesleukin (interleukin-2), altretamine (hexamiethylmelamine), aminoglutethimide, aminoglutethimide (cytadren), aminoimidazole carboxamide, amsacrine (m-AMSA; amsidine), anastrazole (arimidex), ancitabine, anthracyline, anthramycin, asparaginase (elspar), azacitdine, azacitidine (ladakamycin), azaguanine, azaserine, azauridine, 1,1',1"-phosphinothioylidynetris aziridine, azirino(2', 3':3,4)pyrrolo(1,2-a)indole-4,7-dione, BCG (theracys), BCNU, BCNU chloroethyl nitrosoureas, benzamide, 4-(bis(2-chloroethyl)amino)benzenebutanoic acid, bicalutamide, bischloroethyl nitrosourea, bleomycin, bleomycin (blenozane), bleomycins, bromodeoxyuridine, broxuridine, busulfan (myleran), carbamic acid ethyl ester, carboplatin, carboplatin (paraplatin), carmustine, carmustine (BCNU; BiCNU), chlorambucil (leukeran), chloroethyl nitrosoureas, chorozotocin (DCNU), chromomycin A3, cis-retinoic acid, cisplatin (cis-ddpl; platinol), cladribine (2-chlorodeoxyadenosine; 2cda; leustatin), coformycin, cycloleucine, cyclophosphamide, cyclophosphamide anhydrous, chlorambucil, cytarabine, cytarabine, cytarabine HCl (cytosar-u), 2-deoxy-2-(((methylnitrosoamino)carbonyl)amino)-D-glucose, dacarbazine, dactinomycin (cosmegen), daunorubicin, Daunorubincin HCl (cerubidine), decarbazine, decarbazine (DTIC-dome), demecolcine, dexamethasone, dianhydrogalactitol, diazooxonorleucine, diethylstilbestrol, docetaxel (taxotere), doxorubicin HCl (adriamycin), doxorubicin hydrochloride, eflomithine, estramustine, estramustine phosphate sodium (emcyt), ethiodized oil, ethoglucid, ethyl carbamate, ethyl methanesulfonate, etoposide (VP16-213), fenretinide, floxuridine, floxuridine (fudr), fludarabine (fludara), fluorouracil (5-FU), fluoxymesterone (halotestin), flutamide, flutamide (eulexin), fluxuridine, gallium nitrate (granite), gemcitabine (gemzar), genistein, 2-deoxy-2-(3-methyl-3-nitrosoureido)-D-glucopyranose, goserelin (zoladex), hexestrol, hydroxyurea (hydra), idarubicin (idamycin), ifosfagemcitabine, ifosfamide (iflex), ifosfamide with mesna (MAID), interferon, interferon alfa, interferon alfa-2a, alfa-2b, alfa-n3, interleukin-2, iobenguane, iobenguane iobenguane, irinotecan (camptosar), isotretinoin (accutane), ketoconazole, 4-(bis(2-chloroethyl)amino)-L-phenylalanine, L-serine diazoacetate, lentinan, leucovorin, leuprolide acetate (LHRH-analog), levamisole (ergamisol), lomustine (CCNU; cee-NU), mannomustine, maytansine, mechlorethamine, mechlorethamine HCl (nitrogen mustard), medroxyprogesterone acetate (provera, depo provera), megestrol acetate (menace), melengestrol acetate, melphalan (alkeran), menogaril, mercaptopurin, mercaptopurine (purinethol), mercaptopurine anhydrous, MESNA, mesna (mesne), methanesulfonic acid, ethyl ester, methotrexate (mtx; methotrexate), methyl-ccnu, mimosine, misonidazole, mithramycin, mitoantrone, mitobronitol, mitoguazone, mitolactol, mitomycin (mutamycin), mitomycin C, mitotane (o,p'-DDD; lysodren), mitoxantrone, mitoxantrone HCl (novantrone), mopidamol, N,N-bis(2-chloroethyl)tetrahydro-2H-1,3,2-oxazaphosphorin-2-amine-2-oxide, N-(1-methylethyl)-4-((2-methylhydrazino)methyl)benzamide, N-methyl-bis(2-chloroethyl)amine, nicardipine, nilutamide (nilandron), nimustine, nitracrine, nitrogen mustard, nocodazole, nogalamycin, octreotide (sandostatin), pacilataxel (taxon), paclitaxel, pactamycin, pegaspargase (PEGx-1), pentostatin (2'-deoxycoformycin), peplomycin, peptichemio, photophoresis, picamycin (mithracin), picibanil, pipobroman, plicamycin, podofilox, podophyllotoxin, porfiromycin, prednisone, procarbazine, procarbazine HCl (matulane), prospidium, puromycin, puromycin aminonucleoside, PUVA (psoralen+ultraviolet a), pyran copolymer, rapamycin, s-azacytidine, 2,4,6-tris(1-aziridinyl)-s-triazine, semustine, showdomycin, sirolimus, streptozocin (zanosar), suramin, tamoxifen citrate (nolvadex), taxon, tegafur, teniposide (VM-26; vumon), tenuazonic acid, TEPA, testolactone, thio-tepa, thioguanine, thiotepa (thioplex), tilorone, topotecan, tretinoin (vesanoid), triaziquone, trichodermin, triethylene glycol diglycidyl ether, triethylenemelamine, triethylenephosphoramide, triethylenethiophosphoramide, trimetrexate (neutrexin), tris(1-aziridinyl)phosphine oxide, tris(1-aziridinyl)phosphine sulfide, tris(aziridinyl)-p-benzoquinone, tris(aziridinyl)phosphine sulfide, uracil mustard, vidarabine, vidarabine phosphate, vinblastine, vinblastine sulfate (velban), vincristine sulfate (oncovin), vindesine, vinorelbine, vinorelbine tartrate (navelbine), (1)-mimosine, 1-(2-chloroethyl)-3-(4-methylcyclohexyl)-1-nitrosourea, (8S-cis)-10-((3-amino-2,3,6-trideoxy-alpha-L-lyxo-hexopyranosyl)oxy)-7,8,9,10-tetrahydro-6,8,11-trihydroxy-8-(hydroxyacetyl)-1-methoxy-5,12 naphthacenedione, 131-meta-iodobenzyl guanidine (I-131 MIBG), 5-(3,3-dimethyl-1-triazenyl)-1H-imidazole-4-carboxamide, 5-(bis(2-chloroethyl)amino)-2,4(1H,3H)-pyrimidinedione, 2,4,6-tris(1-aziridinyl)-s-thiazine, 2,3,5-tris(1-aziridinyl)-2,5-cyclohexadiene-1,4-dione, 2-chloro-N-(2-chloroethyl)-N-methylethanamine, N,N-bis(2-chloroethyl)tetrahydro-2H-1,3,2-oxazaphosphorin-2-amine-2-oxide, 3-deazauridine, 3-iodobenzylguanidine, 5,12-naphthacenedione, 5-azacytidine, 5-fluorouracil, (1aS,8S,8aR,8bS)-6-amino-8-(((aminocarbonyl)oxy)methyl)-1,1a, 2,8,8a,8b-hexahydro-8a-methoxy-5-methylazirino(2',3': 3,4)pyrrolo(1,2-a)indole-4,7-dione, 6-azauridine, 6-mercaptopurine, 8-azaguanine, and combinations thereof.

Preferred therapeutic agents and combinations that may be administered as an apoptosis-inducing therapy include Doxorubicin and Doxetaxel, Topotecan, Paclitaxel, Carboplatin and Taxol, Taxol, Cisplatin and Radiation, 5-fluorouracil (5-FU), 5-FU and Radiation, Toxotere, Fludarabine, Ara C, Etoposide, Vincristine, and Vinblastin.

Exemplary chemotherapeutic agents also include doxetaxel (TOXOTERE) and to potecan (HYCAMTIN).

Chemotherapeutic agents that may be administered in the method include, but are not limited to, antibiotic derivatives (e.g., doxorubicin, bleomycin, daunorubicin, and dactinomycin); antiestrogens (e.g., tamoxifen); antimetabolites (e.g., fluorouracil, 5-FU, methotrexate, floxuridine, interferon alpha-2b, glutamic acid, plicamycin, mercaptopurine, and 6-thioguanine); cytotoxic agents (e.g., carmustine, BCNU, lomustine, CCNU, cytosine arabinoside, cyclophosphamide, estramustine, hydroxyurea, procarbazine, mitomycin, busulfan, cis-platin, and vincristine sulfate); hormones (e.g., medroxyprogesterone, estramustine phosphate sodium, ethinyl estradiol, estradiol, megestrol acetate, methyltestosterone, diethylstilbestrol diphosphate, chlorotrianisene, and testolactone); nitrogen mustard derivatives (e.g., mephalen, chorambucil, mechlorethamine (nitrogen mustard) and thiotepa); steroids and combinations (e.g., bethamethasone sodium phosphate); and others (e.g., dicarbazine, asparaginase, mitotane, vincristine sulfate, vinblastine sulfate, and etoposide).

In a specific embodiment, antibodies are administered in combination with CHOP (cyclophosphamide, doxorubicin, vincristine, and prednisone) or any combination of the components of CHOP. In another embodiment, antibodies are administered in combination with Rituximab. In a further embodiment, antibodies are administered with Rituxmab arid CHOP, or Rituxmab and any combination of the components of CHOP.

**Table 4: Commonly Used Chemotherapy Drugs for Major Cancer Indications**

| |
|---|
| 1. Breast cancer: Adjuvant therapy (systemic therapy as an adjunct to or in addition to surgery). Doxorubicin (Adriamycin), cyclophosphamide, and taxanes [paclitaxel (Taxol) and docetaxel (Taxotere)]. These three drugs are also active in metastatic breast cancer but if the patient has already received them as adjuvant therapy the commonly used drugs are capecitabine (Xeloda), gemcitabine (Gemzar), vinorelbine (Navelbine). Commonly prescribed hormonal agents for bone metastases of hormone receptor positive tumors are: tamoxifen and aromatase inhibitors (Arimidex, Femara, Aromasin). |
| 2. Colon cancer: 5-FU plus leucovorin, irinotecan (camptosar), oxaliplatin, and capecitabine. |
| 3. Lung cancer: Cisplatin, carboplatin, paclitaxel, docetaxel, gemcitabine, vinorelbine. |
| 4. Prostate cancer: Docetaxel, estramustine, mitoxantrone (Novantrone), and prednisone. |
| 5. Non-Hodgkin's Lymphoma: Cyclophosphamide, doxorubicin, vincristine (Oncovin), and prednisone. |

Therapeutic radiation includes, for example, fractionated radiotherapy, nonfractionated radiotherapy and hyperfractionated radiotherapy, and combination radiation and chemotherapy. Types of radiation also include ionizing (gamma) radiation, particle radiation, low energy transmission (LET), high energy transmission (HET), ultraviolet radiation, infrared radiation, visible light, and photosensitizing radiation. As used herein, chemotherapy includes treatment with a single chemotherapeutic agent or with a combination of agents. In a subject in need of treatment, chemotherapy may be combined with surgical treatment or radiation therapy, or with other antineoplastic treatment modalities.

### Other Therapeutics

In a further embodiment, the antibodies are administered in combination with an antiviral agent. Antiviral agents that may be administered with the antibodies include, but are not limited to, acyclovir, ribavirin, amantadine, and remantidine.

Antibodies may also be administered with antiemetics such as 2-(ethylthio)-10-(3-(4-methyl-1-piperazinyl) propyl)-10H-phenothiazine (ethylthioperazine), 1-(p-chloro-alpha-phenylbenzyl)-4-(m-methylbenzyl)-piperazine (meclozine, meclizine), etc., and combinations thereof Polynucleotides and polypeptides may also be administered with other therapeutic agents, and combinations thereof, disclosed herein or known in the art.

In a further embodiment, the antibodies are administered in combination with an antibiotic agent. Antibiotic agents that may be administered with the antibodies include, but are not limited to, amoxicillin, beta-lactamases, aminoglycosides, beta-lactam (glycopeptide), beta-lactamases, Clindamycin, chloramphenicol, cephalosporins, ciprofloxacin, ciprofloxacin, erythromycin, fluoroquinolones, macrolides, metronidazole, penicillins, quinolones, rifampin, streptomycin, sulfonamide, tetracyclines, trimethoprim, trimethoprim-sulfamthoxazole, and vancomycin.

Conventional nonspecific immunosuppressive agents, that may be administered in combination with the antibodies of the invention include, but are not limited to, steroids, cyclosporine, cyclosporine analogs, cyclophosphamide methylprednisone, prednisone, azathioprine, FK-506, 15-deoxyspergualin, and other immunosuppressive agents that act by suppressing the function of responding T cells.

In specific embodiments, antibodies are administered in combination with immunosuppressants. Immunosuppressants preparations that may be administered with the antibodies include, but are not limited to, ORTHOCLONE.TM. (OKT3), SANDIMMUNE.TM./NEORAL.TM./SANGDYA.TM. (cyclosporin), PROGRAF.TM. (tacrolimus), CELLCEPT.TM. (mycophenolate), Azathioprine, glucorticosteroids, and RAPAMUNE.TM. (sirolimus). In a specific embodiment, immunosuppressants may be used to prevent rejection of organ or bone marrow transplantation.

In an additional embodiment, antibodies are administered alone or in combination with one or more intravenous immune globulin preparations. Intravenous immune globulin preparations that may be administered with the antibodies of the invention include, but not limited to, GAMMAR.TM., IVEEGAM.TM., SANDOGLOBULIN.TM., GAMMAGARD S/D.TM., and GAMIMUNE.TM.. In a specific embodiment, antibodies are administered in combination with intravenous immune globulin preparations in transplantation therapy (e.g., bone marrow transplant).

In an additional embodiment, the antibodies are administered alone or in combination with an anti-inflammatory agent Anti-inflammatory agents that may be administered with the antibodies include, but are not limited to, glucocorticoids and the nonsteroidal anti-inflammatories, aminoarylcarboxylic acid derivatives, arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole, and tenidap.

In another embodiment, antibodies are administered in combination with a chemotherapeutic agent. Chemotherapeutic agents that may be administered with the antibodies i include, but are not limited to, antibiotic derivatives (e.g., doxorubicin, bleomycin, daunorubicin, and actinomycin); antiestrogens (e.g., tamoxifen); antimetabolites (e.g., fluorouracil, 5-FU, methotrexate, floxuridine, interferon alpha-2b, glutamic acid, plicamycin, mercaptopurine, and 6-thioguanine); cytotoxic agents (e.g., carmustine, BCNU, lomustine, CCNU, cytosine arabinoside, cyclophosphamide, estramustine, hydroxyurea, procarbazine, mitomycin, busulfan, cis-platin, and vincristine sulfate); hormones (e.g., medroxyprogesterone, estramustine phosphate sodium, ethinyl estradiol, estradiol, megestrol acetate, methyltestosterone, diethylstilbestrol diphosphate, chlorotrianisene, and testolactone); nitrogen mustard derivatives (e.g., mephalen, chorambucil, mechlorethamine (nitrogen mustard) and thiotepa); steroids and combinations (e.g., bethamethasone sodium phosphate); and others (e.g., dicarbazine, asparaginase, mitotane, vincristine sulfate, vinblastine sulfate, and etoposide).

In a specific embodiment, antibodies are administered in combination with CHOP (cyclophosphamide, doxorubicin, vincristine, and prednisone) or any combination of the components of CHOP. In another embodiment, antibodies are administered in combination with Rituximab. In a further embodiment, antibodies are administered with Rituxmab arid CHOP, or Rituxmab and any combination of the components of CHOP.

In an additional embodiment, the antibodies are administered in combination with cytokines. Cytokines that may be administered with the antibodies include, but are not limited to, IL2, IL3, IL4, IL5, IL6, IL7, IL10, IL12, IL13, IL15, anti-CD40, CD40L, IFN-gamma and TNF-alpha. In another embodiment, antibodies may be administered with any interleukin, including, but not limited to, IL-lalpha, IL-Ibeta, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, and IL-21.

In an additional embodiment, the antibodies are administered in combination with angiogenic proteins. Angiogenic proteins that may be administered with the antibodies include, but are not limited to, Glioma Derived Growth Factor (GDGF), as disclosed in European Patent Number EP-399816; Platelet Derived Growth Factor-A (PDGF-A), as disclosed in European Patent Number EP-6821 10; Platelet Derived Growth Factor-B (PDGF-B), as disclosed in European Patent Number EP-282317; Placental Growth Factor (PIGF), as disclosed in International Publication Number WO 92/06194; Placental Growth Factor-2 (PIGF-2), as disclosed in Hauser et al., Gorwth Factors, 4:259-268 (1993); Vascular Endothelial Growth Factor (VEGF), as disclosed in International Publication Number WO 90/13649; Vascular Endothelial Growth Factor-A (VEGF-A), as disclosed in European Patent Number EP-506477; Vascular Endothelial Growth Factor-2 (VEGF-2), as disclosed in International Publication Number WO 96/39515; Vascular Endothelial Growth Factor B (VEGF-3); Vascular Endothelial Growth Factor B-186 (VEGF-B186), as disclosed in International Publication Number WO 96/26736; Vascular Endothelial Growth Factor-D (VEGF-D), as disclosed in International Publication Number WO 98/02543; Vascular Endothelial Growth Factor-D (VEGF-D), as disclosed in International Publication Number WO 98/07832; and Vascular Endothelial Growth Factor-E (VEGF-E), as disclosed in German Patent Number DE19639601.

In an additional embodiment, the antibodies are administered in combination with hematopoietic growth factors. Hematopoietic growth factors that may be administered with the antibodies of the invention include, but are not limited to, LEUKINE.TM. (SARGRAMOSTIM.TM.) and NEUPOGEN.TM. (FILGRASTTM.TM.).

In an additional embodiment, the antibodies are administered in combination with Fibroblast Growth Factors. Fibroblast Growth Factors that may be administered with the antibodies of the invention include, but are not limited to, FGF-1, FGF-2, FGF-3, FGF-4, FGF-5, FGF-6, FGF-7, FGF-8, FGF-9, FGF-10, FGF-11, FGF-12, FGF-13, FGF-14, and FGF-15.

### EXAMPLE 1

### C35 EXPOSED ON SURFACE MEMBRANE OF BREAST TUMOR CELLS FOLLOWING RADIATION INDUCED APOPTOSIS

A line of continuously growing breast tumor cells that express the C35 tumor antigen was either irradiated with 300 Gy or left untreated. After continued in vitro culture for several days to allow apoptosis to develop, cells were harvested, washed and stained with 50 ng of 1F2 monoclonal anti-C35 antibody or a mouse IgG antibody control each conjugated to the fluorescent dye Alexa 647. Following 50 minutes incubation at 25C, cells were stained with Annexin V and propidium iodide (PI) using a standard commercial kit (Pharmingen). Cells were analyzed for staining with Annexin V, propidium iodide and Alexa 647 by flow cytometry employing standard protocols.

The results in Figure 1 show that untreated live cells (PI negative), that are not undergoing apoptosis (Annexin V negative), do not express C35 on the surface membrane as evidenced by absence of differential staining with anti-C35 antibody and the isotype control antibody (Figure 1A). Similarly, irradiated tumor cells that remain viable (PI negative) and have not been induced to undergo apoptosis (Annexin V negative) also do not express C35 on the tumor cell surface membrane (Figure 1B). In striking contrast, irradiated tumor cells that are viable (PI negative), but undergoing apoptosis (Annexin V positive), are clearly differentially stained with anti-C35 antibodies as compared to isotype control antibody (Figure 1C).

### EXAMPLE 2

### C35 EXPOSED ON SURFACE MEMBRANE OF BREAST TUMOR CELLS FOLLOWING DRUG INDUCED APOPTOSIS

A line of continuously growing breast tumor cells that express the C35 tumor antigen was either treated with 6ug/ml mitomycin C or left untreated. After continued in vitro culture for 48 hours to allow apoptosis to develop, cells were harvested, washed and stained with 50 ng of 1F2 monoclonal anti-C35 antibody or a mouse IgG antibody control each conjugated to the fluorescent dye Alexa 647. Following 50 minutes incubation at 25C, cells were stained with Annexin V and propidium iodide (PI) using a standard commercial kit (Pharmingen). Cells were analyzed for staining with Annexin V, propidium iodide and Alexa 647 by flow cytometry employing standard protocols.

The results in Figure 2 show that untreated live cells (PI negative), that are not undergoing apoptosis (Annexin V negative), do not express C35 on the surface membrane as evidenced by absence of differential staining with anti-C35 antibody and the isotype control antibody (Figure 2A). Similarly, mitomycin, C treated tumor cells that remain viable (PI negative) and have not been induced to undergo apoptosis (Annexin V negative) also do not express C35 on the tumor cell surface membrane (Figure 2B). In striking contrast, mitomycin C treated tumor cells that are viable (PI negative), but undergoing apoptosis (Annexin V positive), are clearly differentially stained with anti-C35 antibodies as compared to isotype control antibody (Figure 2C).

### EXAMPLE 3

### EXPRESSION OF AN ANTIBODY IN MAMMALIAN CELLS

The polypeptide can be expressed in a mammalian cell. A typical mammalian expression vector contains a promoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors include, for example, vectors such as pSVL and pMSG (Pharmacia, Uppsala, Sweden), pRSVcat (ATCC 37152), pSV2dhfr (ATCC 37146), pBC12MI (ATCC 67109), pCMVSport 2.0, and pCMVSport 3.0. Mammalian host cells that could be used include, human Hela, 293, H9 and Jurkat cells, mouse NTH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as DHFR, gpt, neomycin, hygromycin allows the identification and isolation of the transfected cells.

The transfected gene can also be amplified to express large amounts of the encoded protein. The DHFR (dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide is amplified according to protocols known in the art. If a naturally occurring signal sequence is used to produce the polypeptide of the present invention, the vector does not need a second signal peptide. Alternatively, if a naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Calif.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. E. coli HB101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five .mu.g of the expression plasmid pC6 or pC4 is cotransfected with 0.5 .mu.g of the plasmid pSVneo using lipofectin (Felgner et al., supra). The plasmid pSV2-neo contains a dominant selectable marker, the neo gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 uM, 2 uM, 5 uM, 10 mM, 20 mM). The same procedure is repeated until clones are obtained which grow at a concentration of 100-200 uM. Expression of the desired gene product is analyzed, for instance, by SDS-PAGE and Western blot or by reversed phase HPLC analysis.

### EXAMPLE 4

### RADIOLABELED C35-SPECIFIC ANTIBODIES CONCENTRATE IN NECROTIC REGIONS OF VIABLE TUMORS EXPRESSING C35.

BALB/c mice were engrafted on opposite flanks with syngeneic non-small cell lung cancer derived Line 1 tumor cells that either had or had not been transfected with human C35. C35 protein expression was confirmed by immunohistochemical staining with anti-C35 antibodies. After 14 days in vivo growth, animals received intravenous injection of ¹²⁵I-labeled anti-C35 antibody. Animals were sacrificed 120 hrs after injection of radiolabeled antibodies and the concentration of anti-C35 antibodies in C35-positive and C35-negative tumors was determined by exposure of a tumor section to film. As shown in Figure 4, radiolabeled anti-C35 antibodies concentrated only in the C35-positive and not the C35-negative tumors. Comparison of the distribution of label and an H&E stain for intact cells within the tumors, confirmed that under these conditions the labeled anti-C35 antibodies concentrated specifically in the necrotic regions of the C35-positive tumor.

### EXAMPLE 5

### PROTOCOL FOR ADMINISTRATION OF DOSIMETRIC AND THERAPEUTIC RADIOLABELED ANTIBODY

The radiolabeled antibody (or antibody fragment) compositions, which include both the dosimetric radiolabeled antibody and the therapeutic radiolabeled antibody, are administered intravenously or intraarterially in the form of an injection. The injectable radiolabeled antibody compositions will be infused into a vein or artery over the course of 5 minutes to about 60 minutes, preferably from 15 minutes to 30 minutes. Where the tumor is supplied by a known artery, intraarterial administration is preferred for the therapeutic radiolabeled antibody compositions. Both the dosimetric radiolabeled antibody and the therapeutic radiolabeled antibody will be administered as sterile aqueous solutions typically in physiologic phosphate-buffered saline or other vehicle suitable for parenteral injection. The initial dosimetric radiolabeled antibody dose will be approximately 5-100mg of antibody which will deliver approximately 5-50mCi radiation. Approximately 5-10 days following the dosimetric dose, the therapeutic radiolabeled antibody will be administered at a dose of approximately 10-500 mg which will deliver as much as 300 mCi radiation for each therapeutic dose. This dosimetric/therapeutic regimen may be repeated. See also, US 5,057,313 and US 5,120,525.

### EXAMPLE 6

### CLONING OF ANTI-C35 MOUSE AND HUMAN ANTIBODY VARIABLE REGION GENES INTO DEPOSITED TOPO CLONES

The immunoglobulin heavy and light chain variable regions were cloned into the TOPO vector (Invitrogen) by PCR amplification of the V region and TA cloning into the TOPO vector. This ligation system does not require restriction enzyme digestion (although the TOPO vector does incorporate EcoRI sites to allow subsequent excision of inserts). TA cloning takes advantage of naturally added 3' A overhangs in the PCR amplification product of Taq polymerase which can then pair with 5' T overhangs in the linearized vector provided in the TOPO cloning kit (Invitrogen).

To PCR amplify variable region genes for insertion into TOPO, we employed a downstream primer complementary to the 5' end of the constant region sequence (different for heavy and light chains and for mouse and human) and a known fixed primer sequence added at the 5' end of the variable region by 5' RACE using the Invitrogen GeneRacer kit. These methods are well known to those skilled in the art.

### EXAMPLE 7

### CLONING VARIABLE GENES FROM DEPOSITED TOPO CLONES INTO pCMV EXPRESSION CONSTRUCTS

### Generation of pCMV expression constructs

The construction of vaccinia transfer plasmids - pVHE, pVKE and pVLE - has been described in a previous patent application (US 2002 0123057 A1, "In vitro Methods of Producing and Identifying Immunoglobulin Molecules in Eukaryotic Cells", published 2002-09-05). To generate the mammalian expression vectors to express the immunoglobulin heavy and light chains, the expression cassettes, from NotI to SalI, were excised from these vaccinia transfer plasmids and cloned into the pCMV-Script vector (whose XhoI site in the vector multiple cloning site was destroyed by fill-in and blunt end ligation), resulting in the generation of pCMV-VH, pCMV-VK and pCMV-VL vectors. These expression cassettes contain the signal peptide, cloning sites for the V genes and the constant regions from the membrane-bound µ heavy chain and the κ light chain genes.

In pCMV-VH, the cassette contains the signal peptide from amino acid position -19 relative to the start codon [aa(-19)] to aa(-3), followed by aa(109 to 113) of the VH genes and the whole heavy chain constant region. The selected VH genes, from aa(-4) to aa(110) can be cloned into pCMV-VH at BssHII [aa(-4 to -3)] and BstEII [aa(109-110)] sites.

In pCMV-VK (kappa), the cassette contains the signal peptide from aa(-19) to aa(-2), followed by aa(104 to 107) of the VK genes and the whole kappa chain constant region. The selected VK genes, from aa(-3) to aa(105) can be cloned into pCMV-VK at ApaLI [aa(-3 to -2)] and XhoI [aa(104-105)] sites.

In pCMV-VL (lambda), the cassette contains the signal peptide from aa(-19) to aa(-2), followed by aa(103 to 107) of the VL genes and the whole kappa chain constant region. The selected VL genes, from aa(-3) to aa(104) can be cloned into pCMV-VL at ApaLI [aa(-3 to -2)] and HindIII [aa(103-104)] sites. The resulting lambda light chain will exhibit the VλCκ chimeric structure.

To express the selected antibodies as secreted human IgG1, the constant region of IgG1 was cloned from B cells or bone marrow cells by RT-PCR. The primer set used was:
5' forward primer: 5'-ATTAGGATCCGGTCACCGTCTCCTCAGCC-3' (SEQ ID NO:15)
3' reverse primer: 5'-ATTAGTCGACTCATTTACCCGGAGACAGGGA-3' (SEQ ID NO:16)

The resulting PCR product exhibits the following structure: BamHI-BstEII(aa109-110)-(aa111-113)-Cγ₁-TGA-SalI. The PCR product was subcloned into pBluescriptII/KS at BamHI and SalI sites to carry out site directed mutagenesis employing standard protocols to remove the internal BstEII located at the CH1 region via silent mutation. The resulting Cγ₁ was then subcloned into pCMV-VH at BstEII and SalI to generate pCMV-Cγ₁ to direct the expression of secreted IgG1 heavy chain, once a VH gene is subcloned into this vector at BssHII/BstEII.

The sequence of IgG1-secreted, human gamma1 heavy chain leader and constant region cassette for insertion of V genes follows.
Underline = restriction sites
**Bold** = Constant region
***Bold*/*****italics** =* Signal peptide

The sequence of human light chain leader and kappa constant region cassette for insertion of Vκ genes follows.

The sequence of human light chain leader and kappa constant region cassette for insertion of Vλ genes follows.

To construct vectors that express secreted human antibodies of other isotypes, including secreted forms of IgG2, IgG3, IgG4, IgA, IgD, IgE and IgM, the same approach can be taken to clone the respective constant regions, to mutagenize any internal BstEII site, and to substitute the Cγ₁ with the constant regions of other isotypes between the BstEII and SalI sites in the pCMV-Cγ₁ vector.

To clone the constant regions of other isotypes, the following primer pairs were used:
IgG2-F: 5'-ATTAGGATCCGGTCACCGTCTCCTCAGCC-3' (SEQ ID NO:20)
IgG2-R: 5'-ATTAGTCGACTCATTTACCCGGAGACAGGGA-3' (SEQ ID NO:21)
IgG3-F: 5'-ATTAGGATCCGGTCACCGTCTCCTCAGCT-3' (SEQ ID NO:22)
IgG3-R: 5'-ATTAGTCGACTCATTTACCCGGAGACAGGGA-3' (SEQ ID NO:23)
IgG4-F: 5'-ATTAGGATCCGGTCACCGTCTCCTCAGCT-3' (SEQ ID NO:24)
IgG4-R: 5'-ATTAGTCGACTCATTTACCCAGAGACAGGGA-3' (SEQ ID NO:25)
IgAl-F: 5'-ATTAGGATCCGGTCACCGTCTCCTCAGCAT-3' (SEQ ID NO:26)
IgAl-R: 5'-ATTAGTCGACTCAGTAGCAGGTGCCGTCCAC-3' (SEQ ID NO:27)
IgA2-F: 5'-ATTAGGATCCGGTCACCGTCTCCTCAGCAT-3' (SEQ ID NO:28)
IgA2-R: 5'-ATTAGTCGACTCAGTAGCAGGTGCCGTCCAC-3' (SEQ ID NO:29)
IgD-F: 5'-ATTAGGATCCGGTCACCGTCTCCTCAGCAC-3' (SEQ ID NO:30)
IgD-R: 5'-ATTAGTCGACTCATTTCATGGGGCCATGGTC-3' (SEQ ID NO:31)
IgE-F: 5'-ATTAGGATCCGGTCACCGTCTCCTCAGCC-3' (SEQ ID NO:32)
IgE-R: 5'-ATTAGTCGACTCATTTACCGGGATTTACAGA-3' (SEQ ID NO:33)
IgM-F: 5'-ATTAGGATCCGGTCACCGTCTCCTCAGGG-3' (SEQ ID NO:34)
IgM-R: 5'-ATTAGTCGACTCAGTAGCAGGTGCCAGCTGT-3' (SEQ ID NO:35)

Note that, because of the high degree of sequence conservation, primers used are the same between IgG1 and IgG2, between IgG3 and IgG4, and between IgAl and IgA2.

### Cloning Variable genes from Topo clones into pCMV expression constructs

Step 1: Generation of V-gene fragments.
A. Human v-genes
   MMH1
   1. Digest MMH1 plasmid DNA (clone H0009) with BssHII (GCGCGC(SEQ ID NO:36)) and BsteII (GGTCACC (SEQ ID NO:37)) using standard protocols.
   2. Resolve DNA on agarose gel using standard protocols.
   3. Excise 357 bp fragment from gel and isolate DNA using standard protocols.
   MMK1
   1. Digest MMK1 plasmid DNA (clone L0010) with ApaL1 (GTGCAC(SEQ ID NO:38)) and Xho1 (CTCGAG (SEQ ID NO:39)) using standard protocols.
   2. Resolve DNA on agarose gel using standard protocols.
   3. Excise 343 bp fragment from gel and isolate DNA using standard protocols.
B. Mouse hybridoma v-genes:
   1F2 VK
   1. The mouse hybridoma v-gene must be PCR amplified from the ATCC deposited clone 1F2K using the following primers. This is necessary to create a chimeric antibody of the mouse v-gene with human constant region in the human kappa light chain constant region expression cassette.
1F2VK forward primer:
5' - tatccgtgcactccCAAATTGTTCTCACCCAGTCTCCAG - 3' (SEQ ID NO:40)
1F2VK reverse primer:
5' - atattctcgAGCTTGGTCCCCCCTCCGAA - 3' (SEQ ID NO:41) (Lowercase = non-homologous to mouse 1F2 VK sequence, includes restriction site. CAPITALS= homologous to mouse 1F2 VK sequence)
   2. Digest 331 bp PCR product with ApaL1 (GTGCAC (SEQ ID NO:42)) and Xho1 (CTCGAG (SEQ ID NO:43)) using standard protocols.
   3. Resolve DNA on agarose gel using standard protocols.
   4. Excise 315 bp digested fragment from gel and isolate DNA using standard protocols.
1F2 VH
1. The mouse hybridoma v-gene must be PCR amplified from the ATCC deposited clone 1F2G using the following primers. This is necessary to create a chimeric antibody of the mouse v-gene with human constant region in the human heavy chain constant region expression cassette.
1F2VH forward primer:
5' - tataagcgcgcactccGATGTACAGCTTCAGGAGTCAGGAC (SEQ ID NO:44)
1F2VH reverse primer:
5'-atattgGTGACCAGAGTCCCTTGGCCCC-3' (SEQ ID NO:45) (Lowercase = non-homologous, contains restriction sites. CAPITALS= homologous)
2. Digest 360 bp PCR product with BssHII (GCGCGC (SEQ ID NO:36)) and BsteII (GGTCACC (SEQ ID NO:37)) using standard protocols.
3. Resolve DNA on agarose gel using standard protocols.
4. Excise gel slice containing 343 bp digested DNA fragment and isolate DNA using standard protocols.
1B3VK
1. The mouse hybridoma v-gene must be pcr amplified from the deposited clone 1B3K using the following primers. This is necessary to create a chimeric antibody of the mouse v-gene with human constant region in the human kappa light chain constant region expression cassette.
1B3VK forward primer:
5' - tatccgtgcactccGATGTCCAGATAACCCAGTCTCCATC - 3' (SEQ ID NO:46)
   1B3VK reverse primer:
5' -atattctcgAGCTTGGTCCCAGCACCGAA - 3' (SEQ ID NO:47)
   (Lowercase = non-homologous, contains restriction sites. CAPITALS= homologous)
2. Digest 334 bp PCR product with ApaL1 (GTGCAC (SEQ ID NO:42)) and Xho1 (CTCGAG (SEQ ID NO:43)) using standard protocols.
3. Resolve DNA on agarose gel using standard protocols.
4. Excise gel slice containing digested 322 bp DNA fragment and isolate DNA using standard protocols
1B3VH
1. The mouse hybridoma v-gene must be per amplified from the deposited clone 1B3G using the following primers. This is necessary to create a chimeric antibody of the mouse v-gene with human constant region in the human heavy chain constant region expression cassette.
   1B3VH forward primer:
   5'- tataagcgcgcactccGAGGTGCAGCTTCAGGAGTCAGGAC - 3' (SEQ ID NO:48)
   1B3VH reverse primer:
   5' -atattGGTGACCGTGGTCCCAGCG -3' (SEQ ID NO:49) (Lowercase = non-homologous, contains restriction sites. CAPITALS= homologous
2. Digest 378 bp PCR product with BssHII (GCGCGC (SEQ ID NO:36)) and BsteII (GGTCACC (SEQ ID NO:37)) using standard protocols.
3. Resolve DNA on agarose gel using standard protocols.
4. Excise gel slice containing 366 bp digested DNA fragment and isolate DNA using standard protocols.

Step 2: Assembly of expression constructs
1. Digest pCMV-VH and pCMV-VK expression vectors with the appropriate enzymes using standard protocols:
   a. pCMV-VH - BsteII and BssHII
   b. pCMV-VK-ApaL1 and Xho I
2. Resolve DNA on agarose gel and excise linearized vector using standard protocols.
3. Isolate DNA from gel slice using standard protocols.
4. Ligate light chain v-genes into pCMV-VK and heavy chain v-genes into pCMV-VH using standard protocols.
5. Transform ligated DNA into competent cells and isolate plasmid DNA using standard protocols.

### EXAMPLE 8

### SEQUENCES OF IMMUNOGLOBULIN CONSTANT REGIONS

The following genes and encoded amino acids sequences may be used to prepare humanized antibodies, human variant antibodies, chimeric antibodies, and fragments thereof.

Homo sapiens G2 gene for immunoglobulin constant region (IgG2 (n-) allotype) (GenBank No. Z49802) (SEQ ID NO:50)

H. sapiens G2 gene for immunoglobulin constant region (IgG2 (n+) allotype) (GenBank No. Z49801) (SEQ ID NO:51)

Homo sapiens CH gene encoding immunoglobulin, constant region, heavy chain, alpha-2 subunit (GenBank No. AJ012264) (SEQ ID NO:52)

Homo sapiens constant region, heavy chain, alpha-2 subunit (GenBank No. CAA09968.1) (SEQ ID NO:53)

Homo sapiens partial mRNA for immunoglobulin heavy chain constant region alpha 1 (IGHA1 gene) (GenBank No. AJ294729) (SEQ ID NO:54)

Imunoglobulin heavy chain constant region alpha 1 (GenBank No. CAC20453.1) (SEQ ID NO:55)

Additional constant region sequences (human IgM1, IgM2, IgD1, IgA1, IgG1, IgG3, IgE1, IgE2, kappa, and lamda; mouse IgM1, kappa, and lambda; rabbit IgM1, kappa, and lambda; and dog IgM1) may be found at pages 296-300 of FUNDAMENTAL IMMUNOLOGY (3d ed.), William E. Paul (ed.), Raven Press, New York, NY (1993). Many other constant region sequences (polynucleotide and amino acid) are know in the art and may be used.

### EXAMPLE 9

### COMBINATION RADIOIMMUNOTHERAPY AND CHEMOTHERAPY

The combination of chemotherapy and anti-C35 radioimmunotherapy was shown to be more effective at reducing tumor volume than either therapy alone. In a first experiment, the effect of combination radioimmunotherapy with ¹³¹I labeled 1B3 anti-C35 monoclonal antibody and chemotherapy with 5-FluoroUracil (FU) at 150mg/kg, together with Leucovorin (LV) at 100mg/kg was tested in Swiss nude mice grafted with Colau.C35 tumor cells. Colau.C35 are a C35 antigen positive clone of Colau cells that were tissue culture adapted from a human colon carcinoma and transduced with a C35 retroviral recombinant.

Chemotherapy was initiated on day 11 following tumor graft and 300µCi of ¹³¹I-labeled 1B3 anti-C35 monoclonal antibody was administered on day 14. Tumor growth was followed for up to 8 weeks.

The results in Figure 5 show inhibition of tumor growth in the group that received combination radioimmunotherapy and chemotherapy in comparison to the group that received chemotherapy alone or chemotherapy and non-radiolabeled ("cold") 1B3 anti-C35 antibody. Standard parameters of growth inhibition were calculated for the group receiving combination radioimmunotherapy and chemotherapy in comparison to the untreated control group.

As shown in Figure 5, Tumor Doubling Delay (TDD) equals 3.8 (at 400 mm³ tumor volume) where TDD equals (Treated - Control{in days to the specified volume})/TVDT and TVDT = Tumor Volume Doubling Time of Control {during exponential growth phase}. Log Cell Kill (LCK) is defined as TDD/3.3 = 1.15, which meets the accepted standard for an effective tumor therapy (*See*, *e.g.*, Skipper HE et al., Cancer Chemotherapy Rep. 35:1-111 (1964); Coldman AJ and Goldie JH, Mathematical Biosciences 65:291-307 (1983); and Norton L and Simon R, Cancer Treat. Rep. 61:1307-1317 (1977)).

In a second experiment, the effect of combination radioimmunotherapy with ¹³¹I labeled 1B3 anti-C35 monoclonal antibody and chemotherapy with cisplatin at 2 mg/kg on day 15 and 18 was tested in Swiss nude mice grafted with Colau.C35 tumor cells. Cispaltin was administered on days 15 and 18 following tumor graft. 300µCi of ¹³¹I-labeled 1B3 anti-C35 monoclonal antibody was administered on day 21. Tumor growth was followed for up to 10 weeks.

In the same experiment, separate groups of Swiss nude mice grafted with Colau.C35 tumor cells were treated with either 5-FluoroUracil (5FU) at 180mg/kg, together with Leucovorin (LV) at 120mg/kg or this same chemotherapy regimen administered on day 18 followed by 300µCi of ¹³¹I-labeled 1B3 anti-C35 monoclonal antibody administered on day 21.

The results in Figure 6 show some inhibition of Colau.C35 tumor growth in the group that received chemotherapy alone (either cisplatin or 5FU/LV), greater inhibition in the group that received ¹³¹I-labeled 1B3 anti-C35 monoclonal antibody, and even greater inhibition of tumor growth in the group that received combination chemotherapy and ¹³¹I-labeled 1B3 anti-C35 monoclonal antibody. *See* Table 5, below.

**Table 5: Comparison of Effects of Therapeutic Modalities on Tumor Volume in Figure 6**

| | **5FU/LV alone** | **Cisplatin alone** | **RIT: ¹³¹I-1B3** | **5FU/LV + RIT** | **Cisplatin + RIT** |
|---|---|---|---|---|---|
| T-C (days) | 5 | 5 | 29 | 37 | 40 |
| TDD | 0.66 | 0.66 | 3.84 | 4.89 | 5.29 |
| LCK | 0.20 | 0.20 | 1.16 | 1.48 | 1.60 |

| | | | | | |
|---|---|---|---|---|---|
| RIT = radioimmunotherapy T-C = difference in time for treated (T) and control (C) tumors to reach a given volume (1200 mm³) TDD = Tumor doubling delay = T-C/tumor volume doubling time of untreated LCK = Log Cell Kill = TBD/3.32 | | | | | |

For convenience of use in these experimental models, a tumor xenograft was selected that grew relatively rapidly and had to be transduced with recombinant C35. However, the success of combination therapy was not due to abnormally high levels of C35 expression in the transduced tumor. Figure 7 shows that C35 expression was very similar in tumors such as 21MT1 that naturally express C35, and in C35-transduced tumors such as Colau.C35 and MDA231.C35. Cells were stained with Alexa-647 conjugated anti-C35 MAb 1F2 or isotype control. "MFI X" is the ratio of the mean fluorescence intensity of 1F2/mean fluorescence intensity of isotype control. H16N2, derived from normal breast epithelium, and MDAMB231, a breast tumor, and Colau, a colon tumor, express low basal levels of C35. 21MT1, derived from breast carcinoma, naturally expresses high levels of C35. Colau and MDA231 were transduced with empty vector (null) or human C35 recombinant vector. All tumors were grown in vivo, tumors were excised, dissociated and stained.

### EXAMPLE 10

### DETERMINATION OF MAXIMUM TOLERATED DOSE (MTD) OF A CHEMOTHERAPEUTIC AGENT

Because of concerns regarding cumulative dose-limiting bone marrow toxicity when combining chemotherapy and radioimmunotherapy, it is necessary to determine the Maximum Tolerated Dose (MTD) of combination therapy and, where toxicity of the two therapeutic agents is additive, adopt strategies that will permit administration of both toxic agents. MTD is established by regulatory criteria related to the time required for platelet and white cell recovery in peripheral circulation. Such standards are familiar to those skilled in the art. In the case of murine models, an often employed surrogate definition of MTD is the maximum dose that results in an average of less than 20 % weight loss or less than 10% mortality. Currently established MTD for the most common chemotherapeutic agents employed in standard clinical protocols or in animal models are shown in Tables 6 to 9, below.

**Table 6: Representative Chemotherapy Protocols in Xenograft models (nude mice)**

| **Cytotoxic Drug** | **Maximum Tolerated Dose** | **Dose + RIT** | **Schedule** |
|---|---|---|---|
| Fluorouracil/leucovor in | 180/120 mg/kg ¹ | 150/100 mg/kg | bolus, i.v. |
| Oxaliplatin | 5 mg/kg ³ | TBD | i.p. every day for 5 cycles |
| Cisplatin | 4 mg/kg ^{4,5} | 2 mg/kg | i.v. every 3 days for 2 cycles |
| Irinotecan | 15 mg/kg ³ | TBD | i.p. every day for 5 cycles |
| Taxol | No toxicity ² | 30 mg/kg ^{4,6} | i.v. every 7 days for 2-3 cycles |
| Cyclophosphamide | No toxicity ² | 175 mg/kg ^{4,7} | i.v. every 7 days for 2 cycles |
| Adriamycin | 10 mg/kg ¹ | 8 mg/kg | i.v. every 4 days for 3 cycles |
| Gemcitabine | No toxicity ⁵ | 120 mg/kg 5 | i.p. every 3 days for 4 cycles |
| Vinorelbine | 20 mg/kg ⁴ | TBD | bolus, i.v. |
| External Beam Irradiation | No toxicity ² | 20 Gy | delivered locally |

| | | | |
|---|---|---|---|
| Notes TBD: To be determined 1. MTD confirmed by present inventors. Maximum Tolerated Dose is defmed as ≥ 20% average weight loss and/or > 10% lethality. 2. Non-toxic dose at indicated schedule. 3. Fichtner I et al. Anticancer drug response and expression of molecular markers in early-passage xenotransplanted colon carcinomas. Eur J Can 2004. 40: 298-307. 4. Villena-Heinsen C et al. Human ovarian cancer xenografts in nude mice: chemotherapy trials with paclitaxel, cisplatin, vinorelbine, and titanocene dichloride. Anticancer Drugs 1998. 9: 557-563. 5. Higgins B. et al. Antitumor activity of erlotinib (OSI-774, Tarceva) alone or in combination in human non-small cell lung cancer tumor xenograft models. Anti-Cancer Drugs 2004. 15: 503-512. 6. Kraeber-Bodere F. et al. Enhanced Antitumor Activity of Combined pretargeted radioimmunotherapy and Paclitaxel in Medullary Thyroid Cancer Xenograft. Mol Can Ther 2002. 1: 267-274 7. Kraus-Berthier, L et al. Histology and sensitivity to anticancer Drugs of two human non-small cell lung carcinomas implanted in the pleural cavity of nude mice. 2000. Clin Can Res 6:297-304. | | | |

**Table 7: Representative Breast Cancer Chemotherapy Protocols**

| **Regimen** | **Cytotoxic Drug** | **Dosage** | **Schedule** |
|---|---|---|---|
| AC | doxorubicin | 60 mg/m² IV | Repeat every 3 weeks for 4 cycles |
| | cyclophosphamide | 600 mg/m² IV | |
| CAF | cyclophosphamide | 100mg/m²/day PO on days 1-14 | Repeat every 28 days for 6 cycles |
| | doxorubicin | 30 mg/ m² IV on days 1 and 8 | |
| | fluorouracil | 500mg/ m² IV on days 1 and 8 | |
| CMF | cyclophosphamide | 100mg/m²/day PO on days 1-14 | Repeat every 28 days for 6 cycles |
| | methotrexate | 40mg/ m² IV on days 1 and 8 | |
| | fluorouracil | 600mg/ m² IV on days 1 and 8 | |
| | vinorelbine | 30mg/ m² IV on days 1 and 8 | Repeat every 21 days for 6 - 8 cycles |
| | paclitaxel | 175 mg/ m² IV | Repeat every 21 days for 6 cycles |

| | | | |
|---|---|---|---|
| Sources : DataMonitor Pipeline Insight: Breast Cancer June 2004; http:/www.bccancer.bc.ca | | | |

**Table 8: Representative Colon Cancer Therapy Protocols**

| **Regimen** | **Drug** | **Dosage** | **Schedule** |
|---|---|---|---|
| | Leucovorin | 20 mg/m²/day X 5 days (d1-5) IV prior to fluorouracil | Every 28 days X 6 cycles |
| | Fluorouracil | 425 mg/m²/day X 5 days (d1-5) IV | |
| | Irinotecan | 350 20 mg/m² IV | Repeat every 21 days for 2 -6 cycles depending on clinical benefit and toxicity |
| FOLFOX | Oxaliplatin | 100 mg/m²IV | Repeat every 14 days for a maximum of 12 cycles |
| | Leucovorin | 400 mg/m² IV | |
| | Fluorouracil | 400 mg/m² IV bolus after the Leucovorin, THEN | |
| | Fluorouracil | 2400 mg/m² IV over 46 hours | |
| FOLFIRI | Irinotecan | 180 mg/m² IV | Repeat every 14 days for a maximum of 12 cycles |
| | Leucovorin | 400 mg/m² IV | |
| | Fluorouracil | 400 mg/m² IV bolus after the Leucovorin, THEN | |
| | Fluorouracil | 2400 mg/m² IV over 46 hours | |

| | | | |
|---|---|---|---|
| Source: http://www.bccancer.bc.ca | | | |

**Table 9: Representative Lung Cancer Chemotherapy Protocols**

| Drug | Dose | Schedule |
|---|---|---|
| Docetaxel | 75 mg/m² IV | Repeat every 21 days X 6 cycles |
| Docetaxel | 75 mg/m² IV | Repeat every 21 days X 4 cycles |
| Cisplatin | 75 mg/m² IV | |
| Cisplatin | 75 mg/m² IV on Day 1 | Repeat every 21 days X 6 cycles |
| Gemcitabine | 1250 mg/m² IV on Day 1 and Day 8 | |

| | | |
|---|---|---|
| Source: http://www.bccancer.bc.ca | | |

Effective strategies to reduce the combined MTD of treatment with chemotherapy and radioimmunotherapy include: reducing the dose of chemotherapeutic agent administered to a level which does not result in additive toxicity when administered in conjunction with radioimmunotherapy at its MTD. (*see* Example 10A, below); selecting a chemotherapeutic agent that does not contribute additive toxicity when employed in combination with radioimmunotherapy. (*see* Example 10B, below); and reducing the bone marrow toxicity of the radioimmunotherapeutic agent (*see* Example 10C, below).

### A. Reducing the dose of chemotherapeutic agent to reduce toxicity.

2 mg/kg of cisplatin (approximately 50% of MTD) was administered to Swiss nude mice on days 15 and 18 followed 72 hours later by ¹³¹I-labeled 1B3 anti-C35 monoclonal antibody administered at its MTD (300 µCi). As shown in Figure 8, the combined toxicity of cisplatin and radioimmunotherapeutic as determined by weight loss was not significantly different from toxicity of the radioimmunotherapeutic alone administered at its MTD.

### B. Chemotherapeutic agent does not contribute to additive toxicity.

5-FluoroUracil at the MTD of 180 mg/kg together with Leucovorin at 120 mg/kg were administered on day 18 followed by ¹³¹I-labeled 1B3 anti-C35 monoclonal antibody administered at its MTD (300 µCi) on day 21. As shown, in Figure 8, the MTD of the combination of the two toxic agents was not exceeded, even though each agent was administered at its individual MTD.

### C. Reduced bone marrow toxicity of the radioimmunotherapeutic agent.

An alternative strategy is to reduce the bone marrow toxicity of the radioimmunotherapeutic by biochemical modifications that result in accelerated clearance of radiolabeled antibody from peripheral circulation. Appropriate modifications include use of different antibody isotypes such as IgG3, IgA, IgD or IgE as indicated in Table 10 or deletion of the CH2 domain of IgG, which is responsible for its extended serum half life (*See* Mueller BM, RA Reisfeld, and SD Gillies, Proc. Natl. Acad. Sci. USA 87:5702-5705 (1990); Slavin-Chiorini DC, et al., Int. J. Cancer 53:97-103 (1993)).

**Table 10: Serum Half-Life of Human Immunoglobulin Isotypes**

| **Immunoglobulin Isotype** | **Serum Half-Life** |
|---|---|
| IgG1 | 21 days |
| IgG2 | 20 days |
| IgG3 | 7 days |
| IgG4 | 21 days |
| IgM | 10 days |
| IgA | 6 days |
| IgD | 3 days |
| IgE | 2 days |

Other strategies to engineer antibodies and/or antibody fragments, or otherwise modify antibody structure so as to reduce serum half-life are also applicable. Examples of such engineered antibodies and/or antibody fragments include, but are not limited to, domain-deleted antibodies, Fab, F(ab')2, scFv, minibodies, diabodies, triabodies, tetrabodies, etc.

### EXAMPLE 11

### C35 PEPTIDE EPITOPES OF 1B3 AND 1F2 ANTIBODIES

To localize the epitope specificity of 1B3 and 1F2 antibodies, recombinant human C35 (rhC35) synthesized with a 6x His tag in E. coli was digested with Lys-C endoproteinase. This enzyme cuts after lysine (K) residues in the protein sequence. Figure 9 shows the expected peptide fragments following complete digestion of rhC35 with Lys-C. The full sequence of rhC35, including the amino terminal 6x His tag addition is shown. Amino acid positions are numbered relative to the amino terminal methionine (M) of the native human sequence. Note that digestion at the first and third lysine followed by negatively charged residues is inefficient and some longer combination fragments may be generated. Lys-C endoproteinase was added to purified rhC35 at a 50:1 weight ratio and incubated for 18 hours at 37°C in 25mM Tris, pH 8.0. The digest was ethanol precipitated and resolubilized in reducing Tricine sample buffer. After heating 5 minutes at 100°C, samples were separated by electrophoresis on a 16% Tricine gel (Invitrogen). Peptides were transferred to PVDF membranes and the blots depicted in Figure 10 were either stained with Coomassie blue (lanes 1-3 of both left and right panels) to detect all peptides or processed with one of the following: 1 µg/ml of the murine 1F2 anti-C35 antibody followed by alkaline phosphatase conjugated goat anti-mouse antibody (lane 4 of left panel); 1 µg/ml of the 1B3 anti-C35 immunoglobulin variable regions linked to human constant regions (MAb11) followed by alkaline phosphatase conjugated goat anti-human antibody (lane 5 of left panel); or anti-6x His tag mouse antibody (Amersham) followed by alkaline phosphatase conjugated goat anti-mouse antibody (lane 4 and 5 of right panel). BCIP/NBT substrate was added and developed to detect the secondary reagents. Molecular weight markers are indicated in the flanking lanes of both panels.

In Figure 10, the indicated band A migrates at the position of undigested rhC35. Note that band B stains with 1F2 but not MAb11 (1B3) anti-C35 antibody while band C stains with neither 1F2 nor MAb11 (1B3) antibody. Since bands B and C both do stain with anti-6x His tag antibody to the 6x His tag at the amino terminus of rhC35, it may be concluded that both fragments lack C-terminal peptide fragments. In the case of the approximately 15kDa band B, the missing epitope required for staining with MAb11 (1B3) is the 11 amino acid C-terminal C35 peptide ITNSRPPCVIL representing residues 105-115 of the native C35 sequence. This epitope is not required for staining with 1F2. In contrast, the 1F2 antibody does not react with the approximately 8kDa band C which is, in addition, lacking residues 53-104 of the native C35 sequence. The results demonstrate that 1B3 antibody is specific for an epitope within C35 residues 105-115 (ITNSRPPCVIL), whereas 1F2 antibody is specific for an epitope within the C35 residues 53-104.

### EXAMPLE 12

### HUMAN ANTIBODIES RELATED TO 1B3 ANTI-C35 MONOCLONAL ANTIBODY

Two antibodies which are human-derived except for having the same immunoglobulin heavy chain CDR3 region as the mouse 1B3 anti-C35 monoclonal antibody were generated by the method disclosed in US 2002 0123057 A1, published 5 September 2002.

MAb 165 comprises the 141D10 VH H732 heavy chain variable region (SEQ ID NOS: 56 and 57), and the UH8 VK L120 kappa light chain variable region (SEQ ID NOS: 58 and 59). As shown in Figure 11, MAb 165 is C35-specific. 141D10 recombinant vaccinia virus was co-infected into HeLa cells with UH8 recombinant vaccinia virus. The resulting secreted antibody was tested for binding to C35 or control protein A27L (vaccinia virus protein) by ELISA.

MAb 171 comprises the MSH3 VH H835 heavy chain variable region (SEQ ID NOS:60 and 61) and the UH8 VK L120 kappa light chain variable region (SEQ ID NOS: 58 and 59).

### EXAMPLE 13

### IDENTIFICATION OF C35 PEPTIDE EPITOPES RECOGNIZED BY ANTI-C35 ANTIBODIES

Rabbit polyclonal antibodies were raised to recombinant C35 employing standard immunization methods well known in the art. Overlapping peptides 15 amino acids in length were synthesized corresponding to the 115 amino acid long C35 protein sequence beginning at each amino acid residue from 1 to 101. Peptides are named based on the C35 position of the amino terminus residue in each 15 amino acid peptide. In each peptide that overlapped the cysteine residues at positions 30, 33 and 112 of the natural C35 sequence, alanine was substituted for cysteine to avoid formation of disulfide crosslinked peptides.

Wells of 96 well Maxisorp microtiter plates were coated with either 2 µg C35 protein, or 14 µg or 40 µg of the indicated 15 amino acid peptide derived from the C35 protein sequence and binding of antibodies in the rabbit C35 immune serum was determined as described in detail below. Data in Table 11, below, is shown for the positive and negative controls and for those C35 derived peptides for which positive binding was detected. Variations in the level of peptide binding can be due to either differences in the concentration of specific antibody species, differences in antibody affinity or a combination thereof.

### A. Peptide sample preparation

C35 peptide 15-mers in 100% DMSO, 10mg/ml, were aliquotted into 1.5 ml tubes, 40µg/tube, under sterile conditions, then speed vacuumed to remove DMSO. The peptides were resuspended in PBS, pH7.2, 1ml/tube, mixed well and spun down. Each peptide concentration ("peptide solution") was 40µg/ml. Peptide solution should be stored at -20 °C until use. Once thawed, it should be kept at 4 °C for no more than 2 weeks.

### B. Coating samples on the Maxisorp plates

For 14 µg/ml peptide coated plates, 65 µl PBS, pH7.2 per well was added, followed by 35 µl peptide solution per well (for a total volume of 100 µl/well), and mixed well. For 40 µg/ml peptide coated plates, 100µl of peptide solution was placed directly on the plate, 100µl/well. Control C35 protein was diluted into PBS, pH7.2 to 2µg/ml and added to the plates, 100 µl/well. Coated plates were incubated at room temperature for 2 hours, then 4 °C overnight.

### C. ELISA conditions

Each plate was washed 3 times on a plate washer. Plates were blocked with "blocking buffer" (PBS, pH7.2 and 10% FBS) at room temperature for 2 hours, followed by washing each plate 3 times on plate washer. The primary antibody, rabbit anti-human C35 polyclonal antibody (made by Bethyl, 62902 batch) was diluted into "assay diluent" (PBS, pH7.2 plus 0.05% Tween 20 and 10% FBS) and added to the plates, 100 µl/well. Plates were incubated at room temperature for 2 hours. For 14 µg/ml peptide coated plates, 100 ng/ml of primary antibody was added. For 40 µg/ml peptide coated plates, 1 µg/ml of primary antibody was added. Plates were washed 5 times on a plate washer. The secondary antibody, HRP (horseradish peroxidase conjugated goat anti-rabbit Fc polyclonal antibody from Zymed, was diluted at a 1:20,000 dilution into assay diluent and added to the plates, 100 µl/well. The plates were incubated at room temperature for 2 hours. Plates were washed 7 times on a plate washer. Substrate was added as per the kit manufacturers instructions and incubated at room temperature in the dark for 15 minutes. The reaction was stopped by adding 2N H₂SO₄, 100 µl/well. Absorbance at 450-570 nm was read immediately.

**Table 11: Antibody Binding to C35 Epitopes**

| | | | |
|---|---|---|---|
| Absorbance @ 450-570 nm | | | |

| **Peptide** | **Sequence** | **14 µg/ml** | **40 µg/ml** |
|---|---|---|---|
| None | | 0.009 | 0.013 |
| without primary rabbit antibody | C35 protein or peptides | 0.009 | 0.009 |
| C35 protein | C35 protein | 3.765 | 3.5225 |
| P1 | MSGEPGQTSVAPPPE | 0.257 | |
| P2 | SGEPGQTSVAPPPEE | 0.108 | 1.227 |
| P3 | GEPGQTSVAPPPEEV | 1.458 | 3.349 |
| P4 | EPGQTSVAPPPEEVE | 1.254 | 3.282 |
| P5 | PGQTSVAPPPEEVEP | 2.719 | 3.383 |
| P6 | GQTSVAPPPEEVEPG | 2.483 | 3.381 |
| P7 | QTSVAPPPEEVEPGS | 2.635 | 3.388 |
| P8 | TSVAPPPEEVEPGSG | 0.059 | 0.394 |
| P9 | SVAPPPEEVEPGSGV | 2.31 | 3.367 |
| P10 | VAPPPEEVEPGSGVR | 1.736 | 3.407 |
| P11 | APPPEEVEPGSGVRI | 1.526 | 3.317 |
| P12 | PPPEEVEPGSGVRIV | 0.836 | |
| P13 | PPEEVEPGSGVRIW | 0.385 | 2.522 |
| P14 | PEEVEPGSGVRIVVE | 0.127 | 0.972 |
| P15 | EEVEPGSGVRIVVEY | 0.039 | 0.334 |
| P16 | EVEPGSGVRIVVEYA | 0.027 | 0.172 |
| P62 | TGAFEIEINGQLVFS | 0.023 | 0.107 |
| P63 | GAFEIEINGQLVFSK | 0.079 | 0.557 |
| P64 | AFEIEINGQLVFSKL | 0.055 | 0.423 |
| P65 | FEIEINGQLVFSKLE | 0.043 | 0.269 |
| P66 | EIEINGQLVFSKLEN | 0.028 | 0.182 |
| P80 | NGGFPYEKDLIEAIR | 0.018 | 0.1 |
| P81 | GGFPYEKDLIEAIRR | 0.032 | 0.204 |
| P82 | GFPYEKDLIEAIRRA | 0.02 | 0.18 |
| P83 | FPYEKDLIEAIRRAS | 0.025 | 0.19 |
| P84 | PYEKDLIEAIRRASN | 0.037 | 0.391 |
| P85 | YEKDLIEAIRRASNG | 0.024 | 0.179 |
| P86 | EKDLIEAIRRASNGE | 0.023 | 0.166 |
| P88 | DLIEAIRRASNGETL | 0.025 | 0.135 |
| P89 | LIEAIRRASNGETLE | 0.015 | 0.053 |
| P90 | IEAIRRASNGETLEK | 0.04 | 0.403 |
| P91 | EAIRRASNGETLEKI | 0.023 | 0.144 |
| P92 | AIRRASNGETLEKIT | 0.04 | 0.267 |
| P93 | IRRASNGETTLEKITN | 0.051 | 0.389 |
| P94 | RRASNGETLEKITNS | 0.061 | 0.526 |
| P95 | RASNGETLEKITNSR | 0.062 | 0.462 |
| P97 | SNGETLEKTINSRPP | 0.046 | 0.373 |
| P98 | NGETLEKITNSRPPA | 0.035 | 0.213 |
| P99 | GETLEKITNSRPPAV | 0.026 | 0.18 |
| P100 | ETLEKITNSRPPAVI | 0.017 | 0.124 |
| P101 | TLEKITNSRPPAVIL | 0.472 | 2.519 |

## Claims

1. An antibody, wherein said antibody is specific for C-35, for use in treating cancer in a mammal by killing cancer cells, said treatment comprising (a) administering an apoptosis-inducing therapy to the cancer cells, and (b) administering to said cells an antibody, wherein said antibody is specific for C-35, and wherein said antibody is administered at a time such that said antibody binds to C-35 expressed on the surface of said cancer cells at a time after (a) when apoptosis has been induced or is being induced in said cancer cells, thereby killing cancer cells undergoing apoptosis.

2. An antibody, wherein said antibody is specific for C35, for use in treating cancer in a mammal by preventing reversal of apoptotic progression in cells that express C35, said treatment comprising:
(a) administering an apoptosis-inducing therapy to said cells, and
(b) administering to said cells the antibody that specifically binds to C35, and wherein said antibody is administered at a time such that said antibody binds to C-35 expressed on the surface of said cancer cells at a time after (a) when apoptosis has been induced or is being induced in said cells, thereby killing said cells and preventing reversal of apoptotic progression in said cells.

3. The antibody for use in treating cancer according to claim 1 or claim 2, wherein said antibody is conjugated to or complexed with a toxin.

4. The antibody for use in treating cancer according to any preceding claim, wherein said antibody specific for C35 is an antibody selected from the group consisting of:
a) an antibody comprising the VH region defined in SEQ ID NO:8,
b) an antibody comprising the VL region defined in SEQ ID NO:10,
c) an antibody comprising the VH region defined in SEQ ID NO:4,
d) an antibody comprising the VL region defined in SEQ ID NO:6,
e) an antibody comprising the VH region of (a) and the VL region of (b),
f) an antibody comprising the VH region of (c) and the VL region of (d),
g) an antibody comprising at least one of CDR1, CDR2 or CDR3 of the VH region encoded by SEQ ID NO: 56,
h) an antibody comprising at least one of CDR1, CDR2 or CDR3 of the VH region encoded by SEQ ID NO: 60,
i) an antibody comprising at least one of CDR1, CDR2, or CDR3 of the VL region encoded by SEQ ID NO: 58,
j) a chimeric antibody comprising the VH region of (a) or (c),
k) a chimeric antibody comprising the VL region of (b) or (d),
l) a chimeric antibody comprising the VH region of (a) and the VL region of (b),
m) a chimeric antibody comprising the VH region of (c) and the VL region of (d),
n) the chimeric antibody of (j), (k), (1) or (m) which is a human chimeric antibody,
o) a humanized antibody comprising 1, 2, 3, 4, 5, or 6 CDRs of the antibody of (e) or (f), and
p) an antibody which binds the epitope bound by the antibody of any of (a) to (o).

5. The antibody for use in treating cancer according to any preceding claim, wherein said antibody is selected from the group consisting of a whole antibody, an antibody fragment, and a domain-deleted antibody, and wherein (b) occurs 6-12 hours, or 6-24 hours, or 6-36 hours, or 6-48 hours, or 6-72 hours, or 6-96 hours, or 6-120 hours, or 12-24 hours, or 12-36 hours, or 12-48 hours, or 12-72 hours, or 12-96 hours, or 12-120 hours, or 24-36 hours, or 24-48 hours, or 24-72 hours, or 24-96 hours, or 24-120 hours, or 36-48 hours, or 36-72 hours, or 36-96 hours, or 36-120 hours or 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 hours after (a).

6. The antibody for use in treating cancer according to any of claims 1 to 4, wherein the antibody is a whole antibody.

7. The antibody of any preceding claim, wherein the cancer is a C35-associated cancer selected from the group consisting of: breast cancer, ovarian cancer, bladder cancer, lung cancer, prostate cancer, pancreatic cancer, colon cancer, thyroid cancer, brain cancer, testicular cancer, uterine cancer, kidney cancer, liver cancer, lymphoma and melanoma.

8. The antibody of any preceding claim, wherein the mammal is a human.

9. The antibody of any of claims 3 to 8, wherein the toxin is a radioisotope, a radionuclide, a cytotoxin, or a cytotoxic prodrug.

10. The antibody of any preceding claim, wherein the mammal is in need of eradication of smaller tumors and/or micrometastases.

11. The antibody of any of claims 1 to 10, wherein said antibody is an immunoglobulin isotype selected from the group consisting of IgG3, IgM, IgA, IgD, and IgE.

12. The antibody of any of claims 1 to 11, wherein said antibody is a domain-deleted antibody.

13. The antibody of claim 12, wherein said domain-deleted antibody is a CH2 domain-deleted antibody.

14. The antibody of any of claims 1 to 13, wherein said antibody is an antibody fragment selected from the group consisting of an Fab, an F (ab') 2, an scFV, a minibody, a diabody, a triabody, and a tetrabody.

15. The antibody according to any preceding claim, wherein said treatment comprises multiple administrations of the antibody.

16. Use of an antibody, wherein said antibody is specific for C-35, in the manufacture of a composition for treating cancer in a mammal by killing cancer cells, said treatment comprising (a) administering an apoptosis-inducing therapy to the cancer cells, and (b) administering to said cells an antibody, wherein said antibody is specific for C-35, and wherein said antibody is administered at a time such that said antibody binds to C-35 expressed on the surface of said cancer cells at a time after (a) when apoptosis has been induced or is being induced in said cancer cells, thereby killing cancer cells undergoing apoptosis.

17. Use of an antibody, wherein said antibody is specific for C35, in the manufacture of a composition for treating cancer in a mammal by preventing reversal of apoptotic progression in cells that express C35, said treatment comprising:
(a) administering an apoptosis-inducing therapy to said cells, and
(b) administering to said cells the antibody that specifically binds to C35, and wherein said antibody is administered at a time such that said antibody binds to C-35 expressed on the surface of said cancer cell at a time after (a) when apoptosis has been induced or is being induced in said cells, thereby killing said cells and preventing reversal of apoptotic progression in said cells.

18. Use according to claim 16 or claim 17, wherein said antibody is conjugated to or complexed with a toxin.

19. Use according to any of claims 16 to 18, wherein said antibody specific for C35 is an antibody selected from the group consisting of:
a) an antibody comprising the VH region defined in SEQ ID NO:8,
b) an antibody comprising the VL region defined in SEQ ID NO:10,
c) an antibody comprising the VH region defined in SEQ ID NO:4,
d) an antibody comprising the VL region defined in SEQ ID NO:6,
e) an antibody comprising the VH region of (a) and the VL region of (b),
f) an antibody comprising the VH region of (c) and the VL region of (d),
g) an antibody comprising at least one of CDR1, CDR2 or CDR3 of the VH region encoded by SEQ ID NO: 56,
h) an antibody comprising at least one of CDR1, CDR2 or CDR3 of the VH region encoded by SEQ ID NO: 60,
i) an antibody comprising at least one of CDR1, CDR2, or CDR3 of the VL region encoded by SEQ ID NO: 58,
j) a chimeric antibody comprising the VH region of (a) or (c),
k) a chimeric antibody comprising the VL region of (b) or (d),
l) a chimeric antibody comprising the VH region of (a) and the VL region of (b),
m) a chimeric antibody comprising the VH region of (c) and the VL region of (d),
n) the chimeric antibody of (j), (k), (l) or (m) which is a human chimeric antibody,
o) a humanized antibody comprising 1, 2, 3, 4, 5, or 6 CDRs of the antibody of (e) or (f), and
p) an antibody which binds the epitope bound by the antibody of any of (a) to (o).

20. Use according to any of claims 16 to 19, wherein said antibody is selected from the group consisting of a whole antibody, an antibody fragment, and a domain-deleted antibody, and wherein (b) occurs 6-12 hours, or 6-24 hours, or 6-36 hours, or 6-48 hours, or 6-72 hours, or 6-96 hours, or 6-120 hours, or 12-24 hours, or 12-36 hours, or 12-48 hours, or 12-72 hours, or 12-96 hours, or 12-120 hours, or 24-36 hours, or 24-48 hours, or 24-72 hours, or 24-96 hours, or 24-120 hours, or 36-48 hours, or 36-72 hours, or 36-96 hours, or 36-120 hours or 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112,113, 114, 115, 116, 117, 118, 119, or 120 hours after (a).

21. Use according to any of claims 16 to 19, wherein the antibody is a whole antibody.

22. Use according to any of claims 16 to 21, wherein the cancer is a C35-associated cancer selected from the group consisting of: breast cancer, ovarian cancer, bladder cancer, lung cancer, prostate cancer, pancreatic cancer, colon cancer, thyroid cancer, brain cancer, testicular cancer, uterine cancer, kidney cancer, liver cancer, lymphoma and melanoma.

23. Use according to any of claims 16 to 22, wherein the mammal is a human.

24. Use according to any of claims 18 to 23, wherein the toxin is a radioisotope, a radionuclide, a cytotoxin, or a cytotoxic prodrug.

25. Use according to any of claims 16 to 24, wherein the mammal is in need of eradication of smaller tumors and/or micrometastases.

26. Use according to any of claims 16 to 25, wherein said antibody is an immunoglobulin isotype selected from the group consisting of IgG3, IgM, IgA, IgD, and IgE.

27. Use according to any of claims 16 to 26, wherein said antibody is a domain-deleted antibody.

28. Use according to claim 27, wherein said domain-deleted antibody is a CH2 domain-deleted antibody.

29. Use according to any of claims 16 to 28, wherein said antibody is an antibody fragment selected from the group consisting of an Fab, an F (ab') 2, an scFV, a minibody, a diabody, a triabody, and a tetrabody.

30. Use according to any of claims 16 to 29, wherein said treatment comprises multiple administrations of the antibody.

## Patentansprüche

1. Antikörper, wobei der Antikörper für C35 spezifisch ist, für die Verwendung bei der Behandlung von Krebs in einem Säugetier durch Abtöten von Krebszellen, wobei die Behandlung umfasst: (a) Anwenden einer die Apoptose induzierenden Behandlung auf die Krebszellen, und (b) Verabreichen eines Antikörpers an die Zellen, wobei der Antikörper für C35 spezifisch ist, und wobei der Antikörper zeitlich so verabreicht wird, dass der Antikörper an C35, das auf der Oberfläche der Krebszellen exprimiert wird, zu einem Zeitpunkt nach (a) bindet, wenn in den Krebszellen Apoptose induziert wurde oder induziert wird, wodurch die die Apoptose durchlaufenden Krebszellen getötet werden.

2. Antikörper, wobei der Antikörper für C35 spezifisch ist, für die Verwendung bei der Behandlung von Krebs in einem Säugetier, indem die Umkehrung des apoptotischen Verlaufs in den C35 exprimierenden Zellen verhindert wird, wobei die Behandlung umfasst:
(a) Anwenden einer die Apoptose induzierenden Behandlung auf die Zellen und
(b) Verabreichen des Antikörpers, der spezifisch an C35 bindet, an die Zellen und wobei der Antikörper zeitlich so verabreicht wird, dass der Antikörper an C35, das auf der Oberfläche der Krebszellen exprimiert wird, zu einem Zeitpunkt nach (a) bindet, wenn in den Zellen die Apoptose induziert wurde oder induziert wird, wodurch die Zellen getötet werden und die Umkehrung des apoptotischen Verlaufs in den Zellen verhindert wird.

3. Antikörper für die Verwendung bei der Behandlung von Krebs gemäß Anspruch 1 oder Anspruch 2, wobei der Antikörper mit einem Toxin ein Konjugat oder einen Komplex bildet.

4. Antikörper für die Verwendung bei der Behandlung von Krebs gemäß einem vorhergehenden Anspruch, wobei der für C35 spezifische Antikörper ein Antikörper ist, der ausgewählt ist aus der Gruppe, bestehend aus:
a) einem Antikörper, der die in SEQ ID NO. 8 definierte VH-Region umfasst,
b) einem Antikörper, der die in SQ ID NO. 10 definierte VL-Region umfasst,
c) einem Antikörper, der die in SEQ ID NO. 4 definierte VH-Region umfasst,
d) einem Antikörper, der die in SEQ ID NO. 6 definierte VL-Region umfasst,
e) einem Antikörper, der die VH-Region von (a) und die VL-Region von (b) umfasst,
f) einem Antikörper, der die VH-Region von (c) und die VL-Region von (d) umfasst,
g) einem Antikörper, der mindestes eines aus CDR1, CDR2 oder CDR3 aus der VH-Region umfasst, die durch SEQ ID NO. 56 codiert ist,
h) einem Antikörper, der mindestens eines aus CDR1, CDR2 oder CDR3 aus der VH-Region umfasst, die durch SEQ ID NO. 60 codiert ist,
i) einem Antikörper, der mindestens eines aus CDR1, CDR2 oder CDR3 aus der VL-Region umfasst, die durch SEQ ID NO. 58 codiert ist,
j) einem chimären Antikörper, der die VH-Region von (a) oder (c) umfasst,
k) einem chimären Antikörper, der die VL-Region von (b) oder (d) umfasst,
l) einem chimären Antikörper, der die VH-Region von (a) und die VL-Region von (b) umfasst,
m) einem chimären Antikörper, der die VH-Region von (c) und die VL-Region von (d) umfasst,
n) einem chimären Antikörper gemäß (j), (k), (l) oder (m), der ein humaner chimärer Antikörper ist,
o) einem humanisiertem Antikörper, der 1, 2, 3, 4, 5 oder 6 CDRs von dem Antikörper gemäß (e) oder (f) umfasst, und
p) einem Antikörper, der das durch einen Antikörper gemäß (a) bis (o) gebundene Epitop bindet.

5. Antikörper für die Verwendung bei der Behandlung von Krebs gemäß einem der vorhergehenden Ansprüche, wobei der Antikörper ausgewählt ist aus der Gruppe bestehend aus einem ganzen Antikörper, einem Antikörperfragment, einem Antikörper mit deletierter Domäne, und wobei (b) nach 6 bis 12 Stunden, oder 6 bis 24 Stunden, oder 6 bis 36 Stunden, oder 6 bis 48 Stunden, oder 6 bis 72 Stunden, oder 6 bis 96 Stunden, oder 6 bis 120 Stunden, oder 12 bis 24 Stunden, oder 12 bis 36 Stunden, oder 12 bis 48 Stunden, oder 12 bis 72 Stunden, oder 12 bis 96 Stunden, oder 12 bis 120 Stunden, oder 24 bis 36 Stunden, oder 24 bis 48 Stunden, oder 24 bis 72 Stunden, oder 24 bis 96 Stunden, oder 24 bis 120 Stunden, oder 36 bis 48 Stunden oder 36 bis 72 Stunden, oder 36 bis 96 Stunden, oder 36 bis 120 Stunden oder 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 oder 120 Stunden nach a) erfolgt.

6. Antikörper für die Verwendung bei der Behandlung von Krebs gemäß einem der Ansprüche 1 bis 4, wobei der Antikörper ein ganzer Antikörper ist.

7. Antikörper nach einem vorhergehenden Anspruch, wobei der Krebs ein mit C35 assoziierter Krebs ist, der ausgewählt ist aus der Gruppe bestehend aus: Brustkrebs, Eierstockkrebs, Blasenkrebs, Lungenkrebs, Prostatakrebs, Bauchspeicheldrüsenkrebs, Darmkrebs, Schilddrüsenkrebs, Hirntumor, Hodenkrebs, Gebärmutterkrebs, Nierenkrebs, Leberkrebs, Lymphom und Melanom.

8. Antikörper nach einem vorhergehenden Anspruch, wobei das Säugetier ein Mensch ist.

9. Antikörper nach einem der Ansprüche 3 bis 8, wobei das Toxin ein Radioisotop, ein Radionuclid, ein Cytotoxin oder ein cytotoxischer Arzneimittelvorläufer ist.

10. Antikörper nach einem vorhergehenden Anspruch, wobei das Säugetier der Beseitigung kleinerer Tumore und/oder Mikrometastasen bedarf.

11. Antikörper nach einem der Ansprüche 1 bis 10, wobei der Antikörper ein Immunoglobulin-Isotyp ist, der ausgewählt ist aus der Gruppe bestehend aus IgG3, IgM, IgA, IgD und IgE.

12. Antikörper nach einem der Ansprüche 1 bis 11, wobei der Antikörper ein Antikörper mit deletierter Domäne ist.

13. Antikörper nach Anspruch 12, wobei der Antikörper mit deletierter Domäne ein Antikörper mit deletierter CH2-Domäne ist.

14. Antikörper nach einem der Ansprüche 1 bis 13, wobei der Antikörper ein Antikörperfragment ist, das ausgewählt ist aus der Gruppe bestehend aus einem Fab, einem F(ab')2, einem scFV, einem Minibody, einem Diabody, einem Triabody und einem Tetrabody.

15. Antikörper nach einem der vorhergehenden Ansprüche, wobei die Behandlung mehrere Verabreichungen des Antikörpers umfasst.

16. Verwendung eines Antikörpers, wobei der Antikörper für C35 spezifisch ist, bei der Herstellung einer Zusammensetzung für die Behandlung von Krebs in einem Säugetier durch Abtöten der Krebszellen, wobei die Behandlung umfasst (a) Anwenden einer die Apoptose induzierenden Behandlung auf die Krebszellen, und (b) Verabreichen eines Antikörpers an die Zellen, wobei der Antikörper für C35 spezifisch ist, und wobei der Antikörper zeitlich so verabreicht wird, dass der Antikörper an C35, das auf der Oberfläche der Kebszellen exprimiert wird, zu einem Zeitpunkt nach (a) bindet, wenn in den Krebszellen Apoptose induziert wurde oder induziert wird, wodurch die die Apoptose durchlaufenden Krebszellen getötet werden.

17. Verwendung eines Antikörpers, wobei der Antikörper für C35 spezifisch ist, bei der Herstellung einer Zusammensetzung zur Behandlung von Krebs in einem Säugetier, indem man die Umkehrung des apoptotischen Verlaufs in den Zellen verhindert, die C35 exprimieren, wobei die Behandlung umfasst:
(a) Anwenden einer die Apoptose induzierenden Behandlung auf die Zellen und
(b) Verabreichen eines Antikörpers an die Zellen, der spezifisch an C35 bindet, und wobei der Antikörper zeitlich so verabreicht wird, dass der Antikörper an C35, das auf der Oberfläche der Krebszellen exprimiert wird, zu einem Zeitpunkt nach (a) bindet, wenn in den Zellen die Apoptose induziert wurde oder induziert wird und **dadurch** Krebszellen getötet werden und die Umkehrung des apoptotischen Verlaufs in den Zellen verhindert wird.

18. Verwendung gemäß Anspruch 16 oder Anspruch 17, wobei der Antikörper mit einem Toxin ein Konjugat oder einen Komplex bildet.

19. Verwendung gemäß einem der Ansprüche 16 bis 18, wobei der für C35 spezifische Antikörper ausgewählt ist aus einer Gruppe bestehend aus:
a) einem Antikörper, der die in SEQ ID NO. 8 definierte VH-Region umfasst,
b) einem Antikörper, der die in SQ ID NO. 10 definierte VL-Region umfasst,
c) einem Antikörper, der die in SEQ ID NO. 4 definierte VH-Region umfasst,
d) einem Antikörper, der die in SEQ ID NO. 6 definierte VL-Region umfasst,
e) einem Antikörper, der die VH-Region von (a) und die VL-Region von (b) umfasst,
f) einem Antikörper, der die VH-Region von (c) und die VL-Region von (d) umfasst,
g) einem Antikörper, der mindestes eines aus CDR1, CDR2 oder CDR3 aus der VH-Region umfasst, die durch SEQ ID NO. 56 codiert ist,
h) einem Antikörper, der mindestens eines aus CDR1, CDR2 oder CDR3 aus der VH-Region umfasst, die durch SEQ ID NO. 60 codiert ist,
i) einem Antikörper, der mindestens eines aus CDR1, CDR2 oder CDR3 aus der VL-Region umfasst, die durch SEQ ID NO. 58 codiert ist,
j) einem chimären Antikörper, der die VH-Region von (a) oder (c) umfasst,
k) einem chimären Antikörper, der die VL-Region von (b) oder (d) umfasst,
l) einem chimären Antikörper, der die VH-Region von (a) und die VL-Region von (b) umfasst,
m) einem chimären Antikörper, der die VH-Region von (c) und die VL-Region von (d) umfasst,
n) einem chimären Antikörper gemäß (j), (k), (l) oder (m), der ein humaner chimärer Antikörper ist,
o) einem humanisiertem Antikörper, der 1, 2, 3, 4, 5 oder 6 CDRs von dem Antikörper gemäß (e) oder (f) umfasst, und
p) einem Antikörper, der das durch den Antikörper gemäß (a) bis (o) gebundene Epitop bindet.

20. Verwendung gemäß einem der Ansprüche 16 bis 19, wobei der Antikörper ausgewählt ist aus der Gruppe bestehend aus einem ganzen Antikörper, einem Antikörperfragment, einem Antikörper mit deletierter Domäne und wobei (b) nach 6-12 Stunden, oder 6 bis 24 Stunden, oder 6 bis 36 Stunden, oder 6 bis 48 Stunden, oder 6 bis 72 Stunden, oder 6 bis 96 Stunden, oder 6 bis 120 Stunden, oder 12 bis 24 Stunden, oder 12 bis 36 Stunden, oder 12 bis 48 Stunden, oder 12 bis 72 Stunden, oder 12 bis 96 Stunden, oder 12 bis 120 Stunden, oder 24 bis 36 Stunden, oder 24 bis 48 Stunden, oder 24 bis 72 Stunden, oder 24 bis 96 Stunden, oder 24 bis 120 Stunden, oder 36 bis 48 Stunden oder 36 bis 72 Stunden, oder 36 bis 96 Stunden, oder 36 bis 120 Stunden oder 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 oder 120 Stunden nach (a) erfolgt.

21. Verwendung gemäß einem der Ansprüche 16 bis 19, wobei der Antikörper ein ganzer Antikörper ist.

22. Verwendung gemäß einem der Ansprüche 16 bis 21, wobei der Krebs ein mit C35 assoziierter Krebs ist, der ausgewählt ist aus der Gruppe bestehend aus Brustkrebs, Brustkrebs, Eierstockkrebs, Blasenkrebs, Lungenkrebs, Prostatakrebs, Bauchspeicheldrüsenkrebs, Darmkrebs, Schilddrüsenkrebs, Hirntumor, Hodenkrebs, Gebärmutterkrebs, Nierenkrebs, Leberkrebs, Lymphom und Melanom.

23. Verwendung gemäß einem der Ansprüche 16 bis 22, wobei das Säugetier ein Mensch ist.

24. Verwendung gemäß einem der Ansprüche 18 bis 23, wobei das Toxin ein Radioisotop, ein Radionulid, ein Cytotoxin oder ein cytotoxischer Arzneimittelvorläufer ist.

25. Verwendung gemäß einem der Ansprüche 16 bis 24, wobei das Säugetier der Beseitigung kleinerer Tumore und/oder Mikrometastasen bedarf.

26. Verwendung gemäß einem der Ansprüche 16 bis 25, wobei der Antikörper ein Immunoglobulin-Isotyp ist, der ausgewählt ist aus der Gruppe bestehend aus IgG3, IgM, IgA, IgD und IgE.

27. Verwendung gemäß einem der Ansprüche 16 bis 26, wobei der Antikörper ein Antikörper mit deletierter Domäne ist.

28. Verwendung gemäß Anspruch 27, wobei der Antikörper mit deletierter Domäne ein Antikörper mit deletierter CH2-Domäne ist.

29. Verwendung gemäß einem der Ansprüche 16 bis 28, wobei der Antikörper ein Antikörperfragment ist, das ausgewählt ist aus der Gruppe bestehend aus einem Fab, einem F(ab')2, einem scFV, einem Minibody, einem Diabody, einem Triabody und einem Tetrabody.

30. Verwendung gemäß einem der Ansprüche 16 bis 29, wobei die Behandlung mehrere Verabreichungen des Antikörpers umfasst.

## Revendications

1. Anticorps, où ledit anticorps est spécifique pour C-35, destiné à être utilisé dans le traitement du cancer chez un mammifère en tuant des cellules cancéreuses, ledit traitement comprenant les étapes consistant à (a) administrer une thérapie induisant l'apoptose des cellules cancéreuses, et (b) administrer auxdites cellules un anticorps, où ledit anticorps est spécifique pour C-35, et où ledit anticorps est administré à un moment tel que ledit anticorps se lie à C-35 exprimé sur la surface desdites cellules cancéreuses à un moment ultérieur à (a), lorsque l'apoptose a été induite ou est induite dans lesdites cellules cancéreuses, en tuant ainsi les cellules cancéreuses subissant l'apoptose.

2. Anticorps, où ledit anticorps est spécifique pour C35, destiné à être utilisé dans le traitement du cancer chez un mammifère en empêchant l'inversion de la progression de l'apoptose dans des cellules qui expriment C35, ledit traitement comprenant les étapes consistant à :
(a) administrer une thérapie induisant l'apoptose auxdites cellules, et
(b) administrer auxdites cellules l'anticorps qui se lie spécifiquement à C35, et où ledit anticorps est administré à un moment tel que ledit anticorps se lie à C-35 exprimé sur la surface desdites cellules cancéreuses à un moment ultérieur à (a), lorsque l'apoptose a été induite ou est induite dans lesdites cellules, en tuant ainsi lesdites cellules et en empêchant l'inversion de la progression de l'apoptose dans lesdites cellules.

3. Anticorps destiné à être utilisé dans le traitement du cancer selon la revendication 1 ou la revendication 2, où ledit anticorps est conjugué à ou forme un complexe avec une toxine.

4. Anticorps destiné à être utilisé dans le traitement du cancer selon l'une quelconque des revendications précédentes, où ledit anticorps spécifique pour C35 est un anticorps sélectionné dans le groupe consistant en :
a) un anticorps comprenant la région VH définie dans SEQ ID NO: 8,
b) un anticorps comprenant la région VL définie dans SEQ ID NO: 10,
c) un anticorps comprenant la région VH définie dans SEQ ID NO: 4,
d) un anticorps comprenant la région VL définie dans SEQ ID NO: 6,
e) un anticorps comprenant la région VH de (a) et la région VL de (b),
f) un anticorps comprenant la région VH de (c) et la région VL de (d),
g) un anticorps comprenant au moins l'un des CDR1, CDR2 ou CDR3 de la région VH codée par SEQ ID NO: 56,
h) un anticorps comprenant au moins l'un des CDR1, CDR2 ou CDR3 de la région VH codée par SEQ ID NO: 60,
i) un anticorps comprenant au moins l'un des CDR1, CDR2 ou CDR3 de la région VL codée par SEQ ID NO: 58,
j) un anticorps chimérique comprenant la région VH de (a) ou (c),
k) un anticorps chimérique comprenant la région VL de (b) ou (d),
l) un anticorps chimérique comprenant la région VH de (a) et la région VL de (b),
m) un anticorps chimérique comprenant la région VH de (c) et la région VL de (d),
n) l'anticorps chimérique de (j), (k), (1) ou (m) qui est un anticorps chimérique humain,
o) un anticorps humanisé comprenant 1, 2, 3, 4, 5 ou 6 CDR de l'anticorps de (e) ou (f), et
p) un anticorps qui se lie à l'épitope lié par l'anticorps de l'un quelconque de (a) à (o).

5. Anticorps destiné à être utilisé dans le traitement du cancer selon l'une quelconque des revendications précédentes, où ledit anticorps est sélectionné dans le groupe consistant en un anticorps entier, un fragment d'anticorps, et un anticorps présentant une délétion de domaine, et où (b) se produit 6-12 heures, ou 6-24 heures, ou 6-36 heures, ou 6-48 heures, ou 6-72 heures, ou 6-96 heures, ou 6-120 heures, ou 12-24 heures, ou 12-36 heures, ou 12-48 heures, ou 12-72 heures, ou 12-96 heures, ou 12-120 heures, ou 24-36 heures, ou 24-48 heures, ou 24-72 heures, ou 24-96 heures, ou 24-120 heures, ou 36-48 heures, ou 36-72 heures, ou 36-96 heures, ou 36-120 heures ou 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68; 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, ou 120 heures après (a).

6. Anticorps destiné à être utilisé dans le traitement du cancer selon l'une quelconque des revendications 1 à 4, où l'anticorps est un anticorps entier.

7. Anticorps selon l'une quelconque des revendications précédentes, où le cancer est un cancer associé à C35 sélectionné dans le groupe consistant en : le cancer du sein, le cancer de l'ovaire, le cancer de la vessie, le cancer du poumon, le cancer de la prostate, le cancer du pancréas, le cancer du côlon, le cancer de la thyroïde, le cancer du cerveau, le cancer des testicules, le cancer de l'utérus, le cancer du rein, le cancer du foie, un lymphome et un mélanome.

8. Anticorps selon l'une quelconque des revendications précédentes, où le mammifère est un humain.

9. Anticorps selon l'une quelconque des revendications 3 à 8, où la toxine est un radio-isotope, un radionuclide, une cytotoxine, ou un promédicament cytotoxique.

10. Anticorps selon l'une quelconque des revendications précédentes, où le mammifère nécessite une éradication de tumeurs plus petites et/ou de micrométastases.

11. Anticorps selon l'une quelconque des revendications 1 à 10, où ledit anticorps est un isotype d'immunoglobuline sélectionné dans le groupe consistant en IgG3, IgM, IgA, IgD, et IgE.

12. Anticorps selon l'une quelconque des revendications 1 à 11, où ledit anticorps est un anticorps présentant une délétion de domaine.

13. Anticorps selon la revendication 12, où ledit anticorps présentant une délétion de domaine est un anticorps présentant une délétion du domaine CH2.

14. Anticorps selon l'une quelconque des revendications 1 à 13, où ledit anticorps est un fragment d'anticorps sélectionné dans le groupe consistant en un Fab, un F(ab')2, un scFV, un mini-anticorps, un di-anticorps, un tri-anticorps, et un tétra-anticorps.

15. Anticorps selon l'une quelconque des revendications précédentes, où ledit traitement comprend des administrations multiples de l'anticorps.

16. Utilisation d'un anticorps, où ledit anticorps est spécifique pour C-35, dans la fabrication d'une composition destinée au traitement du cancer chez un mammifère en tuant des cellules cancéreuses, ledit traitement comprenant les étapes consistant à (a) administrer une thérapie induisant l'apoptose aux cellules cancéreuses, et (b) administrer auxdites cellules un anticorps, où ledit anticorps est spécifique pour C-35, et où ledit anticorps est administré à un moment tel que ledit anticorps se lie à C-35 exprimé sur la surface desdites cellules cancéreuses à un moment ultérieur à (a), lorsque l'apoptose a été induite ou est induite dans lesdites cellules cancéreuses, en tuant ainsi les cellules cancéreuses subissant l'apoptose.

17. Utilisation d'un anticorps, où ledit anticorps est spécifique pour C35, dans la fabrication d'une composition destinée au traitement du cancer chez un mammifère en empêchant l'inversion de la progression de l'apoptose dans des cellules qui expriment C35, ledit traitement comprenant les étapes consistant à :
(a) administrer une thérapie induisant l'apoptose auxdites cellules, et
(b) administrer auxdites cellules l'anticorps qui se lie spécifiquement à C35, et où ledit anticorps est administré à un moment tel que ledit anticorps se lie à C-35 exprimé sur la surface de ladite cellule cancéreuse à un moment ultérieur à (a), lorsque l'apoptose a été induite ou est induite dans lesdites cellules, en tuant ainsi lesdites cellules et en empêchant l'inversion de la progression de l'apoptose dans lesdites cellules.

18. Utilisation selon la revendication 16 ou la revendication 17, où ledit anticorps est conjugué à ou forme un complexe avec une toxine.

19. Utilisation selon l'une quelconque des revendications 16 à 18, où ledit anticorps spécifique pour C35 est un anticorps sélectionné dans le groupe consistant en :
a) un anticorps comprenant la région VH définie dans SEQ ID NO: 8,
b) un anticorps comprenant la région VL définie dans SEQ ID NO: 10,
c) un anticorps comprenant la région VH définie dans SEQ ID NO: 4,
d) un anticorps comprenant la région VL définie dans SEQ ID NO: 6,
e) un anticorps comprenant la région VH de (a) et la région VL de (b),
f) un anticorps comprenant la région VH de (c) et la région VL de (d),
g) un anticorps comprenant au moins l'un des CDR1, CDR2 ou CDR3 de la région VH codée par SEQ ID NO: 56,
h) un anticorps comprenant au moins l'un des CDR1, CDR2 ou CDR3 de la région VH codée par SEQ ID NO: 60,
i) un anticorps comprenant au moins l'un des CDR1, CDR2 ou CDR3 de la région VL codée par SEQ ID NO: 58,
j) un anticorps chimérique comprenant la région VH de (a) ou (c),
k) un anticorps chimérique comprenant la région VL de (b) ou (d),
l) un anticorps chimérique comprenant la région VH de (a) et la région VL de (b),
m) un anticorps chimérique comprenant la région VH de (c) et la région VL de (d),
n) l'anticorps chimérique de (j), (k), (1) ou (m) qui est un anticorps chimérique humain,
o) un anticorps humanisé comprenant 1, 2, 3, 4, 5 ou 6 CDR de l'anticorps de (e) ou (f), et
p) un anticorps qui se lie à l'épitope lié par l'anticorps de l'un quelconque de (a) à (o).

20. Utilisation selon l'une quelconque des revendications 16 à 19, où ledit anticorps est sélectionné dans le groupe consistant en un anticorps entier, un fragment d'anticorps, et un anticorps présentant une délétion de domaine, et où (b) se produit 6-12 heures, ou 6-24 heures, ou 6-36 heures, ou 6-48 heures, ou 6-72 heures, ou 6-96 heures, ou 6-120 heures, ou 12-24 heures, ou 12-36 heures, ou 12-48 heures, ou 12-72 heures, ou 12-96 heures, ou 12-120 heures, ou 24-36 heures, ou 24-48 heures, ou 24-72 heures, ou 24-96 heures, ou 24-120 heures, ou 36-48 heures, ou 36-72 heures, ou 36-96 heures, ou 36-120 heures ou 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 5, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, ou 120 heures après (a).

21. Utilisation selon l'une quelconque des revendications 16 à 19, où l'anticorps est un anticorps entier.

22. Utilisation selon l'une quelconque des revendications 16 à 21, où le cancer est un cancer associé à C35 sélectionné dans le groupe consistant en : le cancer du sein, le cancer de l'ovaire, le cancer de la vessie, le cancer du poumon, le cancer de la prostate, le cancer du pancréas, le cancer du côlon, le cancer de la thyroïde, le cancer du cerveau, le cancer des testicules, le cancer de l'utérus, le cancer du rein, le cancer du foie, un lymphome et un mélanome.

23. Utilisation selon l'une quelconque des revendications 16 à 22, où le mammifère est un humain.

24. Utilisation selon l'une quelconque des revendications 18 à 23, où la toxine est un radio-isotope, un radionuclide, une cytotoxine, ou un promédicament cytotoxique.

25. Utilisation selon l'une quelconque des revendications 16 à 24, où le mammifère nécessite une éradication de tumeurs plus petites et/ou de micrométastases.

26. Utilisation selon l'une quelconque des revendications 16 à 25, où ledit anticorps est un isotype d'immunoglobuline sélectionné dans le groupe consistant en IgG3, IgM, IgA, IgD, et IgE.

27. Utilisation selon l'une quelconque des revendications 16 à 26, où ledit anticorps est un anticorps présentant une délétion de domaine.

28. Utilisation selon la revendication 27, où ledit anticorps présentant une délétion de domaine est un anticorps présentant une délétion du domaine CH2.

29. Utilisation selon l'une quelconque des revendications 16 à 28, où ledit anticorps est un fragment d'anticorps sélectionné dans le groupe consistant en un Fab, un F(ab')2, un scFV, un mini-anticorps, un di-anticorps, un tri-anticorps, et un tétra-anticorps.

30. Utilisation selon l'une quelconque des revendications 16 à 29, où ledit traitement comprend des administrations multiples de l'anticorps.
